(19) Europäisches Patentamt
European Patent Office
Office européen des brevets

(11) Numéro de publication : **0 041 021**
**B1**

(12) # FASCICULE DE BREVET EUROPÉEN

(45) Date de publication du fascicule du brevet : **25.07.84**

(21) Numéro de dépôt : **81400816.5**

(22) Date de dépôt : **22.05.81**

(51) Int. Cl.[3] : **C 07 C 69/743**, C 07 C121/75, C 07 D213/64, C 07 D233/72, C 07 D209/48, C 07 D307/42, A 01 N 53/00, A 61 K 31/22, A 23 K 1/16

(54) **Nouveaux dérivés de l'acide cyclopropane carboxylique, leur préparation, leur application à la lutte contre les parasites des végétaux et des animaux, les compositions les renfermant et les nouveaux intermédiaires obtenus.**

(30) Priorité : **23.05.80 FR 8011569**

(43) Date de publication de la demande : **02.12.81 Bulletin 81/48**

(45) Mention de la délivrance du brevet : **25.07.84 Bulletin 84/30**

(84) Etats contractants désignés : **AT BE CH DE FR GB IT LI LU NL SE**

(56) Documents cités :
**DE-A- 2 418 572**
**FR-A- 2 143 820**
**FR-A- 2 185 612**
**FR-A- 2 340 925**
**FR-A- 2 418 218**
**US-A- 4 242 357**
**PESTICIDE SCIENCES 1976, 7, 499-502 M. ELLIOTT et al.: "Insecticidal Activity of the Pyrethrins and Related Compounds: X.a5-Benzyl-3-furylmethyl 2,2-dimethylcyclo-propanecarboxylates with ethylenic substituents at position 3 on the cyclopropane ring"**
**JOURNAL OF THE CHEMICAL SOCIETY, Perkin Transactions I, no. JCPRB4 (21), 1974, pages 2470-2474 M. ELLIOTT et al.: " The Pyrethrins and Related Compounds Part XVIII. Insecticidal 2,2-Dimethylcyclopropanecarboxylates with New Unsaturated 3-Substituents"**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140-Bondy (FR)**
Inventeur : **Tessier, Jean**
**30, rue Jean Moulin**
**F-94300-Vincennes (FR)**
Inventeur : **Teche, André**
**15, rue Godot de Mauroy**
**F-75009-Paris (FR)**

(74) Mandataire : **Fritel, Hubert et al**
**102, route de Noisy Boîte Postale No 9**
**F-93230 Romainville (FR)**

**EP 0 041 021 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de l'acide cyclopropane carboxylique, leur préparation, leur application à la lutte contre les parasites, les compositions les renfermant et les nouveaux intermédiaires obtenus.

L'invention a pour objet des dérivés de l'acide cyclopropane carboxylique, portant en position 3 une chaîne latérale insaturée de géométrie Z et de structure

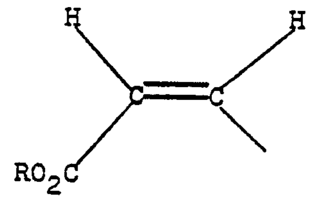

On connaissait certes des dérivés de l'acide cyclopropane carboxylique portant en position 3 une chaîne de structure

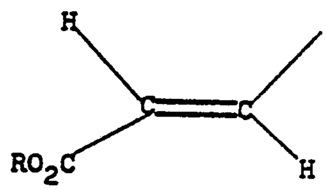

mais dont la géométrie était essentiellement E. Citons par exemple le brevet français 2 185 612 ainsi que J. Chem. Soc. Perkin I, page 2470, 1974 et Pest. Sci *7*, page 499, 1976. Toutefois la géométrie latérale de ces composés était E de façon prédominante. En effet, les procédés de synthèse utilisés pour préparer ces dérivés ne conduisaient presque exclusivement qu'à la géométrie E (voir Agr. Biol. Chem. 34, page 1119 *1970*). Pour ces composés dont la géométrie de la chaîne latérale était E, aucune propriété remarquable n'a pu être mise en évidence.

On vient de découvrir que certains produits dont la chaîne latérale est de structure Z présentent des propriétés insecticides exceptionnelles.

L'invention a pour objet sous toutes les formes isomères possibles, les composés de formule (I')

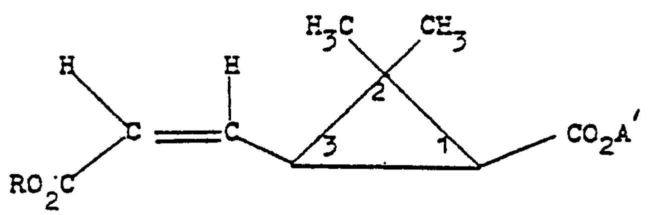

(I')

dans laquelle la double liaison a la géométrie Z, la copule cyclopropanique est de structure IR cis et A' représente :

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le reste méthylène dioxy, et les atomes d'halogène,

soit un groupement

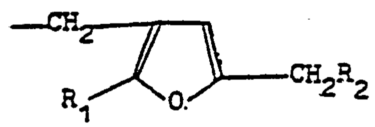

dans lequel le substituant $R_1$ représente un atome d'hydrogène ou un radical méthyle et le substituant $R_2$ un aryle monocyclique ou un groupement $-CH_2-C \equiv CH$ et notamment un groupement 5-benzyl 3-furylméthyle,

soit un groupement

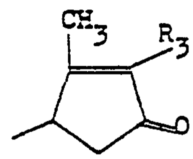

dans lequel $R_3$ représente un radical organique aliphatique comportant de 2 à 6 atomes de carbone et une ou plusieurs insaturations carbone-carbone et notamment les radicaux $-CH_2-CH=CH_2$, $-CH_2-CH=CH-CH_3$, $-CH_2-CH=CH-C_2H_5$, $-CH_2-CH=CH-CH=CH_2$

soit un groupement

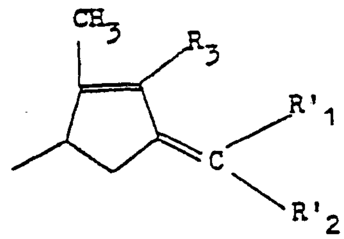

dans lequel $R_3$ conserve la même signification que précédemment, $R_1'$ et $R_2'$ identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

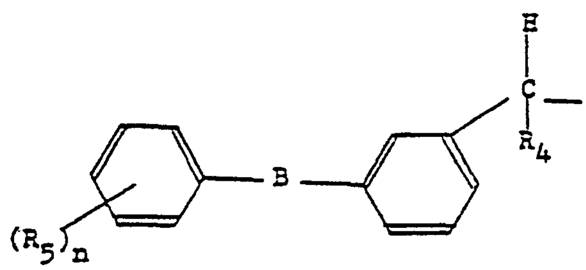

dans lequel B représente un groupement $CH_2$ ou $C=O$, ou un hétéroélément choisi parmi l'oxygène et le soufre, $R_4$ représente un atome d'hydrogène, un radical méthyle, un radical $-CONH_2$, un radical $-CSNH_2$ ou un radical $-C\equiv CH$ et $R_5$ représente un atome d'halogène ou un radical méthyle et n représente un nombre égal à 0, 1 ou 2, et notamment le groupement 3-phénoxy benzyle, α-éthynyl 3-phénoxy benzyle, 3-benzoyl benzyle, 1-(3 phénoxyphényl) éthyle ou α-thiamido 3-phénoxybenzyle,

soit un groupement

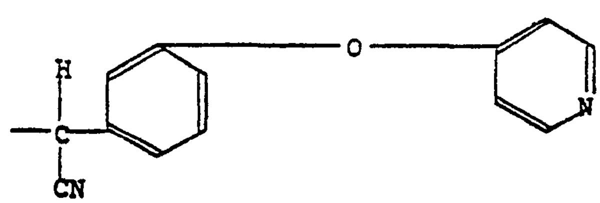

soit un groupement

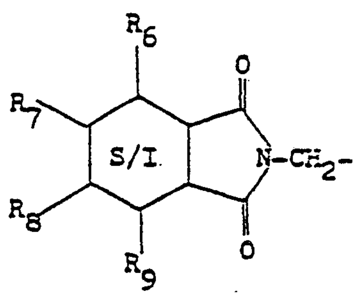

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

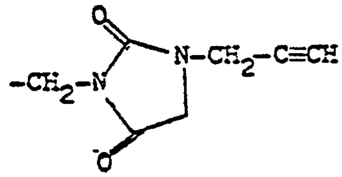

soit un groupement

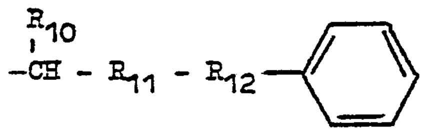

dans lequel $R_{10}$ représente un atome d'hydrogène ou un radical CN, $R_{12}$ représente un radical $—CH_2$ ou un atome d'oxygène, $R_{11}$ représente un radical thiazolyle ou thiadiazolyle dont la liaison avec

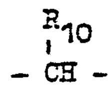

peut se trouver à l'une quelconque des positions disponibles $R_{12}$ étant lié à $R_{11}$ par l'atome de carbone compris entre l'atome de soufre et un atome d'azote,

soit un groupement

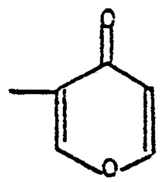

soit un groupement

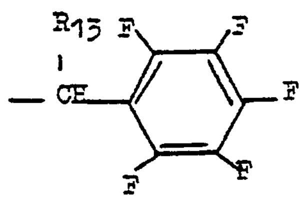

dans lequel $R_{13}$ représente un atome d'hydrogène ou un radical CN,

soit un groupement

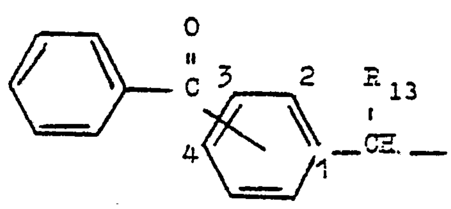

dans lequel $R_{13}$ est défini comme ci-dessus, et le radical benzoyle est en position 3 ou 4,

soit un groupement

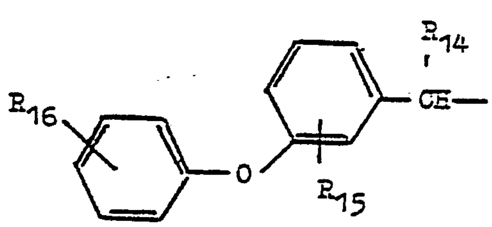

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

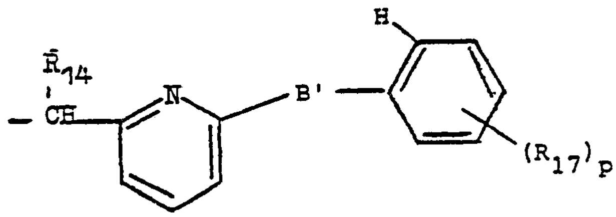

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre et R représente un radical alcoyle linéaire, ramifié ou cyclique, saturé ou insaturé, renfermant de 1 à 18 atomes de carbone, ainsi que les mélanges de ces isomères.

Les composés de formule (I') peuvent exister sous des formes isomères puisqu'ils possèdent tous deux carbones asymétriques en 1 et 3 du cycle cyclopropanique ; ils peuvent posséder également d'autres centres d'asymétrie dans la partie alcool.

Lorsque A représente un radical alcoyle, il s'agit de préférence du radical méthyle, éthyle, n-propyle, isopropyle ou n-butyle.

Lorsque A représente un radical benzyle substitué par un ou plusieurs radicaux alcoyle, il s'agit de préférence des radicaux méthyle ou éthyle.

Lorsque A représente un radical benzyle substitué par un ou plusieurs radicaux alcényle, il s'agit de préférence de radicaux vinyle, allyle, 2-méthylallyle, ou isobutényle.

Lorsque A représente un radical benzyle substitué par un ou plusieurs alcényloxy, il s'agit de préférence de radicaux vinyloxy, allyloxy, 2-méthylallyloxy ou isobutényloxy.

Lorsque A représente un radical benzyle substitué par un ou plusieurs atomes d'halogène, il s'agit de préférence d'atomes de chlore, de brome ou de fluor.

Lorsque R représente un radical alcoyle saturé linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle ou néopentyle.

Lorsque R représente un radical cyclique, il s'agit de préférence d'un radical cyclopropyle, cyclobutyle, cyclopentyle ou cyclohexyle, d'un radical alcoyle linéaire ou ramifié portant l'un de ces radicaux, ou d'un radical cyclopropyle, cyclobutyle, cyclopentyle ou cyclohexyle substitué par un ou plusieurs radicaux alcoyles dont la liaison avec le groupement —COO— est située sur l'un quelconque de ses sommets, par exemple le radical 1 méthylcyclobutyle, 1 méthylcyclopentyle, 1-méthylcyclohexyle ou 2,2,3,3, tétraméthylcyclopropyle.

Lorsque R représente un radical alcoyle insaturé, il s'agit d'un radical éthylénique, comme par exemple d'un radical vinyle, ou 1,1 diméthylallyle, ou d'un radical acétylénique, comme par exemple le radical éthynyle ou propynyle.

L'invention a notamment pour objet, parmi les composés de formule (I'), ceux répondant à la formule (I)

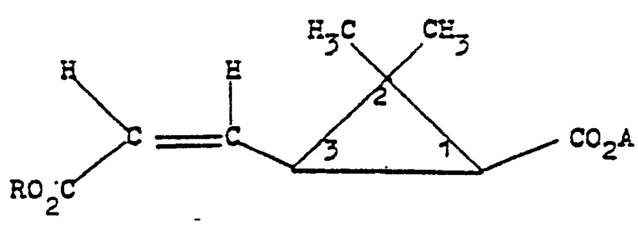

(I)

dans laquelle la double liaison a la géométrie Z et la copule cyclopropanique est de structure IR cis, A représente,

soit un radical alcoyle renfermant de 1 à 18 atomes de carbone,

soit un radical benzyle éventuellement substitué tel que défini précédemment,

soit un groupement

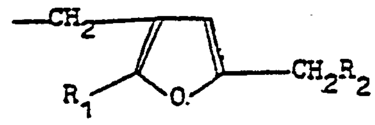

dans lequel les substituants $R_1$ et $R_2$ sont tels que définis précédemment, soit un groupement

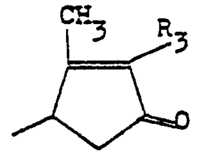

dans lequel $R_3$ est tel que défini précédemment, soit un groupement

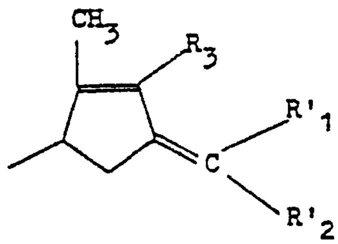

dans lequel $R_3$, $R_1'$ et $R_2'$ conservent les mêmes significations que précédemment, soit un groupement

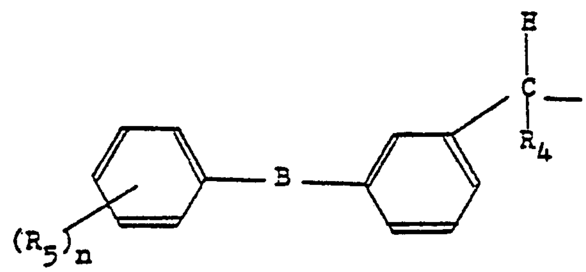

dans lequel B, $R_4$, $R_5$ et n conservent les mêmes significations que précédemment, soit un groupement

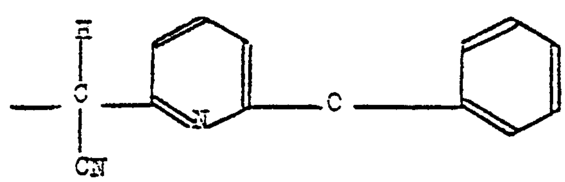

soit un groupement

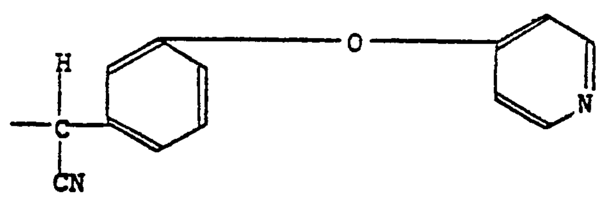

soit un groupement

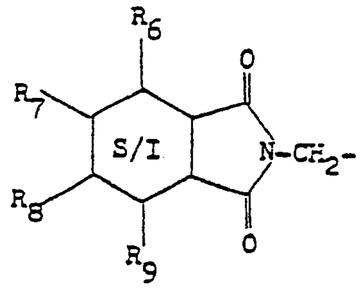

dans lequel les substituants $R_6$, $R_7$, $R_8$, $R_9$ et S/I sont définis comme précédemment,
soit un groupement

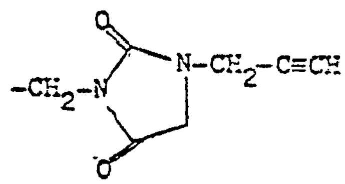

et R est défini comme précédemment, ainsi que les mélanges de ces isomères.

Parmi les composés de l'invention, on peut citer tout particulièrement les composés pour lesquels R représente un radical méthyle, on peut citer également ceux pour lesquels R représente un radical éthyle, n-propyle, isopropyle, tert-butyle ou cyclopropylméthyle.

On peut citer également tout spécialement les composés pour lesquels A représente un groupement (4S) 3-méthyl 2-(2-propenyl) 1-oxo cyclopent-2-en-4-yl. On peut citer encore les composés pour lesquels A représente un groupement (1,3,4,5,6,7 hexahydro-1, 3-dioxo-2H-isoindol-2-yl) méthyle, un groupement (RS) cyano (6-phénoxy-2-pyridinyl) méthyle, un groupement [5-(phénylméthyl)-3-furanyl] méthyle, un groupement 1-(3-propargyl 2,5-dioxo imidazolidinyl) méthyle ou un groupement (R) 3-phénoxyphényl éthyle.

L'invention a notamment pour objet les composés dont la préparation est donnée dans la partie expérimentale. Parmi ces composés de l'invention, on peut citer tout particulièrement :

— le (1R cis) 2,2-diméthyl-3 [(Z) 3-méthoxy-3-oxo-1-propényl] cyclopropane carboxylate de (1S) 2-méthyl-4-oxo 3-(2-propényl) 2-cyclopenten-1-yle ;

— le (1R cis) 2,2-diméthyl-3 [(Z) 3-tert-butoxy-3-oxo-1-propényl] cyclopropane carboxylate de 1-(3-propargyl 2,5-dioxo imidazolidinyl) méthyle ;

— le (1R cis) 2,2-diméthyl-3 [(Z) 2-cyclopropylméthoxycarbonyléthényl] cyclopropane carboxylate de 1-(3-propargyl 2,5-dioxo imidazolidinyl) méthyle ;

— le (1R cis) 2,2-diméthyl-3 [(Z) 2-isopropoxycarbonyl éthényl] cyclopropane carboxylate de 1(R) 3-phénoxyphényl éthyle ;

— le (1R cis) 2,2-diméthyl-3 [(Z) 2-isopropoxy carbonyl éthényl] cyclopropane carboxylate de 1-(3-propargyl 2,5-dioxo imidazolidinyl) méthyle ;

Les composés de formule (I') présentent d'intéressantes propriétés qui permettent leur utilisation dans la lutte contre les parasites : il peut s'agir par exemple de la lutte contre les parasites des végétaux, les parasites domestiques et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I'), tels que définis précédemment, à la lutte contre les parasites des végétaux, les parasites domestiques et les parasites des animaux à sang chaud.

Les produits de formule (I') peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I') peuvent également être utilisés pour lutter contre les insectes dans le domaine domestique, pour lutter notamment contre les mouches, les moustiques et les blattes.

Certains des produits de formule (I') présentent en particulier un excellent pouvoir de knock down. Le produit de l'exemple 1 est particulièrement remarquable.

Les produits de formule (I') sont de plus photostables et ne sont pas toxiques pour les mammifères.

L'ensemble de ces propriétés fait des produits de formule (I') des produits qui correspondent parfaitement aux exigences de l'industrie agrochimique moderne : ils permettent de protéger les récoltes tout en préservant l'environnement.

Les produits de formule (I') peuvent aussi être utilisés pour lutter contre les acariens et les nématodes parasites des végétaux.

Les composés de formule (I') peuvent encore être utilisés pour lutter contre les acariens parasites

des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites domestiques et les parasites des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

L'invention a tout spécialement pour objet les compositions renfermant comme principe actif :

— le (1R cis) 2,2-diméthyl-3 [(Z) 3-méthoxy-3-oxo-1-propényl] cyclopropane carboxylate de (1S) 2-méthyl-4-oxo 3-(propényl)-2-cyclopenten-1-yle ;

— le (1R cis) 2,2-diméthyl-3 [(Z) 3-tert-butoxy 3-oxo 1-propényl] cyclopropane carboxylate de 1-(3-propargyl 2,5-dioxo imidazolidinyl) méthyle ;

— le (1R cis) 2,2-diméthyl-3 [(Z) 2-cyclopropylméthoxy carboxyléthényl] cyclopropane carboxylate de 1-(3-propargyl 2,5-dioxo imidazolidinyl) méthyle ;

— le (1R cis) 2,2-diméthyl-3 [(Z) 2-isopropoxycarbonyl éthényl] cyclopropane carboxylate de 1(R) 3-phénoxyphényl éthyle ;

— le (1R cis) 2,2-diméthyl-3 [(Z) 2-isopropoxycarbonyl éthényl] cyclopropane carboxylate de 1-(3-propargyl 2,5-dioxo imidazolidinyl) méthyle ;

Les compositions selon l'invention sont préparées selon les procédés usuels de l'industrie agrochimique, de l'industrie vétérinaire ou de l'industrie des produits destinés à la nutrition animale.

Dans ces compositions destinées à l'usage agricole et domestique le ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre tel que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage domestique, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention sur un usage domestique peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire contenant de 1 à 80 % ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5-8-trioxadodecyl) 2-propyl 4,5-méthylènedioxy benzène

(ou butoxyde de pipéronyle) ou la N-(2-éthyl heptyl) bicyclo [2,2-1]-5-heptène-2,3-di carboximide, ou le pipéronyl-bis-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions alimentaires définies ci-dessus.

Les composés de formule (I') présentent une excellente tolérance générale, et l'invention a donc également pour objet les produits de formule (I'), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale. Ils peuvent également être utilisés comme anthelmintiques.

Les médicaments selon l'invention peuvent être administrés par voie externe, par vaporisation, par bain ou badigeonnage.

Les médicaments selon l'invention, à usage vétérinaire, peuvent être également administrés par badigeonnage de l'épine dorsale selon la méthode dite méthode « pour-on ». Ils peuvent être également administrés par voie digestive ou parentérale.

L'invention a donc pour objet les compositions pharmaceutiques renfermant comme principe actif au moins l'un des médicaments définis précédemment.

On peut indiquer également que les produits de l'invention peuvent être utilisés comme biocides ou comme régulateur de croissance.

L'invention a également pour objet les associations douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I' peuvent exister sous toutes leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites, plus étendue, soit de manifester, dans certains cas, un effet de synergie.

L'invention a également pour objet un procédé de préparation des composés de formule (I'), caractérisé en ce que l'on soumet un acide de formule (II)

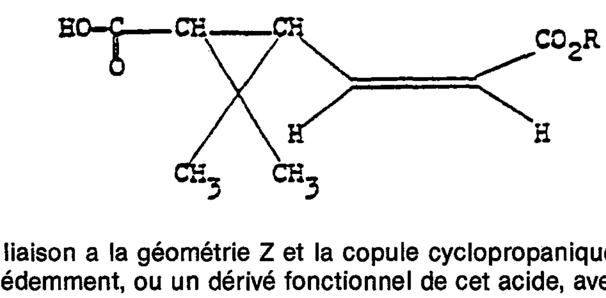

(II)

dans laquelle la double liaison a la géométrie Z et la copule cyclopropanique est de structure IR cis, R étant défini comme précédemment, ou un dérivé fonctionnel de cet acide, avec un alcool de formule (III)

$$A'—OH \qquad (III)$$

dans laquelle A' conserve la même signification que précédemment et obtient ainsi le composé de formule (I') correspondant.

Le dérivé fonctionnel d'acide utilisé est de préférence un chlorure d'acide.

La réaction d'estérification peut être réalisée selon d'autres procédés. On peut par exemple, faire

réagir l'acide de formule (II) avec l'alcool de formule (III) en présence de dicyclohexylcarbodiimide ou de diisopropylcarbodiimide.

Les composés de formule (II) sont des produits chimiques nouveaux dont des exemples de préparations sont donnés plus loin dans la partie expérimentale.

Les composés de formule (II) peuvent être préparés par un procédé selon lequel l'on fait réagir un composé de formule

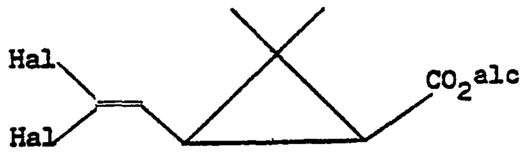

(IV)

dans laquelle Hal représente un atome d'halogène et alc représente un radical alcoyle renfermant de 1 à 20 atomes de carbone, dans un premier temps avec un agent alcalin capable d'arracher les atomes d'halogène et un deuxième temps

soit avec un agent capable d'introduire le groupement carboxylique pour obtenir le composé de formule (V)

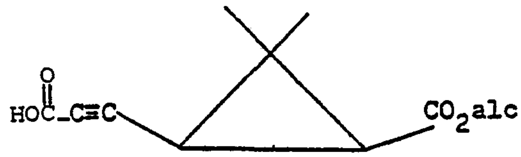

(V)

que l'on soumet à l'action d'un agent d'estérification, pour obtenir le composé de formule

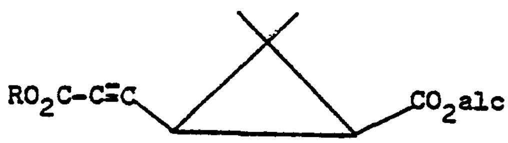

(VI)

dans lequel R conserve la même signification que précédemment

soit avec un chloroformiate d'alcoyle de formule

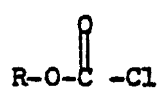

(V')

dans laquelle R conserve la signification précédente, pour obtenir directement le composé de formule (VI)

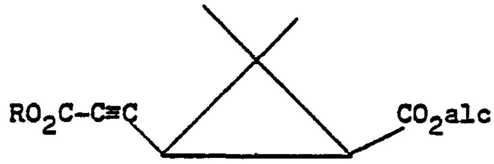

(VI)

puis soumet le composé de formule (VI) à l'action d'un agent d'hydrogénation ménagée, pour obtenir le composé de formule

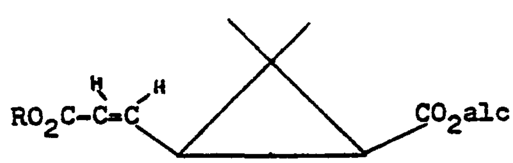

(VII)

dans laquelle le groupement $CO_2R$ est en position Z, que l'on soumet à l'action d'un agent d'hydrolyse acide capable de cliver sélectivement la fonction ester branchée sur le carbone en 1, pour obtenir le composé de formule (II) correspondant

(Voir formule II, page 11)

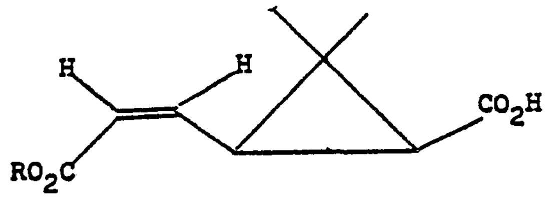

(II)

L'invention a également pour objet un procédé de préparation des composés de formule (I'), caractérisé en ce que l'on soumet un composé de formule (VIII)

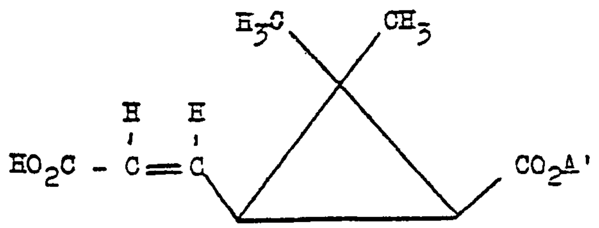

(VIII)

dans lequel A' est défini comme précédemment, à l'action d'un agent d'estérification, pour obtenir le composé de formule (I') correspondant.

L'invention a également pour objet un procédé tel que défini précédemment, caractérisé en ce que le composé de formule (VIII) est préparé en soumettant un composé de formule (IX)

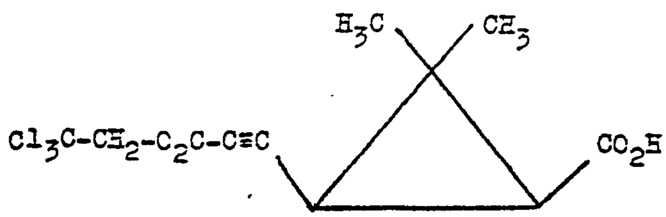

(IX)

à l'action d'un alcool de formule (III)

A'—OH (III)

dans lequel A' est défini comme précédemment, pour obtenir le composé de formule (X)

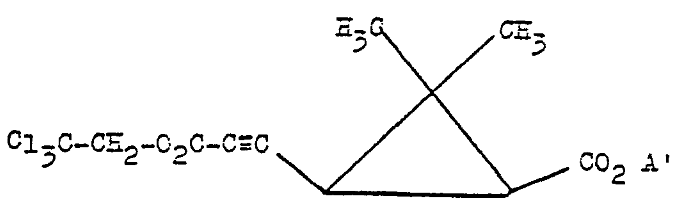

(X)

que l'on soumet à l'action d'un agent de clivage de la fonction ester portée par le carbone acétylénique, pour obtenir le composé de formule (XI)

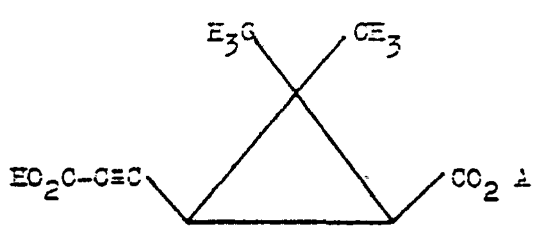

(XI)

que l'on soumet à l'action d'un agent d'hydrogénation ménagée pour obtenir le composé de formule (VIII).

Dans un mode de réalisation préféré du procédé de l'invention, ci-dessus,

— l'estérification du composé (IX) se fait en faisant réagir le composé (IX) avec l'alcool (III), en présence de dicyclohexyl carbodiimide ou de diisopropyle carbodiimide ;

— le clivage de l'ester (X) se fait en utilisant une poudre métallique par exemple, la poudre de zinc, en milieu acide ;

— l'agent d'hydrogénation ménagée est l'hydrogène en présence d'un catalyseur comme le palladium, en présence de traces de quinoléine.

Le procédé ci-dessus comporte une variante évidante selon laquelle les stades d'hydrogénation et d'estérification sont inversés.

L'invention a ainsi également pour objet un procédé tel que défini précédemment, caractérisé en ce que l'on soumet un composé de formule (XI)

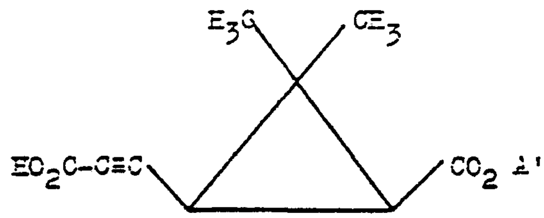

(XI)

dans laquelle A' est défini comme précédemment, à l'action d'un agent d'estérification, pour obtenir le composé de formule (XII)

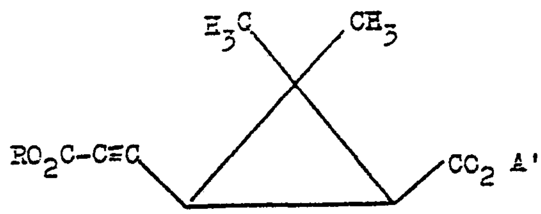

(XII)

dans laquelle R et A' sont définis comme précédemment, que l'on soumet à l'action d'un agent d'hydrogénation ménagée, pour obtenir le composé de formule (I').

Les conditions préférentielles d'exécution du procédé ci-dessus sont identiques à celles qui ont été définies précédemment pour les opérations analogues.

Le composé de formule IX peut être préparé en soumettant un acide de formule (XIII)

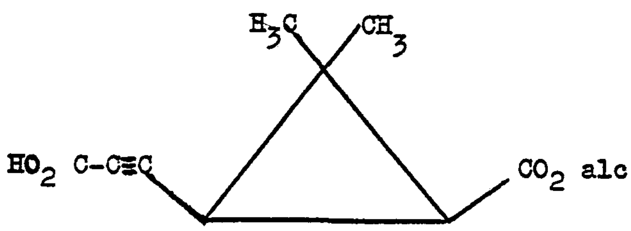

(XIII)

dans laquelle alc représente un radical alcoyle renfermant de 1 à 18 atomes de carbone, à l'action du 2,2,2-trichloroéthanol puis en soumettant l'ester obtenu, à une hydrolyse acide.

Un exemple de préparation d'un composé de formule IX et décrit ci-après dans la partie expérimentale.

Les produits intermédiaires obtenus lors de la mise en œuvre du procédé de l'invention sont des produits chimiques nouveaux et l'invention a donc également pour objet, à titre de produits industriels nouveaux et notamment à titre de produits intermédiaires nécessaires à la mise en œuvre de l'invention, les produits de formule (VIII), (X), (XI) et (XII).

Les exemples suivants illustrent l'invention sans toutefois la limiter.

Exemple 1 : (1R cis, ΔZ) 2,2-diméthyl-3 (3-méthoxy-3-oxo-1-propényl) cyclopropane carboxylate de (1S) 2-méthyl-4-oxo 3-(2-propényl)-2-cyclopenten-1-yle.

Stade A : Chlorure de l'acide (1R cis, ΔZ) 2,2-diméthyl-3 (3-méthoxy 3-oxo 1-propényl) cyclopropane carboxylique.

On introduit à 0 °C 1 $cm^3$ de chlorure de thionyle dans un mélange renfermant 1,8 g d'acide (1R cis, ΔZ) 2,2-diméthyl-3 (2-méthoxy 3-oxo 1-propényl) cyclopropane carboxylique préparé comme indiqué ci-après dans la préparation I et 10 $cm^3$ d'isoprène. On maintient sous agitation pendant 30 minutes à 0 °C, puis à 20 °C pendant 4 heures. On concentre à sec sous pression réduite à 50 °C. On obtient un produit que l'on utilise tel quel dans le stade suivant.

Stade B : (1R cis, ΔZ) 2,2-diméthyl-3 (3-méthoxy-3-oxo-1-propényl) cyclopropane carboxylate de (1S) 2-

méthyl-4-oxo 3-(2-propényl)-2-cyclopenten-1-yle.

On mélange 761 mg de (4S)-hydroxy 3-méthyl 2-(2-propényl) 2-cyclopenten-1-one, 5 $cm^3$ de benzène, 0,4 $cm^3$ de pyridine. On refroidit la solution à 10 °C et ajoute 888 g de chlorure de l'acide (1R cis, ΔZ) 2,2-diméthyl-3 (3-méthoxy 3-oxo-1-propényl) cyclopropane carboxylique, en solution dans 9 $cm^3$ de benzène. On maintient le mélange réactionnel sous agitation pendant 16 heures. On verse dans l'eau, on décante la phase organique et la lave à l'eau. On sèche, filtre et amène à sec. On obtient 1,9 g d'un produit que l'on chromatographie sur silice, éluant benzène acétate d'éthyle (95-5). On obtient ainsi 1,06 g du produit recherché.

$[\alpha]_D = + 71{,}5° \mp 2{,}5°$ (c = 0,5 % $CHCl_3$)
RMN : $CDCl_3$ en ppm
1,3 et 1,32 hydrogène des méthyles en 2,
1,9-2,05 H en 1 du cyclopropane,
3,1-3,3-3,4 H en 3 du cyclopropane,
6,5 à 6,8 H du carbone en 1 du radical éthényle,
5,8-6,04 H du carbone en 2 du radical éthényle,
3,7 H du groupement méthoxy,
5,6 à 5,8 H du carbone en 4 du radical (4S) 3-méthyl 1-oxocyclopent-2-en-4-yle,
2,95 à 3 H du carbone en 1 du radical propényle,
5,5 à 6,2 H du carbone en 2 du radical propényle,
4,8 à 5,2 H du carbone en 3 du radical propényle.

Exemple 2 : (1R cis, ΔZ) 2,2-diméthyl 3-(3-éthoxy-3-oxo-1-propényl) cyclopropane carboxylate de (1S) 2-méthyl-4-oxo-3-(2-propényl) 2-cyclopenten-1-yle.

On introduit 1 g de dicyclohexylcarbodiimide, 6 mg de 4-diméthyl amino pyridine, et 12 $cm^3$ de chlorure de méthylène dans un ballon renfermant 1,1 g d'acide (1R cis, ΔZ) 2,2-diméthyl 3-(3-éthoxy 3-oxo 1-propényl) cyclopropane carboxylique, préparé comme indiqué ci-après dans la préparation 2. On ajoute ensuite 900 mg de (4S) 4-hydroxy 3-méthyl 2-(2-propényl) 2-cyclopenten-1-one en solution dans 1 $cm^3$ de chlorure de méthylène. On maintient le mélange réactionnel sous agitation pendant 16 heures. On filtre et concentre à sec le filtrat sous pression réduite et obtient ainsi 2,4 g d'un produit que l'on purifie par chromatographie sur silice : éluant : n-hexane-éther isopropylique (6-4). On obtient 1,3 g du produit recherché.

$[\alpha]_D = + 34° \mp 2°$ (c = 0,6 % dans le benzène)
RMN : $CDCl_3$ en ppm
1,27 H des méthyles en 2,
3,1 à 3,4 H du carbone en 3 du cyclopropane,
6,4 à 6,8 H du carbone en 1 du radical éthényle,
5,8-6 H du carbone en 2 du radical éthényle,
1,17 à 1,28, 1,4 et 4 à 4,4 H du groupement éthoxy,
4,7 à 5,2 H du carbone en 3 du radical propényle.

Exemple 3 : (1R cis, ΔZ) 2,2-diméthyl-3-(3-oxo-3-propoxy-1-propényl) cyclopropane carboxylate de (1S) 2-méthyl-4-oxo-3-(2-propényl) 2-cyclopenten-1-yle.

En opérant comme à l'exemple 2 à partir de 1,5 g d'acide (1R cis, ΔZ) 2,2-diméthyl 3-(3-oxo-3-propoxy-1-propényl) cyclopropane carboxylique et de 1,1 g de (4S) 4-hydroxy 3-méthyl 2-(2-propényl) 2-cyclopenten-1-one, on obtient 3,1 g du produit recherché que l'on purifie par chromatographie sur silice éluant : hexane-éther isopropylique (7-3). On l'amène à sec et obtient 1,7 g du produit recherché.

$[\alpha]_D = + 39{,}5° \mp 2{,}5°$ (c = 0,5 % dans le benzène)
RMN : $CDCl_3$ en ppm
1,3 et 1,31 H du méthyle en 2 du cyclopropane,
3,1 à 3,42 H porté par le carbone en 3 du cyclopropane,
6,4 à 6,8 H du carbone en 1 du radical éthényle,
5,8-6 H du carbone en 2 du radical éthényle,
4-4,08-4,2 H du radical propoxy en α du $CO_2$,
0,83-0,95-1,06 H du radical propoxy en γ du $CO_2$,
5,6 à 5,7 H du carbone en 4 du radical (4S) 3-méthyl 2-(2-propényl) 1-oxo cyclopent 2-en-4-yle,
2 H du méthyl en 3 du radical (4S) 3-méthyl 2-(2-propényl) 1-oxo cyclopent-2-en-4-yle,
4,7 à 5,2 H du carbone en 3 du radical propényle.

Exemple 4 : (1R cis, ΔZ) 2,2-diméthyl 3-(3-(1-méthyléthoxy)-3-oxo-1-propényl) cyclopropane carboxylate de (1S) 2-méthyl-4-oxo 3-(2-propényl) 2-cyclopenten-1-yle.

Stade A : Chlorure de l'acide (1R cis, ΔZ) 2,2-diméthyl 3-(3-1(1-méthyléthoxy) 3-oxo-1-propényl)

cyclopropane carboxylique.

On agite 4 heures à la température ambiante un mélange de 900 mg d'acide (1R cis, ΔZ) 2,2-diméthyl 3-(3-(1-méthyléthoxy) 3-oxo-1-propényl) cyclopropane carboxylique, 9 $cm^3$ d'isoprène et 1 $cm^3$ de chlorure de thionyle. On amène à sec et obtient 1,4 g d'un produit que l'on utilise tel quel dans le stade suivant.

Stade B : (1R cis, ΔZ) 2,2-diméthyl 3-(3-(1-méthyl éthoxy) 3-oxo 1-propényl) cyclopropane carboxylate de (1S) 2-méthyl-4-oxo-3-(2-propényl) cyclopenten-1-yle.

On ajoute 1,5 $cm^3$ de pyridine, dans un mélange renfermant 1,4 g du produit préparé au stade A, 10 $cm^3$ de benzène et 750 mg de (4S) 4-hydroxy 3-méthyl 2-(2-propényl) cyclopent-2-en-1-one. On maintient le mélange réactionnel sous agitation pendant 3 heures et verse dans un mélange d'eau glacée et d'acide chlorhydrique 2N (100 $cm^3$ et 20 $cm^3$). On extrait la suspension aqueuse à l'acétate d'éthyle. On lave les phases organiques à l'eau jusqu'à neutralité, sèche, filtre et concentre le filtrat à sec sous pression réduite. On obtient 2,3 g d'un produit que l'on chromatographie sur silice, éluant : cyclohexane-acétate d'éthyle (8-2). On obtient ainsi 486 mg du produit recherché.

$[\alpha]_D = +26° \mp 2°$ (c = 0,5 % dans le benzène)

RMN : $CDCl_3$ en ppm

1,28 et 1,32 H des méthyles en 2 du cyclopropane,
1,88-2 H du carbone en 1 du cyclopropane,
3,11 à 3,4 H du carbone en 3 du cyclopropane,
6,4 à 6,8 H du carbone en 1 du radical éthényl,
5,8-6 H du carbone en 2 du radical éthényle,
5 H du carbone de l'isopropyle en α du C = O,
1,3 H des carbones de l'isopropyl en β du CO,
4,8 à 5,2 H du carbone en 3 du radical propényle,
5,7 H du carbone en 4 du radical (4S) 3-méthyl 2-(2-propényl), 1-oxo cyclopent-2-en-4-yl,
2 H du carbone en 3 du radical (4S) 3-méthyl 2-(2-propényl) 1-oxo cyclopent-2-en-4-yl.

Exemple 5 : (1R cis, ΔZ) 2,2-diméthyl 3-(3-méthoxy-3-oxo-1-propényl) cyclopropane carboxylate de (RS) cyano (6-phénoxy-2-pyridinyl) méthyle.

On introduit 4,96 g d'acide (1R cis, ΔZ) 2,2-diméthyl 3-(3-méthoxy 3-oxo 1-propényl) cyclopropane carboxylique dans 75 $cm^3$ de chlorure de méthylène. On ajoute ensuite 2 $cm^3$ de pyridine, puis 5,2 g de dicyclohexyl carbodiimide.

On agite quelques minutes et introduit 5,9 g de (RS) α-hydroxy 6-phénoxy-2-pyridine acétonitrile, puis 0,1 g de 4-diméthylamino pyridine. On maintient le mélange réactionnel pendant une heure sous agitation puis le filtre. On concentre le filtrat sous pression réduite. On chromatographie le résidu sur colonne éluant : cyclohexane acétate — d'éthyle (85-15). On obtient ainsi 7,7 g du produit recherché.

$[\alpha]_D = +59° \mp 2,5°$ (c = 0,5 % $CHCl_3$)

RMN : $CDCl_3$ en ppm

6,3 H porté par le carbone portant le groupement CN,
1,22-1,27 et 1,3 H des méthyles en 2 du cyclopropane,
3,17 à 3,5 H du carbone en 3 du cyclopropane,
3,7 H du groupement méthoxy,
5,8 à 6,0 H éthylénique en α du carboxyle,
6,3 à 6,6 H éthylénique en β du carboxyle,
6,9-7 H porté par les carbones en 3 et 5 de la pyridine,
7,7-7,8-7,9 H porté par le carbone en 4 de la pyridine.

Exemple 6 : (1R cis, ΔZ) 2,2-diméthyle 3-(3-méthoxy 3-oxo-1-propényl) cyclopropane carboxylate de (1,3,4,5,6,7-hexahydro 1,3-dioxo-2H-isoindol-2-yl) méthyle.

En opérant comme à l'exemple 1, stade B, à partir de 1,5 g de chlorure de l'acide (1R cis, ΔZ) 2,2-diméthyl (3-méthoxy carbonyl éthényl) cyclopropane carboxylique et de 1,4 g de 1,3,4,5,6,7-hexahydro-2-hydroxyméthyl-1H-isoindole-1,3-(2H) dione, on obtient 3 g d'un produit que l'on chromatographie sur silice, éluant : cyclohexane-acétate d'éthyle (8-2). On amène à sec et obtient 1,86 g du produit recherché.

RMN : $CDCl_3$ en ppm

1,27 et 1,3 H des méthyles en 2 du cyclopropane,
1,78-1,93 H porté par le carbone en 1 du cyclopropane,
3,06-3,53 H porté par le carbone en 3 du cyclopropane,
5,8-6 H } porté par le carbone en 2 du radical éthényle,
6,4 à 6,8 }

3,7 H du méthyle du radical méthoxy,
1,6 à 2 H des carbones 4, 5, 6 et 7 de l'indole,
5,5 H du méthylène en α du $CO_2$ relié au carbone en 1 du cyclopropane.

Exemple 7 : (1R cis, ΔZ) 2,2-diméthyl-3 (3-méthoxy-3-oxo-1-propényl) cyclopropane carboxylate de (5-(phénylméthyl)-3-furanyl) méthyle.

En opérant comme à l'exemple 1 à partir de 3,22 g de chlorure de l'acide (1R cis, ΔZ), 2,2-diméthyl 3-(3-méthoxy-3-oxo-1-propényl) cyclopropane carboxylique et de 2,8 g de 5-(phénylméthyl-Δ-3-furanyl méthanol, on obtient 6,3 g d'un produit que l'on chromatographie sur silice : éluant cyclohexane-acétate d'éthyle (8-2). On obtient ainsi 2,33 g du produit recherché.
$[\alpha]_D = + 48° \mp 1,5°$ (c = 0,7 % $CHCl_3$)
RMN : $CDCl_3$ en ppm
1,26-1,28 H des méthyles en 2 du cyclopropane,
1,86-2,02 H du carbone en 1 du cyclopropane,
3,0 à 3,3 H du carbone en 3 du cyclopropane,
6,4 à 6,8 H du carbone en 1 du radical éthényle,
5,8 -5,9 H du carbone en 2 du radical éthényle,
3,7 H du groupement méthoxy,
4,9 H du groupement $CH_2$ en α du CO relié au carbone en 1 du groupement cyclopropane,
7,3 H du carbone en 2 du radical furanyl,
6 H du carbone en 4 du radical furanyl,
7,2 H aromatiques.

Exemple 8 : (1R cis, ΔZ) 2,2-diméthyl 3-(3-propoxy-3-oxo-1-propényl) cyclopropane carboxylate de [1-(2-propényl) 2,4-dioxo imidazolidin-3-yl] méthyle.

On mélange 1,2 g d'acide (1R cis, ΔZ) 2,2-diméthyl 3-(3-propoxy-3-oxo-1-propényl) cyclopropane carboxylique, 20 $cm^3$ de chlorure de méthylène, 100 $cm^3$ de 4-diméthyl amino pyridine. On introduit ensuite dans la solution ainsi obtenue 1 g de dicyclohexylcarbodiimide. On introduit sous agitation 900 mg de 3-hydroxyméthyl-1-(2-propynyl)-2,4-imidazolidinedione.

On agite pendant 16 heures à 20 °C le mélange réactionnel et filtre. On lave le filtrat à l'acide chlorhydrique N puis à l'eau à neutralité, on sèche et amène à sec sous pression réduite. On obtient une huile que l'on chromatographie sur silice éluant : benzène acétate d'éthyle (8-2). On obtient 1,2 g du produit recherché.
$[\alpha]_D = + 2° \mp 1°$ (c = 0,5 % dans le benzène)
RMN : $CDCl_3$ en ppm
1,25 et 1,28 H des méthyles en 2,
1,83-1,97 H du carbone en 1 du cyclopropane,
3,0 à 3,4 H du carbone en 3 du cyclopropane,
6,4 à 6,7 H du carbone en 1 du radical éthényle,
5,6 -5,8 H du carbone en 2 du radical éthényle,
4-4,1-4,2 H du propoxy en α du C = O,
0,83-0,95-1,07 H du propoxy en α du C = O,
2,33-2,37-2,41 H du carbone en 3 du radical propynyle,
4 H du carbone en 3 du radical 2,5 dioxoimidazol idinyle,
4,2-4,3 H du méthylène en α de la triple liaison.

Exemple 9 : (1R cis, ΔZ) 2,2-diméthyl 3-(3-oxo 3-méthoxy-1-propényl) cyclopropane-1-carboxylate de métaphénoxy benzyle.

Dans 20 $cm^3$ de chlorure de méthylène on introduit 2 g d'acide (1R cis, ΔZ) 2,2-diméthyl 3-(3-oxo 3-méthoxy-1-propényl) cyclopropane-1-carboxylique, 0,9 $cm^3$ de pyridine, 2,5 g de dicyclohexylcarbodiimide, agite pendant 10 minutes, ajoute 2,4 g d'alcool métaphénoxy benzylique, agite pendant 1 heure et 30 minutes, ajoute 40 mg de diméthylaminopyridine, agite pendant 2 heures et 30 minutes, élimine par filtration l'insoluble formé, concentre le filtrat à sec sous pression réduite, chromatographie le résidu sur silice en éluant avec un mélange cyclohexane-acétate d'éthyle 9-1 et obtient 3 g de (1R cis, ΔZ) 2,2-diméthyl 3-(3-oxo 3-méthoxy-1-propényl) cyclopropane-1-carboxylate de métaphénoxy benzyle.
$[\alpha]_D$ : + 48° ± 1,2° (c = 1 % chloroforme)
Analyse : $C_{23}H_{24}O_5$ (380,444)
calculés C % 72,61 H % 6,36
trouvés C % 72,7 H % 6,4

De manière analogue à celle décrite à l'exemple 9, au départ des acides et des alcools appropriés, on a préparé les composés suivants :

Exemple 10 : Le (1R cis, ΔZ) 2,2-diméthyl 3(3-méthoxy 3-oxo-1-propényl) cyclopropane-1-carboxylate d'α (RS) cyano [3-(4-bromophénoxy) phényl] méthyle.

Analyse : ($C_{24}H_{22}BrNO_5$) (484,342)
calculés C % 59,51 H % 4,58 Br % 16,50 N % 2,89
trouvés C % 59,7 H % 4,6 Br % 16,2 N % 2,8
Spectre de RMN ($CDCl_3$)
— Pics de 1,26 à 1,34 p.p.m. attribués aux hydrogènes des méthyles germinés.
— Pics à 1,97-2,06 et de 3,22 à 3,48 p.p.m. attribués aux hydrogènes en position 1 et 3 du cyclopropyle.
— Pics à 3,7-3,73 p.p.m. attribués aux hydrogènes de $CO_2$—$CH_3$.
— Pics à 5,85-5,98 et 5,9-6,03 p.p.m. attribués aux hydrogènes éthyléniques (côté ester).
— Pic à 6,33 p.p.m. attribué à l'hydrogène de —CH—CN.
— Pics de 6,43 à 6,67 p.p.m. attribués aux hydrogènes éthyléniques (côté cyclopropyle).
— Pics de 6,85 à 7,52 p.p.m. attribués aux hydrogènes du bromophényle.
— Pics de 6,94 à 7,55 p.p.m. attribués aux hydrogènes de l'autre noyau aromatique.

Exemple 11 : Le (1R cis, ΔZ) 2,2-diméthyl 3-(3-méthoxy 3-oxo 1-propényl) cyclopropane-1-carboxylate de 4-oxo 4 (H) pyran 3-yle F = 106 °C.

$[\alpha]_D$ : + 153° ± 2,5° (c = 0,8 %, benzène).

Exemple 12 : Le (1R cis, ΔZ) 2,2-diméthyl 3-(3-isopropoxy 3-oxo 1-propényl) cyclopropane-1-carboxylate de 1 (R) (3-phénoxyphényl) éthyle.

$[\alpha]_D$ : + 140° ± 2,5° (c = 1 %, benzène).

Exemple 13 : Le (1R cis, ΔZ) 2,2-diméthyl-3-(3-éthoxy 3-oxo 1-propényl) cyclopropane-1 carboxylate de 1 (R) (3-phénoxyphényl) éthyle.

$[\alpha]_D$ : + 145° ± 2,5° (c = 1 %, benzène).

Exemple 14 : Le (1R cis, ΔZ) 2,2-diméthyl 3-(3-méthoxy 3-oxo 1-propényl) cyclopropane-1-carboxylate de 1 (R) (3-phénoxyphényle) éthyle.

$[\alpha]_D$ : + 130,5° ± 2,5° (c = 1 %, chloroforme).

Exemple 15 : Le (1R cis, ΔZ) 2,2-diméthyl 3-(3-méthoxy 3-oxo 1-propényl) cyclopropane-1-carboxylate de (RS) α-cyano (3-benzoylphényl) méthyle.

$[\alpha]_D$ : + 43° ± 1° (c = 1 %, toluène).

Exemple 16 : Le (1R cis, ΔZ) 2,2-diméthyl 3-(3-méthoxy 3-oxo 1-propényl) cyclopropane-1-carboxylate de (3-benzoylphényl) méthyle.

$[\alpha]_D$ : + 52° ± 1,5° (c = 1 %, chloroforme).

Exemple 17 : Le (1R cis, ΔZ) 2,2-diméthyl 3-(3-méthoxy 3-oxo 1-propényl) cyclopropane-1-carboxylate de (2,3,4,5,6 pentafluorophényl) méthyle.

$[\alpha]_D$ : + 29,5° ± 2° (c = 0,8 %, chloroforme).

Exemple 18 : Le (1R cis, ΔZ) 2,2-diméthyl 3-(3-méthoxy 3-oxo 1-propényl) cyclopropane-1-carboxylate de (RS) cyano (2,3,4,5,6 pentafluorophényl) méthyle.

Analyse : $C_{18}H_{14}F_5NO_4$ (403,31)
calculés C % 53,61 H % 3,50 N % 3,47 F % 23,55
trouvés C % 53,9 H % 3,5 N % 3,4 F % 23,7

Exemple 19 : Le (1R cis, ΔZ) 2,2-diméthyl-3 (3-oxo 3-n propoxy 1-propényl) cyclopropane-1-carboxylate de (RS) cyano 2-(6-phénoxy pyridyl) méthyle.

$[\alpha]_D$ : + 55° ± 2,5° (c = 0,5 %, chloroforme).

Exemple 20 : Le (1R cis, ΔZ) 2,2-diméthyl 3-(3-propoxy 3-oxo 1-propényl) cyclopropane-1-carboxylate de 1R (3-phénoxyphényle) éthyle.

[α]$_D$ : + 123° ± 2° (c = 0,9 %, chloroforme).

Exemple 21 : Le (1R cis, ΔZ) 2,2-diméthyl 3-(3-méthoxy 3-oxo 1-propényl cyclopropane-1-carboxylate de 1 (R) (3-phénoxyphényl) 2-propyn-1-yle.

[α]$_D$ : + 55,5° ± 1,5° (c = 1 %, chloroforme).

Exemple 22 : Le (1R cis, ΔZ) 2,2-diméthyl 3-(3-isopropoxy 3-oxo 1-propényl) cyclopropane-1-carboxylate de 1 (R) (3-phénoxyphényl) 2-propyn-1-yle F = 66 °C.

[α]$_D$ : + 47° ± 1° (c = 1,2 %, chloroforme).

Exemple 23 : Le (1R cis, ΔZ), 2,2-diméthyl 3-(3-n-propoxy 3-oxo 1-propényl) cyclopropane-1-carboxylate de 1R (3-phénoxyphényl) 2-propyn-1-yle.

[α]$_D$ : + 48° ± 2° (c = 1 %, chloroforme).

Exemple 24 : Le (1R cis, ΔZ) 2,2-diméthyl 3-(3-méthoxy 3-oxo 1-propényl) cyclopropane-1-carboxylate de (4-benzoyl phényl) méthyle.

[α]$_D$ : + 46° ± 2° (c = 1 %, chloroforme).

Exemple 25 : Le (1R cis, ΔZ) 2,2-diméthyl 3-(oxo 3-terbutoxy 1-propényl) cyclopropane-1-carboxylate de (RS) cyano 2-(6-phénoxy pyridyl) méthyle.

[α]$_D$ : + 68° ± 1,5° (c = 1 %, chloroforme).

Exemple 26 : Le (1R cis, ΔZ), 2,2-diméthyl 3-(3-méthoxy 3-oxo 1-propényl) cyclopropane-1-carboxylate de (RS) cyano (4-benzoyl phényl) méthyle.

Analyse : $C_{25}H_{23}O_5N$ (417,466).

| | | | |
|---|---|---|---|
| calculés | C % 71,93 | H % 5,85 | N % 3,35 |
| trouvés | C % 71,9 | H % 5,8 | N % 3,2 |

Exemple 27 : Le (1R cis, ΔZ) 2,2-diméthyl 3-(3-oxo 3-terbutoxy 1-propényl) cyclopropane-1-carboxylate de (3-propyn-2-yl 2,5-dioxoimidazolidinyl) méthyle.

[α]$_D$ : + 31° ± 2° (c = 0,5 %, chloroforme).

Exemple 28 : le (1R cis, ΔZ) 2,2-diméthyl 3-(3-isopropoxy 3-oxo 1-propényl) cyclopropane-1-carboxylate de (3-propyn 2-yl 2,5-dioxoimidazolidinyl) méthyle F = 78 °C.

[α]$_D$ : 15,5° ± 1° (c = 1 %, chloroforme).

Exemple 29 : Le (1R cis, ΔZ) 2,2-diméthyl 3-(3-cyclobutoxy 3-oxo 1-propényl) cyclopropane-1-carboxylate de (3-propyn 2-yl 2,5-dioxoimidazolidinyl) méthyle.

[α]$_D$ : + 18° ± 1° (c = 1,2 % chloroforme).

Exemple 30 : Le (1R cis, ΔZ) 2,2-diméthyl 3-(3-isobutoxy 3-oxo 1-propényl) cyclopropane-1-carboxylate de (3-propyn 2-yl 2,5-dioxoimidazolidinyl) méthyle.

[α]$_D$ : + 20,5° ± 1,5° (c = 1 %, chloroforme).

Exemple 31 : Le (1R cis, ΔZ) 2,2-diméthyl 3-(3-oxo 3-éthoxy 1-propényl) cyclopropane-1-carboxylate de (3-propyn-2 yl 2,5-dioxoimidazolidinyl) méthyle.

[α]$_D$ : + 10° ± 4° (c = 0,2 %, chloroforme).

Exemple 32 : Le (1R cis, ΔZ) 2,2-diméthyl 3-(3-éthoxy 3-oxo 1-propényl cyclopropane-1-carboxylate de 1 (R) (3-phénoxyphényl) 2-propyn-1-yle.

$[\alpha]_D$ : + 48,5° ± 2° (chloroforme).

Exemple 33 : Le (1R cis, ΔZ) 2,2-diméthyl 3-(3-oxo 3-isopropoxy 1-propényl) cyclopropane-1-carboxylate de (5-benzyl 3-furyl) méthyle.

$[\alpha]_D$ : + 44° + 2° (c = 0,4 %, chloroforme).

Exemple 34 : Le (1R cis, ΔZ) 2,2-diméthyl 3-(3-oxo 3-isopropoxypropényl) cyclopropane-1-carboxylate de métaphénoxy benzyle.

$[\alpha]_D$ : + 42° ± 2° (c = 0,5 %, chloroforme).

Exemple 35 : Le (1R cis, ΔZ) 2,2-diméthyl 3-(3-oxo 3(cyclopropyl méthoxy) propényl) cyclopropane-1-carboxylate de (RS) cyano 2-(6-phénoxy pyridyl) méthyle.

$[\alpha]_D$ : + 53° ± 2° (c = 0,5 %, chloroforme).

Exemple 36 : Le (1R cis, ΔZ) 2,2-diméthyl 3-(3-oxo 3-n-propoxy propényl) cyclopropane-1-carboxylate de (5-benzyl 3-furyl) méthyle.

$[\alpha]_D$ : + 41° ± 2° (c = 0,5 %, chloroforme).

Exemple 37 : Le (1R cis, ΔZ) 2,2-diméthyl-3 (3-oxo 3-isopropoxy propényl) cyclopropane-1-carboxylate de 1,3,4,5,6,7-hexahydro 1,3-dioxo 2H isoindol-2-yl) méthyle F = 94 °C.

$[\alpha]_D$ : − 22,5° ± 2° (c = 0,5 %, chloroforme).

Exemple 38 : (1R cis, ΔZ) 2,2-diméthyl 3-(3-oxo 3-n propoxy 1-propényl) cyclopropane-1-carboxylate de (1,3,4,5,6,7-hexahydro 1,3-dioxo 2H isoindol-2 yl) méthyle F = 76 °C.

$[\alpha]_D$ : − 15° (c = 0,15 %, tétrachlorure de carbone).

Exemple 39 : Le (1R cis, ΔZ) 2,2-diméthyl 3-(3-oxo 3-n-propoxy 1-propényl) cyclopropane 1-carboxylate de métaphénoxy benzyle.

$[\alpha]_D$ : + 40° ± 2° (c = 0,5 %, chloroforme).

Exemple 40 : Le (1R cis, ΔZ) 2,2-diméthyl 3-(3-oxo 3-cyclopropylméthoxy-1-propényl) cyclopropane-1-carboxylate de métaphénoxy benzyle.

$[\alpha]_D$ : + 46,5° ± 2° (c = 0,6 %, chloroforme).

Exemple 41 : Le (1R cis, ΔZ) 2,2-diméthyl 3-(3-cyclobutoxy 3-oxo 1-propényl) cyclopropane-1-carboxylate de 1 (R) (3-phénoxyphényl) éthyle.

$[\alpha]_D$ : + 125,5° ± 2° (c = 1 %, chloroforme).

Exemple 42 : Le (1R cis, ΔZ) 2,2-diméthyl 3-(3-oxo 3-(cyclopropyl) méthoxy 1-propényl) cyclopropane-1-carboxylate de (1,3,4,5,6,7-hexahydro 1,3-dioxo 2H isoindol 2-yl) méthyle F : 102 °C.

$[\alpha]_D$ : + 6,5° ± 2° (c = 0,3 %, chloroforme).

Exemple 43 : Le (1R cis, ΔZ) 2,2-diméthyl 3-(3-oxo 3-(cyclopropyl méthoxy) 1-propényl) cyclopropane-1-carboxylate de (5-benzyl 3-furyl) méthyle.

$[\alpha]_D$ : + 42° ± 2° (c = 0,7 %, chloroforme).

Exemple 44 : Le (1R cis, ΔZ) 2,2-diméthyl 3-(3-oxo 3-(cyclopropyl méthoxy) 1-propényl) cyclopropane-1-carboxylate de (S) 2-méthyl 4-oxo 3-(2-propényl) 2-cyclopentényle.

$[\alpha]_D$ : + 64,5° ± 2,5° (c = 1 %, chloroforme).

Exemple 45 : Le (1R cis, ΔZ) 2,2-diméthyl 3-(3-cyclopentyloxy 3-oxo 1-propényl) cyclopropane-1-carboxylate de (3-propyn 2-yl 2,5-dioxoimidazolidinyl) méthyle.

[α]$_D$ : + 15,5° ± 1,5° (c = 1 %, chloroforme).

Exemple 46 : Le (1R cis, ΔZ) 2,2-diméthyl 3-(3-oxo 3-cyclopentyloxy 1-propényl) cyclopropane-1-carboxylate de (RS) cyano 2-(6-phénoxy-6-pyridinyl) méthyle.

[α]$_D$ : + 42° ± 1° (c = 0,9 %, benzène).

Exemple 47 : Le (1R cis, ΔZ) 2,2-diméthyl 3(3-méthoxy 3-oxo 1-propényl) cyclopropane 1-carboxylate de (3-propyn-2 yl 2,5-dioxoimidazolidinyl) méthyle.

[α]$_D$ : ≃ + 1° (c = 0,5 %, benzène).

Exemple 48 : Le (1R cis, ΔZ) 2,2-diméthyl 3(3-sec-butoxy 3-oxo 1-propényl) cyclopropane-1-carboxylate de (3-propyn 2-yl 2,5-dioxoimidazolidinyl) méthyle.

[α]$_D$ : + 16,5° ± 1° (c = 0,8 %, chloroforme).

Exemple 49 : Le (1R cis, ΔZ) 2,2-diméthyl 3(3-méthoxy 3-oxo 1-propényl) cyclopropane-1-carboxylate de (S) cyano 3-phénoxy 4-fluorophényl méthyle.

[α]$_D$ : + 56,5° ± 2,5° (c = 0,5 %, benzène).

Exemple 50 : Le (1R cis, ΔZ) 2,2-diméthyl 3(3-méthoxy-3-oxo-1-propényl) cyclopropane-1-carboxylate de (R,S) 1-(4-fluoro-3-phénoxyphényl)-2-propyn 1-yle.

Spectre de RMN ($CDCl_3$)

— Pics de 1,22 à 1,32 p.p.m. attribués aux hydrogènes des méthyles géminés.
— Pics à 1,09-2,05 et de 3,1 à 3,43 p.p.m. attribués aux hydrogènes en position 1 et 3 du cyclopropyle.
— Pics de 2,62 à 2,66 p.p.m. attribués à l'hydrogène du = CH.
— Pics à 3,72-3,73 p.p.m. attribués aux hydrogènes du $CO_2—CH_3$.
— Pics de 5,8 à 6 p.p.m. attribués à l'hydrogène éthylénique (côté ester).
— Pics de 6,38 à 6,83 p.p.m. attribués à l'hydrogène éthylénique (côté cyclopropyle) et à l'hydrogène du $CO_2—CH_2$.
— Pics de 6,9 à 7,55 p.p.m. attribués aux hydrogènes de noyaux aromatiques.

Exemple 51 : Le (1R cis, ΔZ) 2,2-diméthyl 3(3(1,1-diméthyléthoxy)-3-oxo-1-propényl) cyclopropane-1-carboxylate de (RS) 1(4-fluoro-3-phénoxyphényl) 2-propyn 1-yle.

Spectre de RMN ($CDCl_3$)

— Pics de 1,22 à 1,31 p.p.m. attribués aux hydrogènes des méthyles géminés
— Pics à 1,47-1,5 p.p.m. attribués aux hydrogènes du terbutyle
— Pics à 1,87-2,01 et de 3,12 à 3,43 p.p.m. attribués aux hydrogènes en position 1 et 3 du cyclopropyle
— Pics de 2,6 à 2,65 p.p.m. attribués à l'hydrogène du = CH
— Pics à 5,7-5,9 p.p.m. attribués à l'hydrogène éthylénique (côté ester)
— Pics de 6,28 à 6,72 p.p.m. attribués à l'hydrogène éthylénique (côté cyclopropyle) et à l'hydrogène du $CO_2$-CH
— Pics de 6,91 à 7,55 p.p.m. attribués aux hydrogènes des noyaux aromatiques.

Exemple 52 : Le (1R cis, ΔZ) 2,2-diméthyl 3(3-méthoxy-3-oxo-1-propényl) cyclopropane-1-carboxylate de (R,S) 1-(6-phénoxy-2-pyridinyl) 2-propyn 1-yle

Spectre de RMN ($CDCl_3$)

— Pics de 1,23 à 1,31 p.p.m. attribués aux hydrogènes des méthyles géminés
— Pics à 1,96-2,1 et de 3,13 à 3,47 p.p.m. attribués aux hydrogènes en position 1 et 3 du cyclopropyle
— Pics de 2,59 à 2,63 p.p.m. attribués à l'hydrogène du = CH
— Pic à 3,73 p.p.m. attribué aux hydrogènes de $CO_2CH_3$
— Pics de 5,78 à 6,02 p.p.m. attribués à l'hydrogène éthylénique (côté ester)
— Pics à 6,3-6,34 p.p.m. attribués à l'hydrogène du $CO_2—CH$
— Pics de 6,43 à 6,88 p.p.m. attribués à l'hydrogène éthylénique (côté cyclopropyle)
— Pics de 6,75 à 7,9 p.p.m. attribués aux hydrogènes des noyaux aromatiques.

Exemple 53 : Le (1R cis, ΔZ) 2,2-diméthyl 3(3-(1,1-diméthyl éthoxy) 3-oxo 1-propényl) cyclopropane-1-carboxylate de (R,S) 1-(6-phénoxy 2-pyridinyl) 2-propyn-1-yle

Spectre de RMN ($CDCl_3$)

— Pics de 1,24 à 1,32 p.p.m. attribués aux hydrogènes des méthyles géminés
— Pic à 1,5 p.p.m. attribué aux hydrogènes de terbutyle
— Pics à 1,93-2,07 et de 3,13 à 3,46 p.p.m. attribués aux hydrogènes en position 1 et 3 du cyclopropyle
— Pics de 2,59 à 2,63 p.p.m. attribués à l'hydrogène du ≡CH
— Pics de 5,68 à 5,90 p.p.m. attribués à l'hydrogène éthylénique (côté ester)
— Pics à 6,3-6,34 p.p.m. attribués à l'hydrogène de $CO_2-CH_2$
|
— Pics de 6,47 à 6,86 p.p.m. attribués à l'hydrogène éthylénique (côté cyclopropyle)
— Pics de 6,75 à 7,9 p.p.m. attribués aux hydrogènes des noyaux aromatiques.

Exemple 54 : Le (1R cis, ΔZ) 2,2-diméthyl 3-(3-(1,1-diméthyl éthoxy) 3-oxo 1-propényl) cyclopropane-1-carboxylate de phényl méthyle

Spectre de RMN ($CDCl_3$)

— Pics de 1,28 à 1,32 p.p.m. attribués aux hydrogènes des méthyles géminés
— Pic à 1,5 p.p.m. attribué aux hydrogènes du terbutyle
— Pics à 1,9-2,04 et de 3,08 à 3,41 p.p.m. attribués aux hydrogènes en position 1 et 3 du cyclopropyle
— Pic à 5,15 p.p.m. attribué aux hydrogènes de $CO_2-CH_2-\Phi$
— Pics à 5,72-5,92 p.p.m. attribués à l'hydrogène éthylénique (côté ester)
— Pics de 6,38 à 6,75 p.p.m. attribués à l'hydrogène éthylénique (côté cyclopropyle)
— Pic à 7,4 p.p.m. attribué aux hydrogènes du noyau aromatique.

Exemple 55 : Le (1R cis, ΔZ) 2,2-diméthyl 3-(3-(1-1-diméthyl éthoxy) 3-oxo 1-propényl) cyclopropane-1-carboxylate de (6-phénoxy 2-pyridinyl) méthyle

Spectre de RMN ($CDCl_3$)

— Pics à 1,3-1,32 p.p.m. attribués aux hydrogènes des méthyles géminés
— Pic à 1,51 p.p.m. attribué aux hydrogènes du terbutyle
— Pics à 1,96-2,11 et de 3,13 à 3,47 p.p.m. attribués aux hydrogènes en position 1 et 3 du cyclopropyle
— Pic à 5,13 p.p.m. attribués aux hydrogènes de $CO_2-CH_2-$
— Pics à 5,73-5,92 p.p.m. attribués à l'hydrogène éthylénique (côté ester)
— Pics de 6,37 à 6,73 p.p.m. attribués à l'hydrogène éthylénique (côté cyclopropyle)
— Pics à 7,58-7,72-7,85 p.p.m. attribués aux hydrogènes du noyau pyridyle.

Exemple 56 : Le (1R cis, ΔZ) 2,2-diméthyl 3-(3-méthoxy-3-oxo-1-propényl) cyclopropane-1-carboxylate de (6-phénoxy-2-pyridynyl) méthyle

Spectre de RMN ($CDCl_3$)

— Pic à 1,3 p.p.m. attribué aux hydrogènes des méthyles géminés
— Pics à 1,98-2,13 et de 3,13 à 3,45 p.p.m. attribués aux hydrogènes en position 1 et 3 du cyclopropyle
— Pic à 3,74 p.p.m. attribué aux hydrogènes de $CO_2-CH_3$
— Pic à 5,12 p.p.m. attribué aux hydrogènes de $CO_2-CH_2-$
— Pics à 5,82-6,02 et de 6,5 à 6,87 p.p.m. attribués aux hydrogènes éthyléniques
— Pics de 7 à 7,85 p.p.m. attribués aux hydrogènes des noyaux aromatiques.

Exemple 57 : Le (1R cis, ΔZ) 2,2-diméthyl 3(3-(1,1-diméthyl éthoxy) 3-oxo 1-propényl) cyclopropane-1-carboxylate de (R,S) 1-(6-phénoxy 2-pyridinyl) éthyle

Spectre de RMN ($CDCl_3$)

— Pics de 1,25 à 1,32 p.p.m. attribués aux hydrogènes des méthyles géminés
— Pics à 1,47-1,57 p.p.m. attribués aux hydrogènes de $CO_2-CH-$
|
$CH_3$

— Pic à 1,5 p.p.m. attribué aux hydrogènes du terbutyle
— Pics à 1,95-2,09 et de 3,12 à 3,43 p.p.m. attribués aux hydrogènes en position 1 et 3 du cyclopropyle
— Pics de 5,63 à 5,97 p.p.m. attribués à l'hydrogène de $CO_2$—$CH_2$
— Pics de 5,67 à 5,92 et de 6,32 à 6,75 p.p.m. attribués aux hydrogènes éthyléniques
— Pics de 6,67 à 7,5 p.p.m. attribués aux hydrogènes des noyaux aromatiques
— Pics de 7,55 à 7,83 p.p.m. attribués aux hydrogènes du noyau pyridyle.

Exemple 58 : Le (1R cis, ΔZ) 2,2-diméthyl 3-(3-méthoxy-3-oxo-1-propényl) cyclopropane-1-carboxylate de (R,S) 1-(6-phénoxy 2-pyridinyl) éthyle

Spectre de RMN ($CDCl_3$)

— Pics de 1,25 à 1,3 p.p.m. attribués aux hydrogènes des méthyles géminés
— Pics de 1,43 à 1,57 p.p.m. attribués aux hydrogènes du $CO_2$—CH—
$CH_3$
— Pics à 1,97-2,11 et de 3,1 à 3,42 p.p.m. attribués aux hydrogènes en position 1 et 3 du cyclopropyle
— Pics de 5,63 à 6,08 p.p.m. attribués à l'hydrogène de $CO_2$—$CH_2$
— Pics de 5,78 à 6,02 et de 6,45 à 6,87 p.p.m. attribués aux hydrogènes éthyléniques
— Pics de 6,67 à 6,8 et de 7 à 7,67 p.p.m. attribués aux hydrogènes des noyaux aromatiques
— Pics de 7,55 à 7,83 p.p.m. attribués aux hydrogènes du pyridyle.

Exemple 59 : Le (1R cis, ΔZ) 2,2-diméthyl 3(3-(1,1-diméthyl éthoxy) 3-oxo 1-propényl) cyclopropane-1-carboxylate de (3-(phényl carbonyl) phényl) méthyle

Analyse : $C_{27}H_{30}O_5$ (434,537)
calculés C % 74,63 H % 6,96
trouvés C % 74,60 H % 7,00

Exemple 60 : Le (1R cis, ΔZ) 2,2-diméthyl 3(3-terbutoxy 3-oxo 1-propényl) cyclopropane-1-carboxylate de 1R (3-phénoxyphényl) 2-propyn-1-yle

$[\alpha]_D$ : + 58° ± 1,5° (c = 1,2 %, chloroforme)

Exemple 61 : Le (1R cis, ΔZ) 2,2-diméthyl 3-(3-cyclopropyl méthoxy 3-oxo 1-propényl) cyclopropane carboxylate de (1R) (3-phénoxyphényl) éthyle

$[\alpha]_D$ : + 121° ± 3° (c = 0,6 %, chloroforme)

Exemple 62 : Le (1R cis, ΔZ) 2,2-diméthyl 3-(3-cyclopropyl méthoxy 3-oxo 1-propényl) cyclopropane carboxylate de (3-propyn 2-yl 2,5-dioxoimidazolidinyl) méthyle

$[\alpha]_D$ : + 14° ± 2° (c = 0,5 %, chloroforme)

Exemple 63 : Le (1R cis, ΔZ) 2,2-diméthyl 3-(3-cyclopropyl méthoxy 3-oxo 1-propényl) cyclopropane carboxylate de (1R) (3-phénoxyphényl) 2-propyn-1-yle

$[\alpha]_D$ : + 46° ± 2° (c = 0,5 %, chloroforme)

Exemple 64 : Le (1R cis, ΔZ) 2,2-diméthyl 3-(3-n-butoxy 3-oxo 1-propényl) cyclopropane carboxylate de (3-propyn 2-yl 2,5-dioxoimidazolidinyl) méthyle

$[\alpha]_D$ : + 17° ± 1° (c = 1 %, chloroforme)

Exemple 65 : Le (1R cis, ΔZ) 2,2-diméthyl 3-(3-éthoxy 3-oxo 1-propényl) cyclopropane carboxylate de (S) cyano (3-phénoxy 4-fluorophényl) méthyle

$[\alpha]_D$ : + 47,5° ± 2° (c = 0,5 %, chloroforme)

Exemple 66 : Le (1R cis, ΔZ) 2,2-diméthyl 3-(3-isopropoxy 3-oxo 1-propényl) cyclopropane carboxylate de (S) cyano (3-phénoxy 4-fluorophényl) méthyle

$[\alpha]_D$ : + 50° ± 2° (c = 0,3 %, chloroforme)

Exemple 67 : Le (1R cis, ΔZ) 2,2-diméthyl 3-(3-cyclopropyl méthoxy 3-oxo 1-propényl) cyclopropane carboxylate de (S) cyano (3-phénoxy 4-fluorophényl) méthyle

$[\alpha]_D$ : + 53° (c = 0,25 %, chloroforme)

Exemple 68 : Le (1R cis, ΔZ) 2,2-diméthyl 3-(3-terbutoxy 3-oxo 1-propényl) cyclopropane carboxylate de (2,3,4,5,6-pentafluorophényl) méthyle

$[\alpha]_D$ : + 37,5° ± 1,5° (c = 1 %, chloroforme)

Exemple 69 : Le (1R cis, ΔZ) 2,2-diméthyl 3-(3-terbutoxy 3-oxo 1-propényl) cyclopropane carboxylate de (3-phénoxy 4-fluorophényl) méthyle

$[\alpha]_D$ : + 55,5° ± 2,5° (c = 0,5 %, chloroforme)

Exemple 70 : Le (1R cis, ΔZ) 2,2-diméthyl 3-(3-méthoxy 3-oxo 1-propényl) cyclopropane carboxylate de (3-phénoxy 4-fluorophényl) méthyle

$[\alpha]_D$ : + 50,5° ± 2,5° (c = 0,75 %, chloroforme)

Exemple 71 : Le (1R cis, ΔZ) 2,2-diméthyl 3-(3-terbutoxy 3-oxo 1-propényl) cyclopropane carboxylate de (1 RS) (3-phénoxy 4-fluorophényl) éthyle

Spectre de RMN ($CDCl_3$)

— Pics de 1,19 à 1,28 p.p.m. attribués aux hydrogènes des méthyles géminés
— Pics à 1,48-1,5 p.p.m. attribués aux hydrogènes du terbutyle
— Pics de 1,41 à 1,53 p.p.m. attribués aux hydrogènes du $CO_2$—CH—
|
$CH_3$
— Pics à 1,85-1,99 et de 3,07 à 3,38 p.p.m. attribués aux hydrogènes en position 1 et 3 du cyclopropyle
— Pics de 5,58 à 6 et de 6,27 à 6,7 p.p.m. attribués aux hydrogènes éthyléniques
— Pics de 6,27 à 6,7 p.p.m. attribués à l'hydrogène de $CO_2$—$CH_2$
|
— Pics de 6,92 à 7,53 p.p.m. attribués aux hydrogènes des noyaux aromatiques.

Exemple 72 : Le (1R cis, ΔZ) 2,2-diméthyl 3-(3-méthoxy 3-oxo 1-propényl) cyclopropane carboxylate de (1 RS) (3-phénoxy 4-fluorophényl) éthyle

Spectre de RMN ($CDCl_3$)

— Pics de 1,19 à 1,29 p.p.m. attribués aux hydrogènes des méthyles géminés
— Pics de 1,41 à 1,54 p.p.m. attribués aux hydrogènes du $CO_2$—CH—
|
$CH_3$
— Pics à 1,87-2,02 et de 3,07 à 3,22 p.p.m. attribués aux hydrogènes en position 1 et 3 du cyclopropyle
— Pics de 5,82 à 6,17 attribués à l'hydrogène de $CO_2$—$CH_2$
|
— Pics de 5,93 à 6,2 et de 6,58 à 7 p.p.m. attribués aux hydrogènes éthyléniques
— Pics de 7,03 à 7,72 p.p.m. attribués aux hydrogènes des noyaux aromatiques.

Exemple 73 : Le (1R cis, ΔZ) 2,2-diméthyl 3-(3-terbutoxy 3-oxo 1-propényl) cyclopropane carboxylate de 5-benzyl 3-furyl méthyle

$[\alpha]_D$ : + 54,5° ± 1,5° (c = 1 %, chloroforme)

Exemple 74 : Le (1R cis, ΔZ) 2,2-diméthyl 3-(3-terbutoxy 3-oxo (1-propényl) cyclopropane carboxylate de (1S) 2-méthyl 4-oxo-3 (2-propényl) cyclopenten-1-yle

$[\alpha]_D$ : + 73° (c = 0,25 %, chloroforme)

Exemple 75 : Le (1R cis, ΔZ) 2,2-diméthyl 3-(3-méthoxy 3-oxo 1-propényl) cyclopropane carboxylate de (2-phénoxy 5-thiazolyl) méthyle. F = 62 °C.

$[\alpha]_D$ : + 60,5° ± 1,5° (c = 1,5 %, benzène)

Exemple 76 : Le (1R cis, ΔZ) 2,2-diméthyl 3-(3-méthoxy 3-oxo 1-propényl) cyclopropane carboxylate de (RS) thioamido phénoxyphényl méthyle

Spectre IR :

— Absorptions à 1 632 $cm^{-1}$ (C = C)
3 475-2 365 $cm^{-1}$ (C = S)
|
$NH_2$
1 713-1 736 $cm^{-1}$ (C = O)

Spectre de RMN (deutérochloroforme)

— Pics à 1,25-1,27-1,30 p.p.m. attribués aux hydrogènes des méthyles géminés
— Pics à 1,97-2,11 p.p.m. attribués à l'hydrogène en position 1 du cyclopropyle
— Pics à 3,08-3,43 p.p.m. attribués à l'hydrogène en position 3 du cyclopropyle
— Pics à 3,69-3,72 p.p.m. attribués aux hydrogènes du méthyle du méthoxy carbonyle
— Pics à 5,73-5,92 p.p.m. et 5,8-5,99 p.p.m. attribués à l'hydrogène éthylénique en α du $CO_2$—$CH_3$
— Pics à 6,32-6,66 p.p.m. attribués à l'hydrogène éthylénique en 1 du —$CO_2$—$CH_3$
— Pic à 6,42 p.p.m. attribué à l'hydrogène lié au carbone porteur du groupement —C—$NH_2$
||
S

Exemple 77 : Le (1R cis, ΔZ) 2,2-diméthyl 3-(3-terbutoxy 3-oxo 1-propényl) cyclopropane carboxylate de (2-phénoxy 5-thiazolyl) méthyle

$[\alpha]_D$ : + 68° ± 1,5° (c = 1 %, chloroforme)

Exemple 78 : Le (1R cis, ΔZ) 2,2-diméthyl 3-(3-méthoxy 3-oxo 1-propényl) cyclopropane carboxylate de (RS) cyano (2-phénoxy 5-thiazolyl) méthyle

$[\alpha]_D$ : + 60° ± 2° (c = 1 %, chloroforme)

Exemple 79 : Le (1R cis, ΔZ) 2,2-diméthyl 3-(3-terbutoxy 3-oxo 1-propényl) cyclopropane carboxylate de (RS) cyano (2-phénoxy 5-thiazolyl) méthyle

$[\alpha]_D$ : + 65° ± 2° (c = 0,5 %, chloroforme)

Exemple 80 : (1R cis, ΔZ) 2,2-diméthyl 3(3-allyloxy 3-oxo 1-propényl) cyclopropane carboxylate de 1(R) (3-phénoxyphényl) éthyle

Stade A : (1R cis) 2,2-diméthyl 3-(3,3,3-trichloroéthoxy 3-oxo propynyl) cyclopropane carboxylate de terbutyle.

On introduit 6,2 g de dicyclohexylcarbodiimide dans une solution renfermant 7,15 g de (1R cis) 2,2-diméthyl 3(3-hydroxy 3-oxo propynyl) cyclopropane carboxylate de terbutyle, préparé comme il a été indiqué à la préparation 2 A, et 80 mg de diméthylaminopyridine dans 35 $cm^3$ de chlorure de méthylène. On agite le mélange réactionnel pendant 10 minutes et ajoute 4,5 g de 2,2,2-trichloroéthanol. On maintient sous agitation pendant une heure et élimine par filtration le précipité formé. On lave le filtrat à l'acide chlorhydrique N puis à l'eau jusqu'à neutralité, le sèche et l'amène à sec. On obtient 14 g d'une huile que l'on chromatographie sur silice en éluant par le mélange benzène acétate d'éthyle (97-3). On isole ainsi 9 g du produit recherché fondant à 70-71 °C.

Stade B : Acide (1R cis) 2,2-diméthyl 3-(3,3,3-trichloroéthoxy-3-oxo propynyl) cyclopropane carboxylique.

On porte au reflux pendant une heure un mélange renfermant 11,4 g du produit préparé selon le stade A, 120 $cm^3$ de toluène et 300 mg d'acide paratoluènesulfonique. On laisse revenir à la température ambiante, lave le mélange réactionnel à l'eau, le sèche, et l'amène à sec. On obtient ainsi 9,5 g du produit recherché que l'on utilise tel quel dans le stade suivant.

Stade C : (1R cis) 2,2-diméthyl 3-(3,3,3-trichloroéthoxy 3-oxo propynyl) cyclopropane carboxylate de 1R (3-phénoxyphényl) éthyle.

On mélange 6 g du produit obtenu selon le stade B, 30 $cm^3$ de chlorure de méthylène, 600 mg de 4-diméthylaminopyridine.

On introduit ensuite dans la solution ainsi obtenue lentement à 0 °C sous agitation 3,95 g de dicyclohexylcarbodiimide, agite encore pendant 30 minutes toujours à 0 °C puis, ajoute goutte à goutte à la même température une solution de 4,1 g de 1 (R) (3-phénoxyphényle) éthanol dans 12 $cm^3$ de chlorure de méthylène.

On agite pendant 17 heures à la température ambiante le mélange réactionnel et filtre. On amène à sec le filtrat sous pression réduite. On obtient une huile que l'on chromatographie sur silice éluant cyclohexane-acétate d'éthyle (8-2). On obtient 4,4 g du produit recherché.

Stade D : (1R cis) 2,2-diméthyl 3-(3-hydroxy 3-oxo propynyl) cyclopropane carboxylate de 1R (3-phénoxyphényl) éthyle.

On introduit sous agitation 0,53 g de zinc en poudre dans une solution de 4,16 g du produit préparé au stade C dans 4 $cm^3$ de chlorure de méthylène et 45 $cm^3$ d'acide acétique. On agite pendant 30 minutes à température ambiante puis ajoute à nouveau 0,53 g de zinc en poudre. On renouvelle cette opération encore deux fois puis élimine par filtration le zinc résiduel que l'on rince avec 40 $cm^3$ d'eau et 100 $cm^3$ de chlorure de méthylène. On extrait au chlorure de méthylène. On lave, sèche et amène à sec sous pression réduite. On obtient ainsi 3,05 g du produit recherché.

Stade E : (1R cis, ΔZ) 2,2-diméthyl 3-(3-hydroxy 3-oxo 1-propényl) cyclopropane carboxylate de 1(R) (3-phénoxyphényl) éthyle.

On fait une hydrogénation de 3 g du produit préparé au stade précédent dans 80 $cm^3$ d'acétate d'éthyle en présence de 500 mg d'hydroxyde de palladium à 10 % sur sulfate de baryum et 0,9 $cm^3$ de quinoléine. On filtre, lave le filtrat à l'acide chlorhydrique puis à l'eau. On sèche et amène à sec sous pression réduite. On obtient ainsi 2,9 g du produit recherché.

Stade F : (1R cis, ΔZ) 2,2-diméthyl 3-(allyloxy 3-oxo 1-propényl) cyclopropane carboxylate de 1(R) (3-phénoxyphényl) éthyle.

On mélange 1,4 g de (1R cis, ΔZ) 2,2-diméthyl 3-(3-hydroxy 3-oxo 1-propényl) cyclopropane carboxylate de 1(R) (3-phénoxy phényl) éthyle (préparé au stade E) 7 $cm^3$ de chlorure de méthylène, 0,14 g de 4-diméthylaminopyridine. On introduit à 0 °C sous agitation 760 mg de dicyclohexylcarbodiimide agite encore 20 minutes à 0 °C puis à cette même température on ajoute 0,26 $cm^3$ d'alcool allylique dans 2 $cm^3$ de chlorure de méthylène. On agite pendant 1 heure à température ambiante le mélange réactionnel puis filtre. On amène à sec le filtrat sous pression réduite et obtient une huile que l'on chromatographie sur silice éluant : hexane-éther isopropylique (8-2). On obtient 570 mg du produit recherché.

$[\alpha]_D$ : + 109,5° ± 2° (c = 1 %, chloroforme)

Exemple 81 : (1R cis, ΔZ) 2,2-diméthyl 3-(3-terbutoxy 3-oxo 1-propényl) cyclopropane carboxylate de 1(R) (3-phénoxyphényle) éthyle.

On introduit sous agitation à température ambiante 1,4 g de o-terbutyl N,N'-diisopropyl urée dans une solution de 1,74 g de (1R cis, ΔZ) 2,2-diméthyl 3-(3-hydroxy 3-oxo 1-propényl) cyclopropane carboxylate de 1(R) (3-phénoxyphényl) éthyle (préparé selon le procédé décrit au stade E de l'exemple 81) dans 8 $cm^3$ d'acétate d'éthyle. On agite encore pendant 4 heures puis filtre. Le filtrat est amené à sec sous pression réduite. Le produit obtenu est chromatographié sur silice éluant : hexane-éther (8-2). On obtient ainsi 930 mg du produit recherché fondant à 80 °C.

$[\alpha]_D$ : + 125° ± 2° (c + 1 %, chloroforme)

Exemple 82 : (1R cis, ΔZ) 2,2-diméthyl 3-(3-terbutoxy 3-oxo 1-propényl) cyclopropane carboxylate de métaphénoxy benzyle.

Stade A : (1R cis) 2,2-diméthyl 3-(3,3,3-trichloroéthoxy 3-oxo propynyl) cyclopropane carboxylate de métaphénoxy benzyle.

On mélange 6,7 g d'acide (1R, cis) 2,2-diméthyl 3-(3,3,3-trichloroéthoxy 3-oxo propynyl) cyclopropane carboxylique (préparé selon le stade B de l'exemple 81) 67 $cm^3$ de chlorure de méthylène, 200 mg de 4-diméthylaminopyridine. On introduit ensuite à 0 °C et sous agitation 4,5 g d'alcool métaphénoxy benzylique dans 5 $cm^3$ de chlorure de méthylène puis une solution de 4,4 g de dicyclohexylcarbodiimide

dans 5 $cm^3$ de chlorure de méthylène. On agite pendant 3 heures à la température ambiante le mélange réactionnel et filtre. On amène à sec le filtrat sous pression réduite et obtient 11,5 g d'une huile que l'on chromatographie sur silice éluant : cyclohexane-acétate d'éthyle (85-15). On obtient 7,1 g du produit recherché.

Stade B : (1R cis) 2,2-diméthyl 3-(hydroxy 3-oxo propynyl) cyclopropane carboxylate de métaphénoxy benzyle.

On introduit sous agitation 5 g de zinc en poudre dans une solution de 5 g du produit préparé au stade A dont 18 $cm^3$ d'acide acétique et 2 $cm^3$ d'eau. On agite pendant 2 heures à température ambiante puis élimine par filtration le zinc résiduel que l'on rince à l'eau puis au chlorure de méthylène. On lave à l'eau la phase organique, la sèche, et amène à sec sous pression réduite. On reprend le résidu obtenu au toluène que l'on amène à sec sous pression réduite. Cette dernière opération est répétée 3 fois. On obtient ainsi 3,6 g de produit que l'on utilise tel quel pour le stade suivant.

Stade C : (1R cis) 2,2-diméthyl 3-(3-terbutoxy 3-oxo propynyl) cyclopropane carboxylate de métaphénoxy benzyle.

On introduit goutte à goutte sous agitation à température ambiante 4 $cm^3$ de o-terbutyl N,N'-diisopropylurée dans 2 g du produit obtenu au stade précédent. On ajoute ensuite 25 $cm^3$ d'acétate d'éthyle et agite pendant 1 heure. On détruit l'excès de réactif par addition de 2 $cm^3$ d'acide acétique et agite encore pendant 15 minutes. On filtre, rince à l'acétate d'éthyle et amène le filtrat à sec. On obtient 2,8 g d'une huile que l'on purifie par chromatographie sur silice éluant : cyclohexane, acétate d'éthyle (8-2). On obtient ainsi 1,4 g du produit recherché.

Stade D : (1R cis, ΔZ) 2,2-diméthyl 3-(3-terbutoxy 3-oxo 1-propényl) cyclopropane carboxylate de métaphénoxy benzyle.

On fait une hydrogénation de 1,4 g du produit préparé au stade précédent dans 30 $cm^3$ d'acétate d'éthyle en présence de 500 mg d'hydroxyde de palladium à 10 % sur sulfate de baryum et 0,7 $cm^3$ de quinoléine. On filtre, lave le filtrat à l'acide chlorhydrique 2N, et à l'eau, le sèche et amène à sec sous pression réduite. On obtient ainsi une huile que l'on purifie par 2 chromatographies sur silice éluant : cyclohexane-acétate d'éthyle (1ère fois 9-1) (2ème fois 95-5). On obtient ainsi 1 g du produit recherché.

$[\alpha]_D$ : + 38,5° ± 2,5° (c = 0,5 %, benzène)

Exemple 83 : (1R cis, ΔZ) 2,2-diméthyl 3-(3-oxo 3-éthoxy 1-propényl) cyclopropane carboxylate de (R,S) cyano 2-(6-phénoxy pyridyl) méthyle.

Stade A : Acide (1R cis) 2,2-diméthyl 3-(3-oxo 3-méthoxy propynyl)cyclopropane carboxylique.

On porte au reflux pendant 1 heure 30 minutes une solution renfermant 2,6 g de (1R cis) 2,2-diméthyl 3-(3-oxo 3-méthoxy propynyl)cyclopropane carboxylate de terbutyle préparé selon le stade A de la préparation 1, 25 $cm^3$ de toluène et 100 mg d'acide paratoluènesulfonique. On laisse revenir à température ambiante, maintient le mélange réactionnel sous agitation pendant 20 heures puis concentre à sec sous pression réduite. On recueille 1,95 g du produit recherché.

Stade B : (1R cis) 2,2-diméthyl 3-(3-oxo 3-méthoxy propynyl) cyclopropane carboxylate de (RS) 2-(6-phénoxypyridyl) méthyle.

On mélange sous agitation et sous atmosphère d'azote une solution de 19,9 g de (RS) cyano 2-(6-phénoxypyridyl) méthanol dans 75 $cm^3$ de chlorure de méthylène à une solution de 21,6 g d'acide (1R cis) 2,2-diméthyl 3-(3-oxo 3-méthoxypropynyl) cyclopropane carboxylique (préparé selon le stade précédent) dans 75 $cm^3$ de chlorure de méthylène. On introduit ensuite à 5 °C 1 g de 4-diméthyl aminopyridine puis après dissolution 21 g de dicyclohexylcarbodiimide. On agite pendant 4 heures à la température ambiante. On filtre, et concentre le filtrat sous pression réduite à 40 °C. On chromatographie le résidu obtenu sur silice éluant cyclohexane-acétate d'éthyle (7-3). On obtient ainsi 35,4 g de l'ester recherché.

Stade C : (1R cis) 2,2-diméthyl 3-(3-oxo 3-hydroxypropynyl) cyclopropane carboxylate de (RS) cyano 2-(6-phénoxypyridyl) méthyle.

On porte au reflux pendant 24 heures un mélange de 35,4 g du produit obtenu au stade précédent de 175 $cm^3$ de dioxane, de 35 $cm^3$ d'eau et de 5 g d'acide paratoluènesulfonique. On concentre la solution sous pression réduite et reprend le résidu par 250 $cm^3$ de chlorure de méthylène et 100 $cm^3$ d'eau. On lave la phase organique à l'eau, sèche et concentre sous pression réduite. On chromatographie sur silice le produit résultant cyclohexane-acétate d'éthyle (6-4) à 0,1 % d'acide acétique et obtient ainsi 17,7 g du produit recherché.

Stade D : (1R cis, ΔZ) 2,2-diméthyl 3-(3-oxo 3-hydroxy 1-propényl) cyclopropane carboxylate de (RS) cyano 2-(6-phénoxypyridyl) méthyle.

On fait une hydrogénation de 17,7 g de produit obtenu au stade précédent dans 200 $cm^3$ d'acétate d'éthyle en présence de 2,5 g d'hydroxyde de palladium à 10 % sur sulfate de Baryum et 2,5 $cm^3$ de quinoléine à une température de 20 °C. On filtre, sèche, évapore le solvant sous pression réduite et chromatographie sur silice éluant cyclohexane-acétate d'éthyle-acide acétique (6-4-0,1). On obtient ainsi 15 g du produit recherché.

Stade E : (1R cis, ΔZ) 2,2-diméthyl 3-(3-oxo 3-éthoxy 1-propényl) cyclopropane carboxylate de (RS) cyano 2-(6-phénoxypyridyl) méthyle.

On introduit sous agitation et sous atmosphère d'azote 70 mg de 4-diméthylaminopyridine puis 1,13 g de dicyclohexylcarbodiimide dans une solution refroidie à 0 °C, de 1,95 g de (1R cis, ΔZ) 2,2-diméthyl 3-(3-oxo 3-hydroxy 1-propényl) cyclopropane carboxylate de (RS) cyano 2-(6-phénoxypyridyl) méthyle (préparé comme au stade précédent) dans 10 $cm^3$ de chlorure de méthylène et 2 $cm^3$ d'éthanol. On agite encore pendant 1 heure 30 minutes à la température ambiante, filtre, rince, lave l'insoluble au chlorure de méthylène et concentre le filtrat sous pression réduite. On chromatographie 2 fois le résidu sur silice éluant cyclohexane-acétate d'éthyle (8-2) la 1ère fois, et (85-15) la 2ème fois, et obtient ainsi 1,4 g du produit recherché.

$[\alpha]_D$ : + 48° ± 2° (c = 0,5 %, chloroforme)

Exemple 84 : (1R cis, ΔZ) 2,2-diméthyl 3-(3-oxo 3-isopropyloxy-1-propényl) cyclopropane carboxylate de (RS) cyano 2-(6-phénoxy pyridyl) méthyle

En opérant comme à l'exemple 84 à partir de 1,95 g de (1R cis, ΔZ) 2,2-diméthyl 3-oxo 3-hydroxy 1-propényl) cyclopropane carboxylate de (RS) cyano 2-(6-phénoxypyridyl) méthyle préparé comme indiqué au stade D de l'exemple 84 et de 2 $cm^3$ d'isopropanol on obtient une huile que l'on chromatographie sur silice éluant cyclohexane-acétate d'éthyle (9-1). On obtient ainsi 1,7 g du produit recherché.
$[\alpha]_D$ : + 53° ± 2° (c = 0,75 %, chloroforme).

Exemple 85 : (1R cis, ΔZ) 2,2-diméthyl 3-(3-oxo 3-cyclobutoxy-1-propényl) cyclopropane carboxylate de (RS) cyano 2-(6-phénoxypyridyl) méthyle

En opérant comme à l'exemple 84 à partir de 2,1 g de (1R cis, ΔZ) 2,2-diméthyl 3-(3-oxo 3-hydroxy 1-propényl) cyclopropane carboxylate de (RS) cyano 2-(6-phénoxypyridyl) méthyle préparé comme indiqué au stade D de l'exemple 84 et de 1 $cm^3$ de cyclobutanol on obtient une huile que l'on chromatographie sur silice éluant hexane-actate d'éthyle (8-2). On obtient ainsi 1,6 g du produit recherché.
$[\alpha]_D$ : + 44,5° ± 2° (c = 0,5 %, benzène).

Exemple 86 : (1R cis, ΔZ) 2,2-diméthyl-3-(3-oxo 3-n-butoxy 1-propényl) cyclopropane carboxylate de (RS) cyano 2-(6-phénoxypyridyl) méthyle

En opérant comme à l'exemple 84 à partir de 2,1 g de (1R cis, ΔZ) 2,2-diméthyl 3-(3-oxo 3-hydroxy 1-propényl) cyclopropane carboxylate de (RS) cyano 2-(6-phénoxypyridyl) méthyle préparé comme indiqué au stade D de l'exemple 84 et de 1 $cm^3$ de n-butanol on obtient une huile que l'on chromatographie sur silice éluant n-hexane-acétate d'éthyle (9-1). On obtient ainsi 1,55 g du produit recherché.
$[\alpha]_D$ : + 52° ± 2,5° (c = 0,5 %, chloroforme)

Exemple 87 : (1R cis, ΔZ) 2,2-diméthyl 3-(3-oxo 3-allyloxy 1-propényl) cyclopropane carboxylate de (RS) cyano 2-(6-phénoxypyridyl) méthyle

En opérant comme à l'exemple 84 à partir de 2,1 g de (1R cis, ΔZ) 2,2-diméthyl 3-(3-oxo 3-hydroxy 1-propényl) cyclopropane carboxylate de (RS) cyano 2-(6-phénoxypyridyl) méthyle préparé comme indiqué au stade D de l'exemple 84 et de 0,8 $cm^3$ d'alcool allylique on obtient une huile que l'on chromatographie sur silice éluant n-hexane-acétate d'éthyle (85-15). On obtient ainsi 1,55 g du produit recherché.
$[\alpha]_D$ : + 53° ± 3° (c = 0,3 % chloroforme).

Exemple 88 : (1R cis, ΔZ) 2,2-diméthyl 3-[3-oxo 3-(1,1-diméthylpropoxy) propényl] cyclopropane carboxylate de 3-phénoxyphényl méthyle

Stade A : (1R cis) 2,2-diméthyl 3-[3-oxo 3-(1,1-diméthylpropoxy) propynyl] cyclopropane carboxylate de 3-phénoxyphényl méthyle.

On mélange 3 g de produit obtenu selon le stade B de l'exemple 83, 3 $cm^3$ de chlorure de méthylène, 1 $cm^3$ d'alcool ter-amylique et 0,1 g de diméthylaminopyridine. On ajoute sous agitation et à + 5 °C, 1,75 g de dicyclohexylcarbodiimide dans 1 $cm^3$ de chlorure de méthylène, laisse revenir à température ambiante, et agite pendant 4 heures 30 minutes. On filtre, amène à sec le filtrat sous pression réduite, et chromatographie le résidu sur silice (éluant hexane-acétate d'éthyle 8-2). On obtient 1,05 g de produit attendu.

Stade B : (1R cis, ΔZ) 2,2-diméthyl 3-[3-oxo 3-(1,1-diméthyl propoxy) propényl] cyclopropane carboxylate de 3-phénoxyphényl méthyle.

On fait une hydrogénation de 1,05 g du produit obtenu au stade précédent dans 20 $cm^3$ d'acétate d'éthyle en présence de 0,2 g d'hydroxyde de palladium à 10 % sur sulfate de baryum et de 0,2 $cm^3$ de quinoléine. On filtre, lave le filtrat à l'acide chlorhydrique 0,1 N puis à l'eau, le sèche, le concentre à sec sous pression réduite, chromatographie le résidu sur silice (éluant hexane-acétate d'éthyle 95-5) et obtient 0,756 g du produit attendu.

$[\alpha]_D$ : + 56,5° ± 2,5° (c = 1 %, chloroforme)

Préparation 1 : Acide (1R cis, ΔZ) 2,2-diméthyl 3-[3-(3-méthoxy-3-oxo 1-propynyl) cyclopropane carboxylique.

Stade 1 : (1R cis) 2,2-diméthyl 3-(3-méthoxy-3-oxo 1-propynyl) cyclopropane carboxylate de terbutyle.

On introduit 55 g de (1R, cis) 2,2-diméthyl 3-(2,2-dibromovinyl) cyclopropane carboxylate de terbutyle dans 550 $cm^3$ de tétrahydrofurane. On refroidit à − 70 °C et ajoute en 40 minutes, 132 $cm^3$ d'une solution de butyl lithium dans le cyclohexane (à 20 %) et agite 30 minutes à − 65 °C. On ajoute ensuite 12,5 $cm^3$ de chloroformiate de méthyle. Après 2 heures de réaction à − 70 °C, on laisse la température remonter à − 20 °C et verse le mélange obtenu dans une solution aqueuse de phosphate monosodique et extrait à l'éther. On lave, sèche et amène à sec sous pression réduite. On obtient ainsi 38,3 g d'un produit que l'on chromatographie sur silice éluant cyclohexane-acétate d'éthyle (8-2). On obtient ainsi 17,2 g du produit recherché.

Stade B : (1R cis, ΔZ) 2,2-diméthyl 3-(3-méthoxy 3-oxo 1-propényl) cyclopropane carboxylate de terbutyle.

On fait une hydrogénation de 12 g du produit préparé au stade A dans 240 $cm^3$ d'acétate d'éthyle, en présence de 2,4 g d'hydroxyde de palladium à 10 % sur sulfate de baryum et 2,4 $cm^3$ de quinoléine. On filtre et sèche. On obtient ainsi 11 g du produit recherché.

Stade C : Acide (1R cis, ΔZ) 2,2-diméthyl 3-(3-méthoxy-3-oxo 1-propényl) cyclopropane carboxylique.

On porte au reflux pendant 3 heures une solution renfermant 13,5 g du produit préparé au stade B, 100 $cm^3$ de toluène et 400 mg d'acide paratoluènesulfonique hydraté. On amène à sec sous pression réduite et obtient 11,2 g d'un produit que l'on chromatographie sur silice, éluant ; cyclohexane-acétate d'éthyle-acide acétique (60-39-1). On amène à sec sous pression réduite et obtient 9,6 g du produit recherché fondant à 110 °C.

$[\alpha]_D^{20}$ = 75,5° ± 2° (c = 1 %, $CHCl_3$)

RMN $CDCl_3$ en ppm

1,3 : protons des méthyles en 2 du cyclopropane,
1,86-2 : proton en 1 du cyclopropane,
3,1-3,28-3,43 : proton en 3 du cyclopropane,
5,8-5,99 : proton éthylénique en α du groupement $CO_2CH_3$,
6,42-6,58 } : proton éthylénique en β du groupement $CO_2CH_3$
6,61-6,77 } :
8,63 : proton du groupement $CO_2H$.
3,71 : protons du méthoxy.

Préparation 2 : Acide (1R cis, ΔZ) 2,2-diméthyl 3-(3-éthoxy 3-oxo 1-propényl) cyclopropane carboxylique.

Stade 1 : (1R cis) 2,2-diméthyl 3-(3-hydroxy 3-oxo 1-propynyl) cyclopropane carboxylate de terbutyle.

On introduit 26 g de (1R cis) 2,2-diméthyl 3-(2,2-dibromovinyl) cyclopropane carboxylate de terbutyle dans 175 $cm^3$ de tétrahydrofurane anhydre. On ajoute ensuite à − 65 °C 60 $cm^3$ d'une solution de butyl lithium à 20 % dans le cyclohexane. On agite 1 heure à − 60 °C puis fait barboter un courant de gaz carbonique pendant une heure et demie, verse le mélange réactionnel dans l'eau glacée additionnée de

soude N. On lave à l'éther. La phase aqueuse alcaline est acidifiée à pH 4 et extraite à l'éther. On sèche les phases organiques, amène à sec sous pression réduite. On obtient ainsi un produit que l'on recristallise dans l'éther de pétrole (Eb 60-80 °C). On obtient alors 8,3 g du produit recherché fondant à 144 °C.

RMN $CDCl_3$ en ppm

1,22 et 1,37 : protons des méthyles en 2 du cyclopropane,
1,78 : proton en 1 et 3 du cyclopropane,
1,47 : protons du terbutyle,
8,25 : proton du groupement $-\underset{\underset{O}{\|}}{C}-OH$.

Stade B : (1R cis) 2,2-diméthyl 3-(3-éthoxy 3-oxo 1-propynyl) cyclopropane carboxylate de terbutyle.

On introduit 4 g du produit préparé au stade A, 3,4 g de dicyclohexylcarbodiimide et 6 mg de 4-diméthylamino pyridine dans 30 $cm^3$ de chlorure de méthylène. On ajoute ensuite 1,5 $cm^3$ d'éthanol et agite 16 heures à 20 °C. On filtre et concentre le filtrat sous pression réduite. On obtient ainsi 5,5 g d'un produit que l'on purifie par chromatographie sur silice éluant cyclohexane-acétate d'éthyle (9-1). On obtient ainsi 4,25 g du produit recherché.

RMN $CDCl_3$ en ppm

1,18-1,21 et 1,36-1,47 : protons en 2 du cyclopropane,
1,73 et 1,82 : protons en 1 et 3 du cyclopropane,
1,47 ; protons du terbutyle,
1,27-1,38-1,5 }
4,0-4,13-4,25-4,36 } : protons de l'éthyle.

Stade C : (1R cis, ΔZ) 2,2-diméthyl 3-(3-éthoxy 3-oxo 1-propényl) cyclopropane carboxylate de terbutyle.

On fait une hydrogénation de 4,3 g du produit préparé au stade précédent dans 100 $cm^3$ d'acétate d'éthyle en présence de 800 mg d'hydroxyde de palladium sur sulfate de baryium et 0,8 $cm^3$ de quinoléine. On filtre, amène le filtrat à un pH inférieur à 7, avec de l'acide chlorhydrique 2N et lave à l'eau. On sèche et amène à sec sous pression réduite. On obtient 4,6 g d'un produit que l'on chromatographie sur silice, éluant : cyclohexane-acétate d'éthyle (95-5). On obtient ainsi 2,5 g du produit recherché.

RMN $CDCl_3$ en ppm

1,25 et 1,28 : protons des méthyles en 2 du cyclopropane,
1,78-1,93 : proton en 1 du cyclopropane,
2,98-3,1-3,2 : proton en 3 du cyclopropane,
6,4-6,6-6,8 : proton du carbone éthylénique en α du cyclopropane,
5,7-5,9 : proton du carbone éthylénique portant le groupement éthoxy carbonyle,
4,0-4,13-4,25-4,36 : proton du méthylène de l'éthoxy.

Stade D : Acide (1R cis, ΔZ) 2,2-diméthyl 3-(3-éthoxy 3-oxo 1-propényl) cyclopropane carboxylique.

On introduit 2,3 g du produit préparé au stade C et 20 mg d'acide paratoluènesulfonique hydraté dans 20 $cm^3$ de toluène. On porte au reflux pendant 40 minutes, amène à sec sous pression réduite et obtient ainsi 2,1 g d'un résidu que l'on chromatographie sur silice, éluant : cyclohexane-acétate d'éthyle-acide acétique (60-39-1). On isole 1,7 g du produit que l'on recristallise dans le cyclohexane. On obtient ainsi 1,5 g du produit recherché fondant à 96 °C.

RMN $CDCl_2$ en ppm

1,3 et 1,32 : protons des méthyles en 2 du cyclopropane,
1,86-2,02 : proton du carbone en 1 du cyclopropane,
3,15-3,28 }
3,3-3,45 } : proton du carbone en 3 du cyclopropane
6,38-6,53 }
6,55-6,73 } : proton du carbone éthylénique branché sur le cyclopropane,
5,78-5,96 : proton du carbone éthylénique portant le groupement éthoxy carbonyle,

1,18-1,3-1,41 : protons du méthyle du groupement éthoxy carbonyle,
4,0-4,13 / 4,25-4,36 : protons du méthylène du groupement éthoxycarbonyle.

Préparation 3 : Acide (1R cis, ΔZ) 2,2-diméthyl 3-(3-propoxy 3-oxo 1-propenyl) cyclopropane carboxylique.

Stade A : (1R cis) 2,2-diméthyl 3-propoxy 3-oxo 1-propynyl cyclopropane carboxylate de terbutyle.

On introduit 22,8 g de (1R cis) 2,2-diméthyl 3-(2,2-dibromovinyl) cyclopropane carboxylate de terbutyle, 250 $cm^3$ de tétrahydrofurane puis, à − 60 °C, 55 $cm^3$ d'une solution de butyl lithium dans le cyclohexane à 20 %. On maintient à − 65 °C pendant une heure et introduit à − 65 °C en 15 minutes 8 $cm^3$ de chloroformiate de n-propyle. On maintient l'agitation pendant 1 heure à − 65 °C, laisse remonter à la température ambiante en 1 heure, et agite à nouveau pendant 1 heure à la température ambiante. On verse sur une solution aqueuse saturée de phosphate monosodique, agite, extrait à l'éther et lave à l'eau. On sèche et amène à sec sous pression réduite. On obtient ainsi 19,5 g d'une huile que l'on purifie par chromatographie sur silice éluant cyclohexane-acétate d'éthyle (9-1). On obtient ainsi 11,5 g du produit attendu.

Spectre RMN $CDCl_3$ en ppm

1,17 et 1,37 : protons des méthyles en 2 du cyclopropane,
1,72 : protons en 1 et 3 du cyclopropane,
1,44 : protons du terbutyle,
4,0-4,12-4,23 : proton du méthylène en 1 du propoxy carbonyle,
0,83-0,95-1,06 : protons du méthyle du propoxy carbonyle.

Stade B : (1R cis, ΔZ) 2,2-diméthyl 3-(3-propoxy 3-oxo-1-propényl) cyclopropane carboxylate de terbutyle.

On hydrogène 7 g de (1R, cis) 2,2-diméthyl 3-(n-propoxy 3-oxo-1-propynyl) cyclopropane carboxylate de terbutyle dans 140 $cm^3$ d'acétate d'éthyle en présence de 1,4 g d'hydroxyde de palladium à 10 % sur sulfate de baryum et de 1,4 $cm^3$ de quinoléine. On lave le filtrat à l'aide d'une solution d'acide chlorhydrique 2 N, puis lave à l'eau, sèche et amène à sec sous pression réduite. On obtient ainsi 7,2 g d'un produit que l'on chromatographie sur silice, éluant : cyclohexane-acétate d'éthyle (95-5). On obtient ainsi 6,1 g du produit recherché.

Spectre RMN $CDCl_3$ en ppm

1,25 et 1,29 : proton des méthyles en 2 du cyclopropane,
1,5 à 2,03 : proton du carbone en 1 du cyclopropane,
3,03 à 3,35 : proton du carbone en 3 du cyclopropane,
6,5-6,66 / 6,69-6,85 : proton du carbone éthylénique relié au cyclopropane,
5,82-6,0 : proton du carbone éthylénique portant le groupement propoxy carbonyle,
4,02-4,12-4,23 : proton du méthylène en 1 du groupement propoxy carbonyle,
0,86-0,98-1,1 : proton du méthyle du groupement propoxy carbonyle.

Stade C : Acide (1R cis, ΔZ) 2,2-diméthyl 3-(3-propoxy 3-oxo 1-propényl) cyclopropane carboxylique

On porte à reflux pendant une heure un mélange de 5,8 g du produit préparé au stade B, 200 mg d'acide paratoluène sulfonique hydraté, et 60 $cm^3$ de toluène. On amène à sec sous pression réduite et obtient 5 g d'un produit que l'on chromatographie sur silice éluant cyclohexane-acétate d'éthyle-acide acétique (70-29-1). On obtient 4,2 g du produit recherché.

Spectre RMN $CDCl_3$ en ppm

1,27 et 1,29 : H des méthyles en 2 du cyclopropane,
1,86-2 : H en 1 du cyclopropane,
3,13 à 3,45 : H en 3 du cyclopropane,
5,8-6 : H du carbone éthylénique portant le groupement $CO_2CH_2CH_2CH_3$,
6,4-6,56-6,59 : H du carbone éthylénique relié au cyclopropane,
3,98-4,08-4,18 : H du méthylène en 1 du groupement propoxy carbonyle,
0,83-0,95-1,06 : H des méthyles du groupement propoxy carbonyle.

Préparation 4 : Acide (1R cis, ΔZ) 2,2-diméthyl 3-[3-(1-méthyl-éthoxy) 3-oxo-1-propényl] cyclopropane carboxylique.

Stade A : (1R cis, ΔZ) 2,2-diméthyl 3-(3-hydroxy 3-oxo 1-propényl) cyclopropane carboxylate de terbutyle

On hydrogène 2 g de (1R cis) 2,2-diméthyl 3(3-hydroxy 3-oxo 1-propynyl) cyclopropane carboxylate de terbutyle dans 40 $cm^3$ d'acétate d'éthyle en présence de 0,38 g d'hydroxyde de palladium à 10 % sur sulfate de baryium et 0,4 $cm^3$ de quinoléine. On filtre, lave le filtrat à l'acide chlorhydrique 0,5 N puis à l'eau jusqu'à neutralisé, sèche, concentre à sec sous pression réduite et obtient 2 g du produit recherché fondant à 94 °C.

Stade B : (1R cis, ΔZ) 2,2-diméthyl 3-[3-(1-méthyléthoxy)-3-oxo 1-propényl] cyclopropane carboxylate de terbutyle

On mélange 2,7 g de (1R cis) 2,2-diméthyl 3-[(Z) 3-hydroxy 3-oxo 1-propényl] cyclopropane carboxylate de terbutyle, dans 10 $cm^3$ d'acétate d'éthyle, ajoute ensuite 2 g d'O-isopropyl N,N'-diisopropyl isourée et agite pendant une heure à température ambiante. On porte au reflux pendant une heure trente, laisse revenir à 20 °C, filtre l'insoluble et amène à sec le filtrat sous pression réduite. On obtient 3,5 g d'une huile que l'on chromatographie sur silice éluant benzènecyclohexane (7-3). On obtient ainsi 1 g du produit recherché que l'on utilise tel quel dans le stade suivant.

Stade C : Acide (1R cis, ΔZ) 2,2-diméthyl 3-[3-(1-méthyléthoxy) 3-oxo 1-propényl] cyclopropane carboxylique

On porte à 120 °C sous agitation pendant 2 heures 30, un mélange renfermant 1,4 g de (1R cis, ΔZ) 2,2-diméthyl 3-[3-(1-méthyléthoxy) 3-oxo 1-propényl] cyclopropane carboxylate de terbutyle, 100 mg d'acide paratoluène sulfonique et 14 $cm^3$ de toluène. On amène à sec sous pression réduite. On obtient résidu que l'on recristallise dans l'éther isopropylique. On glace, essore, sèche et obtient ainsi 900 mg du produit recherché fondant à 98 °C.

Préparation 5 : Acide (1R cis, ΔZ) 2,2-diméthyl 3-(3-cyclobutyloxy 3-oxo 1-propényl) cyclopropane carboxylique

Stade A : (1R cis, ΔZ) 2,2-diméthyl 3-(3-cyclobutyloxy 3-oxo 1-propényl) cyclopropane carboxylate de terbutyle

On dissout 4 g de (1R cis, ΔZ) 2,2-diméthyl 3-[3-hydroxy 3-oxo 1-propenyl] cyclopropane carboxylate de terbutyle dans 20 mg de chlorure de méthylène puis on ajoute 1,7 mg de cyclobutanol. On baisse la température à 0/+ 5 °C, ajoute 3,45 g de dicyclohexylcarbodiimide, 28 mg de diméthylaminopyridine dans 20 mg de chlorure de méthylène et maintient l'agitation pendant 2 heures à 5 °C environ et 2 heures à température ambiante. On élimine la dicyclohexylurée formée, concentre à sec le filtrat et chromatographie sur silice en éluant par un mélange n-hexane-éther isopropylique (9-1). On obtient 2,3 g de produit attendu.

Stade B : Acide (1R cis, ΔZ) 2,2-diméthyl 3-(3-cyclobutyloxy 3-oxo 1-propenyl) cyclopropane carboxylique

On chauffe au reflux pendant 15 minutes 2,3 g du produit obtenu précédemment, 25 $cm^3$ de toluène et 250 mg d'acide paratoluène sulfonique, refroidit et agite pendant 2 heures à 0/45 °C. On filtre l'insoluble et concentre à sec le filtrat pour 1,8 g de produit attendu.

Spectre IR

—OH : acide 3 500 $cm^{-1}$
—C = O : acide 1 733 $cm^{-1}$
+ ester conj 1 702 $cm^{-1}$
gem di Me : 1 390 $cm^{-1}$
1 380 $cm^{-1}$

Préparation 6 : Acide (1R cis, ΔZ) 2,2-diméthyl 3-[3-(RS) 1-méthyl-propyloxy) 3-oxo 1-propenyl : cyclopropane carboxylique

Stade A : (1R cis) 2,2-diméthyl 3-[3(RS) 1-méthyl propyloxy 3-oxo 1-propynyl] cyclopropane carboxylate de terbutyle

On mélange 4 g de (1R, cis) 2,2-diméthyl 3-(3-hydroxy 3-oxo 1-propynyl) cyclopropane carboxylate de terbutyle, 40 $cm^3$ de chlorure de méthylène et 6 mg de 4-diméthylamino pyridine puis on ajoute 3,4 g de dicyclohexylcarbodiimide. Après 30 minutes d'agitation sous atmosphère inerte, on ajoute, en 5 minutes, 2 $cm^3$ de 1-méthyl propanol et 2 $cm^3$ de chlorure de méthylène et maintient l'agitation pendant 3 heures à température ambiante. On filtre la dicyclohexylurée formée, concentre à sec le filtrat sous pression

réduite et chromatographie le résidu sur silice, en éluant par un mélange n-hexane-éther isopropylique (8-2). On obtient 3,5 g du produit attendu.

Stade B : (1R cis, ΔZ) 2,2-diméthyl 3[3-(RS) 1-méthyl propyloxy 3-oxo 1-propenyl] cyclopropane carboxylate de terbutyle

On hydrogène 3 g du produit obtenu précédemment comme indiqué dans la préparation 4. On chromatographie sur silice en éluant par un mélange n-hexane éther isopropylique (9-1) et obtient 2,5 g de produit attendu.

Stade C : Acide (1R cis, ΔZ) 2,2-diméthyl 3-[3-(RS)-1-méthyl propyloxy 3-oxo 1-propényl] cyclopropane carboxylique

On agite 3 g de produit obtenu ci-dessus avec 250 mg d'acide paratoluène sulfonique dans 25 $cm^3$ de toluène. On chauffe au reflux jusqu'à cessation du dégagement gazeux. On concentre à sec sous pression réduite, chromatographie le résidu sur silice en éluant par un mélange : cyclohexane, acétate d'éthyle et d'acide acétique (70-30-1). On obtient 1,85 g de produit attendu.

Spectre IR ($CHCl_3$)

—OH : acide 3 510 $cm^{-1}$
—C = O : acide 1 735 $cm^{-1}$
ester 1 170 $cm^{-1}$
C = C conj 1 637 $cm^{-1}$
gem di Me 1 381 $cm^{-1}$

Préparation 7 : Acide (1R cis, ΔZ) 2,2-diméthyl 3[3-oxo 3-(cyclopropylméthoxy)] propenyl cyclopropane-1-carboxylique

Stade A : (1R cis, ΔZ) 2,2-diméthyl 3[3-oxo 3-cyclopropylméthoxy] propenyl cyclopropane-1-carboxylate de terbutyle

Dans 150 $cm^3$ de chlorure de méthylène on introduit 20 g de (1R cis, ΔZ) 2,2-diméthyl 3(3-hydroxy 3-oxo 1-propenyl) cyclopropane 1-carboxylate de terbutyle et 6,2 g de cyclopropylcarbinol, ajoute 2 g de diméthyl aminopyridine et 17,5 g de dicyclohexylcarbodiimide en solution dans 60 $cm^3$ de chlorure de méthylène, on agite pendant 2 heures à 20 °C, élimine de la préparation l'insoluble formé, concentre à sec par distillation sous pression réduite, chromatographie le résidu sur silice en éluant par un mélange d'hexane et d'éther isopropylique (9/1) et obtient 12,32 g de (1R cis, ΔZ) 2,2-diméthyl) 3[3-oxo 3-cyclopropylméthoxy] propenyl cyclopropane-1-carboxylate de terbutyle.

Stade B : Acide (1R cis, ΔZ) 2,2-diméthyl 3[3-oxo 3-cyclopropylméthoxy] propenyl cyclopropane-1-carboxylique

Dans 120 $cm^3$ de toluène on introduit 12,32 g de (1R cis, ΔZ) 2,2-diméthyl 3-[3-oxo 3-cyclopropylméthoxy] propenyl cyclopropane-1-carboxylate de terbutyle, 0,6 g d'acide paratoluènesulfonique, porte au reflux, maintient le reflux pendant 45 minutes, refroidit, concentre à sec par distillation sous pression réduite, chromatographie le résidu sur silice en éluant avec un mélange d'hexane et d'acétate d'éthyle (7/3) à 1 % d'acide acétique et obtient 8,94 g d'acide (1R cis, ΔZ) 2,2-diméthyl 3[3-oxo 3-cyclopropylméthoxy] propenyl cyclopropane-1-carboxylique F = 106 °C.

Spectre RMN ($CDCl_3$) ppm

1,29-1,31 protons des méthyles géminés
1,13 proton du —CH<
1,85-2 2 proton en 1 du cyclopropane
3,9-4 proton du O—$CH_2$—<
5,82-6,01 et de 6,42 à 6,77 protons éthyléniques cis.

Préparation 8 : Acide (1R cis, ΔZ) 2,2-diméthyl 3 (3-oxo 3-tert-butoxy propenyl) cyclopropane carboxylique

Stade A : (1R cis) 2,2-diméthyl 3[3-oxo 3-hydroxy propynyl] cyclopropane-1-carboxylate de méthyle

Dans 360 $cm^3$ de tétrahydrofuranne on introduit 36,5 g de (1R, cis) 2,2-diméthyl 3-(2,2-dibromoéthényl) cyclopropane-1-carboxylate de méthyle ajoute à – 70 °C 100 $cm^3$ de suspension de buthylithium à

20 % dans le cyclohexane, agite pendant 10 minutes, fait barboter du gaz carbonique pendant 30 minutes laisse remonter la température à − 20 °C, verse le mélange réactionnel sur un mélange d'eau, de glace, et de bicarbonate de sodium, extrait à l'éther éthylique, lave à l'eau, acidifie les phases aqueuses, les extrait à l'éther, lave les phases organiques réunies à l'eau, les concentre à sec par distillation sous pression réduite, chromatographie le résidu sur silice en éluant avec un mélange de cyclohexane et d'acétate d'éthyle, 6/4 à 1 % d'acide acéthique obtient 6,8 g de (1R, cis) 2,2-diméthyl 3[3-oxo 3-hydroxy propynyl] cyclopropane-1-carboxylate de méthyle.

Stade B : (1R cis) 2,2-diméthyle 3(3-oxo-3-tert-butoxy propynyl) cyclopropane-1-carboxylate de méthyle

Dans 10 $cm^3$ d'acétate d'éthyle on introduit 6,8 g de (1R cis) 2,2-diméthyl 3(3-oxo 3-hydroxy propynyl) cyclopropane-1-carboxylate de méthyle ajoute à + 15 °C 13,5 g de 0-tert-butyle N,N'-diisopropyle urée, agite pendant 2 heures, élimine par filtration, l'insoluble formé, concentre à sec le filtrat, chromatographie le résidu sur silice en éluant par un mélange de cyclohexane et d'acétate d'éthyle (85-15) et obtient 7 g de (1R, cis) 2,2-diméthyl 3(3-oxo 3-tert-butoxy propynyl) cyclopropane-1-carboxylate de méthyle.

Stade C : (1R cis, ΔZ) 2,2-diméthyl 3(3-oxo 3-tert-butoxy-1-propényl) cyclopropane-1-carboxylate de méthyle

On hydrogène, à 20 °C en présence d'hydroxyde de palladium à 10 % sur sulfate de barium et de 1,4 $cm^3$ de quinoléine 7 g de (1R, cis) 2,2-diméthyl 3(3-oxo 3-tert-butoxy propynyl) cyclopropane-1-carboxylate de méthyle dans 149 $cm^3$ d'acétate d'éthyle. On filtre, lave le filtrat à l'acide chlorhydrique N, puis à l'eau, le sèche, le concentre à sec par distillation sous prestion réduite et obtient 5,8 g de (1R cis, ΔZ) 2,2-diméthyl 3(3-oxo 3-tert-butoxy 1-propenyl) cyclopropane-1-carboxylate de méthyle.

Stade D : Acide (1R cis, ΔZ) 2,2-diméthyl 3(3-oxo 3-tert-butoxy-1-propenyl) cyclopropane-1-carboxylique

Dans un mélange de 25 $cm^3$ de méthanol et de 9,8 $cm^3$ de solution aqueuse N de soude, on introduit 2,5 g de (1R cis, ΔZ) 2,2-diméthyl 3(3-oxo 3-tert-butoxy1-1propényl) cyclopropane-1-carboxylate de méthyle, agite pendant 3 heures à 50 °C, refroidit à − 75 °C, verse le mélange réactionnel dans l'eau, extrait à l'éther éthylique, acidifie la phase aqueuse à pH 1 par de l'acide chlorhydrique, extrait à l'éther, concentre les extraits organiques à sec par distillation sous pression réduite, chromatographie le résidu sur silice en éluant par un mélange de cyclohexane et d'acétate d'éthyle 7/3 à 1 % d'acide acétique et obtient 1,576 g d'acide (1R, cis ΔZ) 2,2-diméthyl 3(3-oxo 3-tert-butoxy propenyl) cyclopropane-1-carboxylique.

Spectre IR ($CHCl_3$)

Absorption à 3 500 $cm^{-1}$ attribué à l'OH acide (monomère + dimère)
Absorptions à 1 730 $cm^{-1}$ et 1 695 $cm^{-1}$ attribué au C = O acide et ester
Absorption à 1 628 $cm^{-1}$ attribué au C = C conjugué
Absorptions à 1 390 $cm^{-1}$ et 1 377 $cm^{-1}$ attribués aux méthyles géminés
Absorption à 1 368 $cm^{-1}$ attribué au t. Bu.

Préparation 9 : Acide (1R cis, ΔZ) 2,2-diméthyl 3-(3-isobutyloxy 3-oxo 1-propenyl) cyclopropane-1-carboxylique

Stade A : (1R cis, ΔZ) 2,2-diméthyl 3-(3-isobutoxy 3-oxo-1-propenyl) cyclopropane-1-carboxylate de tert-butyle

Dans 10 $cm^3$ de chlorure de méthylène et 1 g d'isobutanol on introduit 4 g de (1R cis, ΔZ) 2,2-diméthyl 3-(3-hydroxy 3-oxo 1-propenyl) cyclopropane-1-carboxylate de tert-butyle préparé selon la préparation 4 Stade A, puis ajoute à 0 °C, 2,8 g de dicyclohexylcarbodiimide et 30 mg de diméthylaminopyridine en solution dans 10 $cm^3$ de chlorure de méthylène ; on agite pendant 17 heures à 20 °C, élimine l'insoluble par filtration, concentre à sec pour distillation sous pression réduite, chromatographie le résidu sur silice en éluant par un mélange de cyclohexane et d'éther isopropylique (9/1) et obtient 2,1 g de (1R cis, ΔZ) 2,2-diméthyl 3-(3-isobutoxy 3-oxo 1-propenyl) cyclopropane-1-carboxylate de tert-butyle.

Stade B : Acide (1R cis, ΔZ) 2,2-diméthyl 3(3-isobutoxy 3-oxo-1-propenyl) cyclopropane-1-carboxylique

Dans 20 $cm^3$ de toluène on introduit 2,1 g de (1R cis, ΔZ) 2,2-diméthyl 3-(3-isobutoxy 3-oxo 1-propenyl) cyclopropane-1-carboxylate de tert-butyle, 0,1 g d'acide paratoluène sulfonique, porte au reflux l'y maintient pendant 20 minutes, concentre à sec par distillation sous pression réduite, chromatographie sur silice en éluant par un mélange d'hexane et d'acétate d'éthyle (7/3) à 1 % d'acide acétique et obtient 1,53 g d'acide (1R cis, ΔZ) 2,2-diméthyl 3-(3-isobutoxy 3-oxo-1-propenyl) cyclopropane-1-carboxylique.

Spectre IR (chloroforme)

Absorption à 3 300 $cm^{-1}$ attribué à l'OH acide
Absorption à 1 730 $cm^{-1}$, 1 706 $cm^{-1}$, 1 694 $cm^{-1}$ attribué au C = O acide et ester conjugué.
Absorption à 1 630 $cm^{-1}$ attribué à C = C (ΔZ)
Absorption à 1 390 $cm^{-1}$, 1 380 $cm^{-1}$ attribué aux méthyles géminés.

Préparation 10 : Acide (1R cis, ΔZ) 2,2-diméthyl 3-(3-n butoxy 3-oxo 1-propenyl) cyclopropane carboxylique

Stade A : (1R, cis) 2,2-diméthyl 3-(3-n butoxy 3-oxo 1-propynyl) cyclopropane carboxylate de tert-butyle

On mélange 4 g de (1R, cis) 2,2-diméthyl 3-(3-hydroxy 3-oxo 1-propynyl) cyclopropane carboxylate de tert-butyle, 40 $cm^3$ de chlorure de méthylène et 6 mg de 4-diéthylaminopyridine puis on ajoute 3,4 g de dicyclohexylcarbodiimide. Après 30 minutes d'agitation sous atmosphère inerte, on ajoute, en 5 minutes, 4 $cm^3$ d'un mélange (1-1) de n-butanol et de chlorure de méthylène et maintient l'agitation pendant 3 heures à température ambiante. On filtre la dicyclohexylurée formée, concentre à sec le filtrat sous pression réduite et chromatographie le résidu sur silice, en éluant par un mélange cyclohexane acétate d'éthyle (9-1). On obtient 4,7 g du produit attendu.

Stade B : (1R cis, ΔZ) 2,2-diméthyl 3-(3-n butoxy 3-oxo-1-propenyl) cyclopropane carboxylate de tert-butyle

On agite sous hydrogène pendant 15 minutes 800 mg d'hydroxyde de palladium sur sulfate de baryium dans 20 $cm^3$ d'acétate d'éthyle puis on ajoute 4,7 g de produit obtenu ci-dessus dans 50 $cm^3$ d'acétate d'éthyle et 0,8 $cm^3$ de quinoléine et laisse sous hydrogène pendant 30 minutes. On filtre, lave le filtrat à l'acide chlorhydrique N puis à l'eau, sèche concentre à sec sous pression réduite et chromatographie le résidu sur silice en éluant avec un mélange cyclohexane, acétate d'éthyle (95-5). On obtient 3,4 g de produit attendu.

Stade C : Acide (1R cis, ΔZ) 2,2-diméthyl 3(3-n butoxy 3-oxo-1-propenyl) cyclopropane carboxylique

On agite 3,3 g de produit obtenu ci-dessus avec 350 mg d'acide paratoluène sulfonique dans 40 $cm^3$ de toluène. On chauffe au reflux jusqu'à cessation du dégagement gazeux d'isobutylène soit environ 40 minutes. On concentre à sec sous pression réduite chromatographie le résidu sur silice en éluant par un mélange cyclohexane, acétate d'éthyle et acide acétique (75-25-1). On obtient 2 g de produit attendu.

RMN $CDCl_3$ ppm

1,26 et 1,3 : protons des méthyles en 2 du cyclopropane
1,85-1,99 : proton en 1 du cyclopropane
3,13 à 3,47 : proton en 3 du cyclopropane
6,4-6,57 et 6,59-6,75 : proton en 1 de la chaîne allylique
5,8-5,99 : proton en 2 de la chaîne allylique

Préparation 11 : Acide (1R cis, ΔZ) 2,2-diméthyl 3-(3-cyclopentoxy 3-oxo 1-propenyl) cyclopropane-1-carboxylique

Stade A : (1R cis, ΔZ) 2,2-diméthyl 3-(3-cyclopentoxy 3-oxo-1-propenyl) cyclopropane-1-carboxylate de tert-butyle

Dans 15 $cm^3$ de chlorure de méthylène on introduit 4 g de (1R cis, ΔZ) 2,2-diméthyl 3-(3-hydroxy 3-oxo-1-propenyl) cyclopropane-1-carboxylate de tert-butyle, 1,43 g de cyclopentanol, ajoute à 0 °C, 3,43 g de cyclohexylcarbodiimide, 40 mg de diméthyl amino pyridine en solution dans 10 $cm^3$ de chlorure de méthylène, agite la suspension à 20 °C pendant 17 heures, élimine l'insoluble par filtration, concentre le filtrat à sec par distillation sous pression réduite, chromatographie le résidu sur silice en éluant par un mélange de cyclohexane et d'éther isopropylique (9/1) on obtient 1,38 g de (1R cis, ΔZ) 2,2-diméthyl 3-(3-cyclopentoxy 3-oxo 1-propenyl) cyclopropane-1-carboxylate de tert-butyle. F = 57 °C

Stade B : Acide (1R cis, ΔZ) 2,2-diméthyl 3-(3-cyclopentoxy-3-oxo 1-propenyl) cyclopropane-1-carboxylique

Dans 25 $cm^3$ de toluène on introduit 2,54 g de (1R cis, ΔZ) 2,2-diméthyl 3-(3-cyclopentoxy 3-oxo 1-propenyl) cyclopropane-1-carboxylate de tert-butyle, 0,1 g d'acide paratoluène sulfonique, porte au

reflux, maintient le reflux pendant 20 minutes, concentre à sec par distillation sous pression réduite, chromatographie le résidu sur silice en éluant avec un mélange d'hexane et d'acétate d'éthyle (7/3) à 1 % d'acide acétique et obtient 1,82 g d'acide (1R cis, ΔZ) 2,2-diméthyl 3-(3-cyclopentoxy 3-oxo 1-propenyl) cyclopropane-1-carboxylique.

Spectre IR (chloroforme)

Absorption à 3 510 $cm^{-1}$ attribué à OH acide
Absorptions à 1 735 $cm^{-1}$, 1 702 $cm^{-1}$ attribués à C = O acide et l'ester conjugué.
Absorption à 1 632 $cm^{-1}$ attribué à C = C conjugué
Absorption à 1 380 $cm^{-1}$ attribué à des méthyles géminés

Préparation 12 : 3-phenoxy α-hydroxy benzène éthane thioamide

Dans une solution de 20 g d'alcool α-cyano 3-phenoxy benzylique, dans 200 $cm^3$ de toluène, et dans 4,5 $cm^3$ de triéthylamine, on fait barboter l'hydrogène sulfuré pendant 22 heures, verse le mélange réactionnel sur une solution aqueuse d'acide chlorhydrique N, sépare la phase organique par décantation, la lave à l'eau, la sèche, la concentre à sec par distillation sous pression réduite, chromatographie le résidu sur silice en éluant avec un mélange de benzène et d'acétate d'éthyle (8/2), cristallise dans l'éther isopropylique et obtient 18,5 g de 3-phenoxy α-hydroxy benzène éthane thioamide F = 70 °C.

Spectre IR (chloroforme)

Absorption à 3 600 $cm^{-1}$ attribué à l'OH libre et associé
Absorptions à 3 478, 3 360 $cm^{-1}$ attribués à —$NH_2$
Absorptions à 1 670, 1 578, 1 477 $cm^{-1}$ attribués aux noyaux aromatiques et à 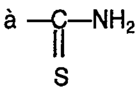

Spectre de RMN (deutérochloroforme)

Pics à 3,97-4,03 ppm attribués à l'hydrogène de l'hydroxyle
Pics à 5,18-5,25 ppm attribués à l'hydrogène porté par le carbone en α de 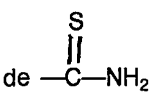
Pics de 6,92 à 7,58 ppm attribués aux hydrogènes des noyaux aromatiques
Pic à 7,5 ppm attribué aux hydrogènes du —$NH_2$

Préparation 13 : (2-phénoxy 5-thiazolyl) méthanol

Stade A : 2-phénoxy thiazole 5-carboxylate d'éthyle

On mélange 2 g de 2-chloro thiazole 5-carboxylate d'éthyle, 50 $cm^3$ de diméthylformamide, 2,5 $cm^3$ d'hexa méthyl phosphorotriamide, et 1,5 g d'iodure de sodium, porte le mélange réactionnel à 100 °C, le maintient pendant 1 heure à cette température, refroidit à 20 °C, introduit, par portions, 1,32 g de phénate de potassium, porte le mélange réactionnel au reflux pendant 1 heure 30 minutes, ajoute 0,66 g de phénate de potassium, maintient au reflux pendant 1 heure et 30 minutes refroidit, ajoute de l'eau et de l'acétate d'éthyle, extrait à l'acétate d'éthyle, lave à l'eau les phases organiques, les concentre à sec, chromatographie le résidu sur silice en éluant par un mélange d'hexane, d'éther isopropylique et de triéthylamine (7/3/1) et obtient 1,08 g de 2-phénoxy thiazole 5-carboxylate d'éthyle, F = 67 °C.

Stade B : (2-phénoxy 5-thiazolyl) méthanol

Dans une solution de 12 g de 2-phénoxy thiazole 5-carboxylate d'éthyle dans 60 $cm^3$ de toluène on introduit, lentement, à – 10 °C, 54 $cm^3$ de solution toluénique d'hydrure de diéthyl sodium aluminium titrant 2 moles/litre, agite pendant 1 heure à – 5 °C, introduit, à – 20 °C 80 $cm^3$ d'une solution aqueuse d'acide chlorhydrique 2N puis de l'eau, élimine par filtration l'insoluble formé, décante le filtrat, lave la phase organique à l'eau, par une solution aqueuse 2N de soude, puis à l'eau, concentre à sec, chromatographie le résidu sur silice en éluant par un mélange de chlorure de méthylène et d'acétate d'éthyle (8/2) et obtient 8,15 g de (2-phénoxy 5-thiazolyl) méthanol.

Spectre IR (chloroforme)

Absorption à 3 590 $cm^{-1}$ attribué à l'hydroxyle

Absorptions à 1 551, 1 530, 1 503, 1 486 $cm^{-1}$ attribués au noyau aromatique et au thiazole
Absorption à 690 $cm^{-1}$ attribué au phényl (déformation).

Spectre de RMN (deutérochloroforme)

Pic à 4,5 p.p.m. attribué aux hydrogènes de $CH_2$—O
Pic à 3,5 p.p.m. attribué à l'hydrogène de —CH
Pic à 6,66 p.p.m. attribué à l'hydrogène thiazolique
Pics de 7,10 à 7,50 p.p.m. attribués aux hydrogènes du noyau aromatique.

Préparation 14 : (RS) α-cyano (2-phénoxy 5-thiazolyl) méthanol

Stade A : (2-phénoxy 5-thiazolyl) méthanal

Dans une solution de 4,6 g de (2-phénoxy 5-thiazolyl) méthanol on introduit 19,1 g de bioxyde de manganèse, agite pendant 17 heures à 40 °C, puis pendant 3 heures à 60 °C, élimine par filtration l'insoluble résiduel, concentre à sec sous pression réduite le filtrat, chromatographie le résidu sur silice en éluant par un mélange de chlorure de méthylène et d'acétate d'éthyle (8/2) et obtient 2,6 g de (2-phénoxy 5-thiazolyl) méthanal, F = 63 °C.

Stade B : (RS) α-cyano (2-phénoxy 5-thiazolyl) méthanol

Dans une solution de 0,85 g de cyanure de sodium, dans 5 $cm^3$ d'eau, on introduit à + 10 °C, 2,4 g de (2-phénoxy 5-thiazolyl) méthanal en solution dans 10 $cm^3$ d'éther, agite pendant 10 minutes, introduit goutte à goutte à 0 °C, un mélange de 2 $cm^3$ de solution aqueuse concentrée d'acide sulfurique, et de 3 $cm^3$ d'eau, agite pendant 2 heures à 0 °C, sépare la phase organique par décantation, la lave à l'eau, la sèche, la concentre à sec sous pression réduite, ajoute au résidu de l'éther isopropylique, essore le précipité, le sèche et obtient 2,28 g de Rs α-cyano (2-phénoxy 5-thiazolyl) méthanol.

Spectre IR (chloroforme)

Absorption à 3 580 $cm^{-1}$ attribué à l'hydroxyle
Absorption à 3 550 $cm^{-1}$ attribué à l'hydroxyle associé
Absorption à 1 590, 1 504, 1 487 $cm^{-1}$ attribué au noyau aromatique et au thiazole.

Spectre de RMN (deutérochloroforme)

Pic à 4,08 p.p.m. attribué à l'hydrogène du —OH
Pic à 5,41 p.p.m. attribué à l'hydrogène de CH—OH
Pic à 7,33 p.p.m. attribué aux hydrogènes du phényle
Pic à 7,0 p.p.m. attribué à l'hydrogène thiazolique.

Préparation 15 : Alcool 3-phénoxy 4-fluoro α-méthyl benzylique

A 20 °C, on ajoute 15,8 $cm^3$ d'une solution d'iodure de méthyl magnésium à 2,6 M dans l'éther, à un mélange renfermant 8 g de 3-(4-fluorophénoxy) benzaldéhyde dans 50 $cm^3$ d'éther éthylique. On laisse une heure à température ambiante, verse le mélange dans une solution aqueuse de chlorure d'ammonium, décante, extrait à l'éther et obtient le produit attendu que l'on cristallise dans l'éther isopropylique (F = 64 °C).

Exemples de Compositions

Exemple A : Préparation d'un concentré soluble

On effectue un mélange homogène de :

| | |
|---|---|
| Produit de l'exemple 1 | 0,25 g |
| Butoxyde de pipéronyle | 1 g |
| Tween 80 | 0,25 g |
| Topanol A | 0,1 g |
| Eau | 98,4 g |

Exemple B : Préparation d'un concentré émulsifiable

On mélange intimement :

| | |
|---|---|
| Produit de l'exemple 1 | 0,015 g |
| Butoxyde de pipéronyle | 0,5 g |
| Topanol A | 0,1 g |
| Xylène | 95,885 g |
| Tween 80 | 3,5 g |

Exemple C : Préparation d'un concentré émulsifiable

On effectue un mélange homogène de :

| | |
|---|---|
| Produit de l'exemple 1 | 1,5 g |
| Tween 80 | 20 g |
| Topanol A | 0,1 g |
| Xylène | 78,4 g |

Exemple D : Préparation d'une composition fumigène

On mélange d'une façon homogène :

| | |
|---|---|
| Produit de l'exemple 1 | 0,25 g |
| Poudre de tabu | 25 g |
| Poudre de feuille de cèdre | 40 g |
| Poudre de bois de pin | 33,75 g |
| Vert brillant | 0,5 g |
| p-nitrophénol | 0,5 g |

Etude de l'activité des composés selon l'invention sur les parasites

Exemple 1 : Etude de l'effet létal des composés des exemples 1 à 8 sur mouches domestiques

Les insectes tests sont des mouches domestiques femelles de souche sensible aux pyréthrinoïdes élevées à 22-23 °C et 60-65 % d'humidité relative et âgées de 4 à 5 jours. On opère par application topique de 1 µl de solution acétonique sur le thorax dorsal des insectes à l'aide du micro manipulateur d'Arnold. On utilise 50 individus par dose du produit à tester. On effectue le contrôle de mortalité vingt-quatre heures après traitement.

On constate que dans le test utilisé les produits présentent une bonne activité létale.

Exemple 2 : Etude de l'effet létal des composés des exemples 1 à 8 sur larves de Spodoptera littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24 °C et 65 % d'humidité relative. Après traitement, les individus sont placés sur un milieu nutritif artificiel (milieu de Poitout).

On effectue le contrôle des mortalités 48 heures après traitement.

On constate que dans le test utilisé, les produits présentent une bonne activité létale.

Exemple 3 : Etude de l'activité des produits des exemples 1 à 8 sur larves d'Epilachna Varivestris

Les essais sont effectués par application topique de manière analogue à celle utilisée pour les mouches et les larves de Spodoptera. On utilise des larves de l'avant dernier stade larvaire et après traitement les larves sont alimentées par des plants de haricots. On effectue le contrôle de mortalité 72 heures après traitement.

On constate que dans le test utilisé, les produits présentent une bonne activité létale.

Exemple 4 : Etude de l'activité de choc sur mouche domestique

Les insectes tests sont des mouches domestiques femelles âgées de 4 à 5 jours. On opère par pulvérisation directe en cylindre de Kearns et March en utilisant comme solvant un mélange d'acétone (5 %) et d'Isopar L (solvant pétrolier) (quantité de solvant utilisée 2 ml en une seconde). On utilise 50 insectes par dose. On effectue les contrôles toutes les minutes jusqu'à 10 minutes, puis à 15 minutes et l'on détermine le KT 50 par les méthodes habituelles.

On constate que les produits présentent une bonne activité, l'un d'entre eux, le produit de l'exemple 1 présente une activité remarquable puisque son KT 50 est de 0,4 mn, 1,26 mn et 2,02 mn pour des concentrations respectives de 1, 0,5 et 0,25 g/litre.

Exemple 5 : Activité sur Tetranychus Urticae

Essai adulticide

On utilise des plants de haricots comportant deux feuilles qui sont traitées au pistolet Fisher à différentes doses des produits à tester. Après séchage ces plants sont infestés à raison de 25 femelles de Tetranychus Urticae par feuilles et maintenus à 22-23 °C, 60-65 % d'humidité relative artificielle permanente. Les dénombrements des acariens vivants et morts sont effectués 24 heures et 48 heures après traitement.

Les produits des exemples 1 à 8 présentent une bonne activité adulticide dans ce test.

Exemple 6 : Etude de l'activité insecticide, sur moustiques Culex pipiens, du composé de l'exemple 1 utilisé sous forme de serpentin fumigène

Des supports neutres de serpentins fumigènes sont imprégnés de matière active en solution dans l'acétone. On lâche dans un cylindre fermé, en verre, de 13,50 $dm^3$, 20 moustiques femelles âgées de 4 à 5 jours et introduit, pendant 2 minutes, un serpentin fumigène se consumant à une extrémité. On effectue le contrôle de knock-down toutes les minutes et arrête l'essai 5 minutes après que tous les insectes aient été abattus.

Les résultats sont les suivants :

— à la dose de 0,60 % de matière active pour poids de coïl, le KT 50 est de 5,42 mn.

— à cette dose l'effet létal est de 98,3 %.

**Revendications** (pour les Etats contractants : BE, CH, DE, FR, GB, IT, LI, LU, NL, SE)

1. Sous toutes les formes isomères possibles, les composés de formule (I')

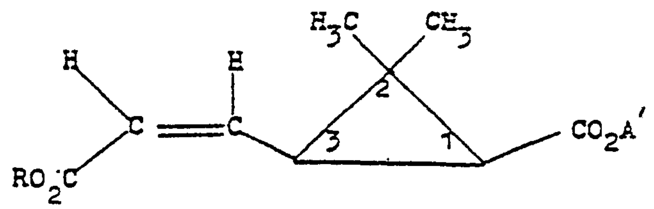

(I')

dans laquelle la double liaison a la géométrie Z, la copule cyclopropanique est de structure 1R cis et A' représente :

soit un radical alcoyle renfermant de 1 à 18 atomes de carbone,

soit un radical benzyle éventuellement substitué par un ou plusieurs radicaux choisis dans le groupe constitué par les radicaux alcoyles cómportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le reste méthylène dioxy, et les atomes d'halogène,

soit un groupement

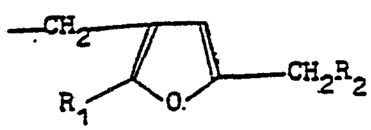

dans lequel le substituant $R_1$ représente un atome d'hydrogène ou un radical méthyle et le substituant $R_2$ un aryle monocyclique ou un groupement $-CH_2-C \equiv CH$ et notamment un groupement 5-benzyl 3-furylméthyle,

soit un groupement

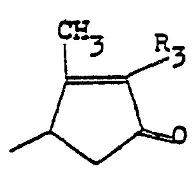

dans lequel $R_3$ représente un radical organique aliphatique comportant de 2 à 6 atomes de carbone et une ou plusieurs insaturations carbone-carbone et notamment les radicaux $-CH_2-CH=CH_2$, $-CH_2-CH=CH-CH_3$, $-CH_2-CH=CH-C_2H_5$, $-CH_2-CH=CH-CH=CH_2$

soit un groupement

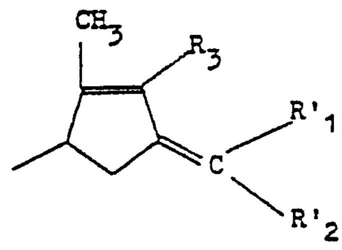

dans lequel $R_3$ conserve la même signification que précédemment, $R'_1$ et $R'_2$ identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

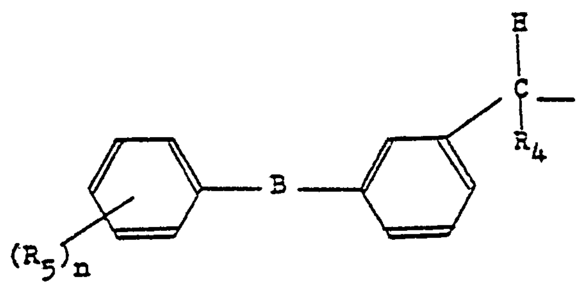

dans lequel B représente un groupement $CH_2$, ou C = O, ou un hétéroélément choisi parmi l'oxygène et le soufre, $R_4$ représente un atome d'hydrogène, un radical méthyle, un radical $—CONH_2$, un radical $—CSNH_2$ ou un radical :

$$—C \equiv CH$$

$R_5$ représente un atome d'halogène ou un radical méthyle et n représente un nombre égal à 0, 1 ou 2, et notamment le groupement 3-phénoxy benzyle, α-éthynyl 3-phénoxy benzyle, 3-benzoylbenzyle, 1-(3-phénoxy phényl) éthyle ou α-thioamido 3-phénoxy benzyle.

soit un groupement

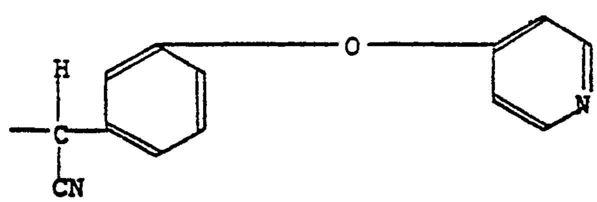

soit un groupement

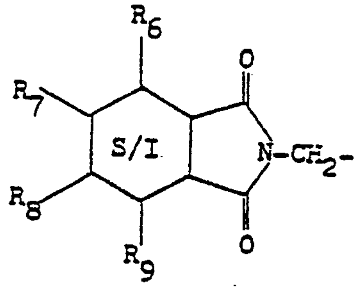

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

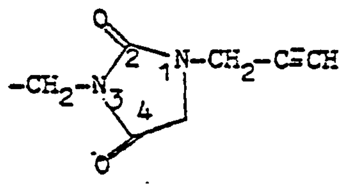

soit un groupement

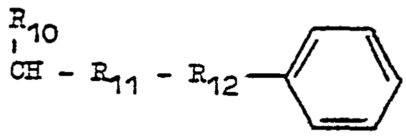

dans lequel $R_3$ conserve la même signification que précédemment, $R_1'$ et $R_2'$ identiques ou différents, ou un atome d'oxygène, $R_{11}$ représente un radical thiazolyle ou thiadiazolyle dont la liaison avec

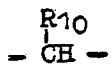

peut se trouver à l'une quelconque des positions disponibles, $R_{12}$ étant lié à $R_{11}$ par l'atome de carbone compris entre l'atome de soufre et un atome d'azote,

soit un groupement

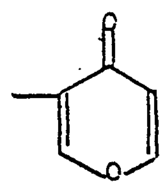

soit un groupement

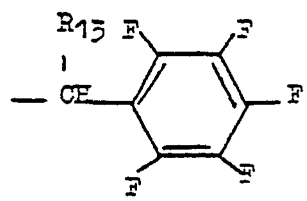

dans lequel $R_{13}$ représente un atome d'hydrogène ou un radical CN,

soit un groupement

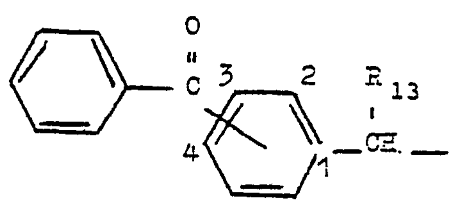

dans lequel $R_{13}$ est défini, comme ci-dessus et le radical benzoyle est en position 3 ou 4,

soit un groupement

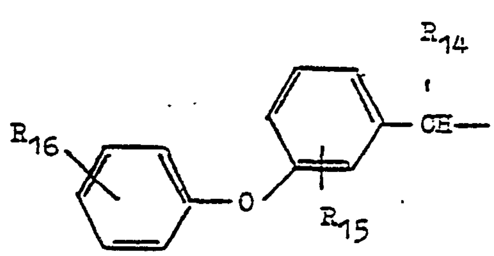

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

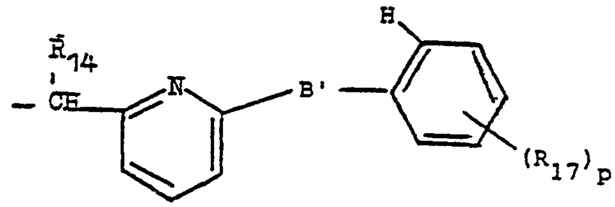

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou bromo, p représente un nombre égal à 0,1 ou 2 et B' représente un atome d'oxygène ou un atome de soufre,

et R représente un radical alcoyle linéaire, ramifié ou cyclique, saturé ou insaturé, renfermant de 1 à 18 atomes de carbone, ainsi que les mélanges de ces isomères.

2. Sous toutes les formes isomères possibles, les composés de formule (I') tels que définis à la revendication 1, répondant à la formule (I)

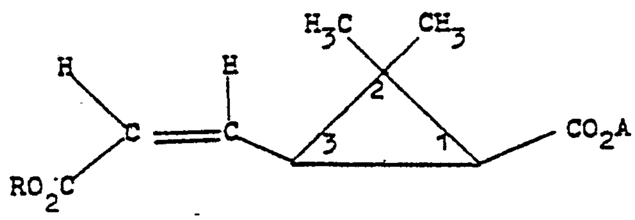

(I)

dans laquelle la double liaison a la géométrie Z, et la copule cyclopropanique est de structure 1R cis, A représente,

soit un radical alcoyle renfermant de 1 à 18 atomes de carbone,

soit un radical benzyle éventuellement substitué tel que défini à la revendication 1,

soit un groupement

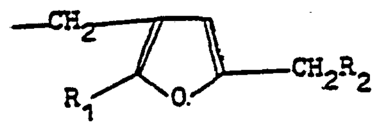

dans lequel les substituants $R_1$ et $R_2$ sont tels que définis à la revendication 1,

soit un groupement

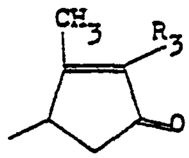

dans lequel $R_3$ est tel que défini à la revendication 1,

soit un groupement

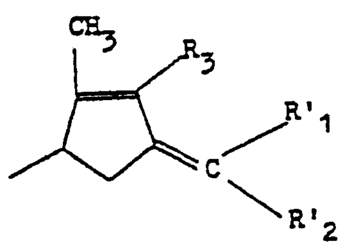

dans lequel $R_3$ $R_1'$ et $R_2'$ sont tels que définis à la revendication 1,

soit un groupement

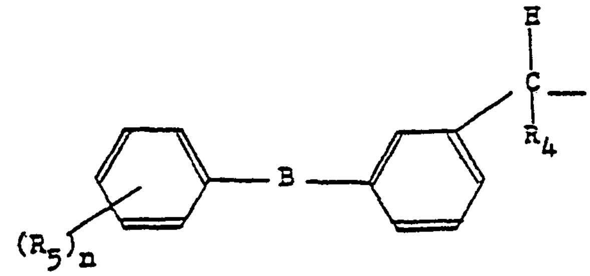

dans lequel B, $R_4$, $R_5$ et n sont tels que définis à la revendication 1,

soit un groupement

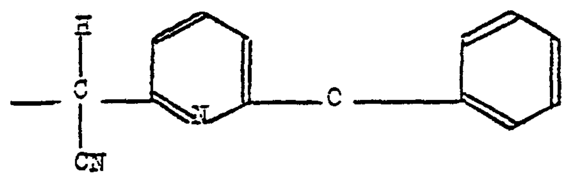

soit un groupement

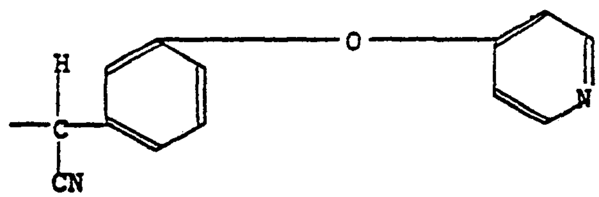

soit un groupement

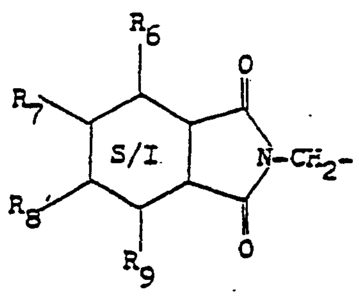

dans lequel les substituants $R_6$, $R_7$, $R_8$, $R_9$ et S/I sont tels que définis à la revendication 1,

soit un groupement

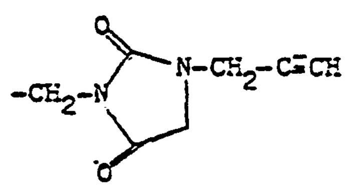

et R est tel que défini à la revendication 1, ainsi que les mélanges de ces isomères.

3. Les composés de (I') tels que définis à l'une quelconque des revendications 1 ou 2, pour lesquels R représente un radical méthyle.

4. Les composés de formule (I') tels que définis à l'une quelconque des revendications 1 ou 2, pour lesquels R représente un radical éthyle, n-propyle, isopropyle, tert-butyle ou cyclopropylméthyle.

5. Les composés de formule (I') tels que définis à l'une quelconque des revendications de 1 à 4, pour lesquels A' est choisi dans le groupe constitué par le groupement (4S) 3-méthyl 2-(2-propenyl) 1-oxo cyclopent-2-en-yl, le groupement (1,3,4,5,6,7 hexahydro-1,3-dioxo-2H-isoindol-2-yl) méthyl, le groupement (RS) cyano (6-phénoxy-2-pyridinyl) méthyle, le groupement [5(phénylméthyl)-3-furanyl] méthyl, le groupement 1-(3-propargyl 2,5-dioxo imidazolidinyl) méthyle et le groupement (R) 3-phénoxyphényl éthyle.

6. L'un quelconque des composés de formule (I') dont les noms suivent :

le (1R cis) 2,2-diméthyl-3[(Z) 3-méthoxy-3-oxo-1-propényl] cyclopropane carboxylate de (1S) 2-méthyl-4-oxo 3-(2-propényl) 2-cyclopenten-1-yl ;

le (1R cis) 2,2-diméthyl-3[(Z) 3-tert-butoxy 3-oxo-1-propényl] cyclopropane carboxylate de 1-(3-propargyl 2,5-dioxo imidazolidinyl) méthyle ;

le (1R cis) 2,2-diméthyl 3[(Z) 2-cyclopropylméthoxy carbonyl éthényl] cyclopropane carboxylate de 1-(3-propargyl 2,5-dioxo imidazolidinyl) méthyle ;

le (1R cis) 2,2-diméthyl 3[(Z) 2-isopropoxycarbonyléthényl] cyclopropane carboxylate de 1(R) 3-phénoxyphényl éthyle ;

le (1R cis) 2,2-diméthyl 3[(Z) 2-isopropoxycarbonyléthényl] cyclopropane carboxylate de 1-(3-propargyl 2,5-dioxo imidazolidinyl) méthyle ;

7. Procédé de préparation des composés de formule (I') tels que définis à l'une quelconque des revendications 1 à 6, caractérisé en ce que l'on soumet un acide de formule (II)

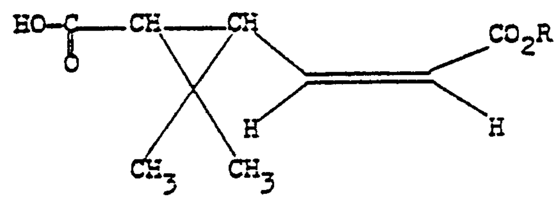

(II)

dans laquelle la double liaison a la géométrie Z et la copule cyclopropanique est de structure 1R cis, R étant défini comme à la revendication 1, ou un dérivé fonctionnel de cet acide, avec un alcool de formule (III)

A'OH

dans laquelle A' conserve la même signification que dans la revendication 1 et obtient ainsi le composé de formule (I') correspondant.

8. Procédé de préparation des composés de formule (I') tels que définis à l'une quelconque des revendications 1 à 6, caractérisé en ce que l'on soumet un composé de formule (VIII)

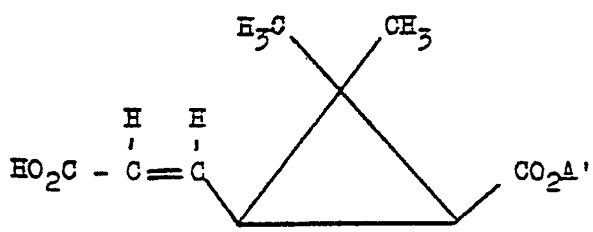

(VIII)

dans lequel la copule cyclopropanique est de structure 1R cis et A' est défini comme à la revendication 1, à l'action d'un agent d'estérification, pour obtenir le composé de formule (I') correspondant.

9. Procédé selon la revendication 8, caractérisé en ce que le composé de formule (VIII) est préparé en soumettant un composé de formule (IX) :

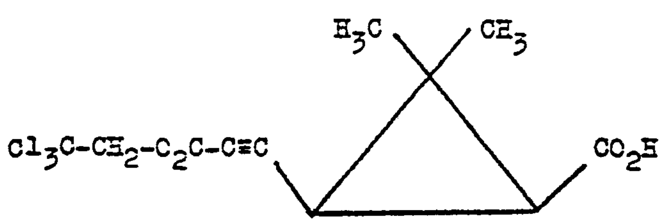

(IX)

à l'action d'un alcool de formule (III)

A'—OH (III)

dans lequel A' est défini comme à la revendication 1, pour obtenir le composé de formule (X)

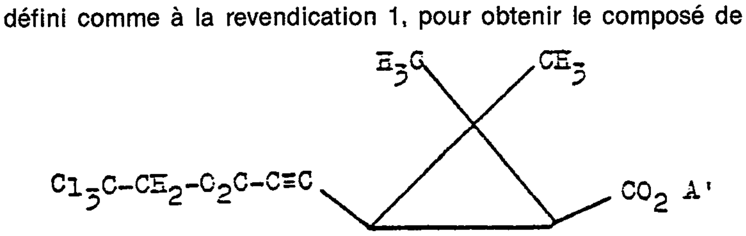

(X)

que l'on soumet à l'action d'un agent de clivage de la fonction ester portée par le carbone acétylénique, pour obtenir le composé de formule (XI)

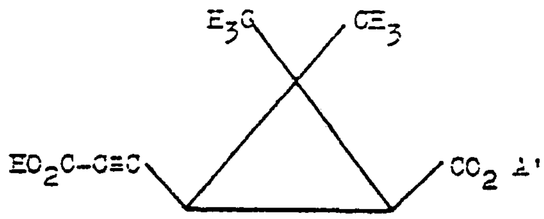

(XI)

que l'on soumet à l'action d'un agent d'hydrogénation ménagée pour obtenir le composé de formule (VIII).

10. Procédé selon la revendication 8 ou 9, caractérisé en ce que l'on soumet un composé de formule (XI)

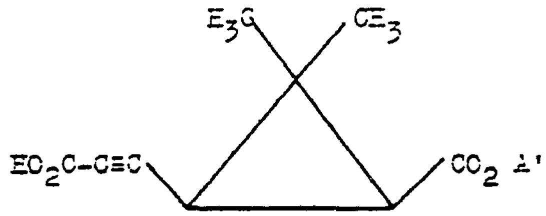

(XI)

dans laquelle A' est défini comme à la revendication 1, à l'action d'un agent d'estérification, pour obtenir le composé de formule (XII)

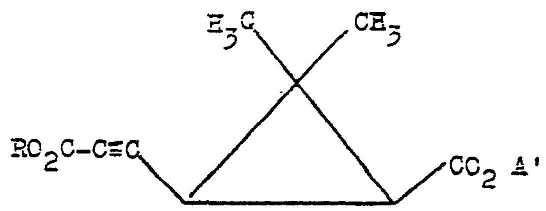

(XII)

dans laquelle R et A' sont définis comme à la revendication 1, que l'on soumet à l'action d'un agent d'hydrogénation ménagée, pour obtenir le composé de formule (I').

11. A titre de produits industriels nouveaux et notamment à titre de produits intermédiaires, les produits de formules (VIII), (X), (XI) et (XII), telles que définies à la revendication 9 ou 10.

12. Application des composés de formule (I') tels que définis à l'une quelconque des revendications 1 à 6, à la lutte contre les parasites des végétaux, les parasites domestiques, et les parasites des animaux à sang chaud.

13. Les compositions destinées à la lutte contre les parasites des végétaux, les parasites domestiques et les parasites des animaux à sang chaud, caractérisées en ce qu'elles renferment au moins l'un des produits définis à l'une quelconque des revendications 1 à 6.

14. Les compositions insecticides renfermant comme principe actif au moins l'un des produits définis à l'une quelconque des revendications 1 à 5.

15. Les compositions insecticides renfermant comme principe actif l'un au moins des produits définis à la revendication 6.

16. Les compositions destinées à l'alimentation animale renfermant comme principe actif au moins l'un des produits définis à l'une quelconque des revendications 1 à 6.

17. Associations douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5-6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I' peuvent exister sous toutes leurs formes stéréoisomères possibles de même que les copules acides et alcools des esters pyréthrinoïdes ci-dessus.

**Revendications** (pour l'Etat contractant AT)

1. Procédé pour préparer sous toutes les formes isomères possibles, les composés de formule (I')

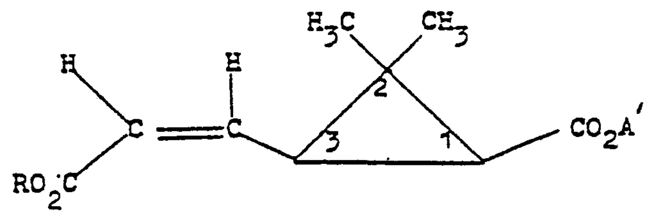

(I')

dans laquelle la double liaison a la géométrie Z, A' représente :

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le reste méthylène dioxy, et les atomes d'halogène,

soit un groupement

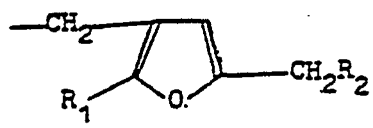

dans lequel le substituant $R_1$ représente un atome d'hydrogène ou un radical méthyle et le substituant $R_2$ un aryle monocyclique ou un groupement $-CH_2-C \equiv CH$ et notamment un groupement 5-benzyl 3-furylméthyle,

soit un groupement

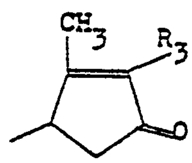

dans lequel $R_3$ représente un radical organique aliphatique comportant de 2 à 6 atomes de carbone et une ou plusieurs insaturations carbone-carbone et notamment les radicaux $-CH_2-CH=CH_2$, $-CH_2-CH=CH-CH_3$, $-CH_2-CH=CH-C_2H_5$, $-CH_2-CH=CH-CH=CH_2$

soit un groupement

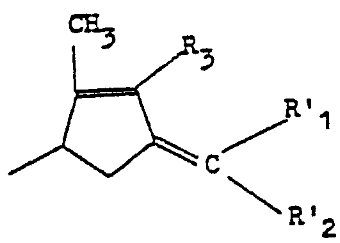

dans lequel $R_3$ conserve la même signification que précédemment, $R'_1$ et $R'_2$ identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

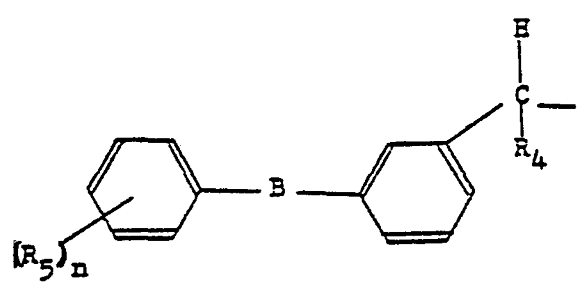

dans lequel B représente un groupement $CH_2$ ou C=O, ou un hétéroélément choisi parmi l'oxygène et le

soufre, $R_4$ représente un atome d'hydrogène, un radical méthyle, un radical $—CONH_2$, un radical $—CSNH_2$ ou un radical :

$$—C{\equiv}CH$$

$R_5$ représente un atome d'halogène ou un radical méthyle et n représente un nombre égal à 0, 1 ou 2, notamment le groupement 3-phénoxy benzyle, α-éthynyl 3-phénoxy benzyle, 3-benzoylbenzyle, 1-(3-phénoxy phényl) éthyle ou α-thioamido 3-phénoxy benzyle,

soit un groupement

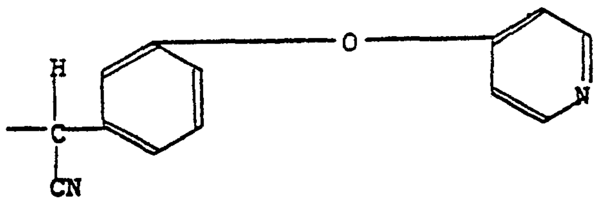

soit un groupement

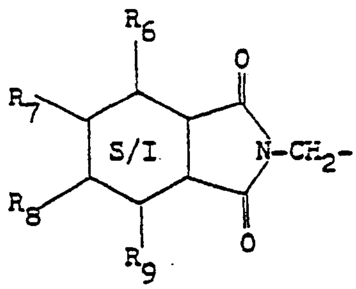

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

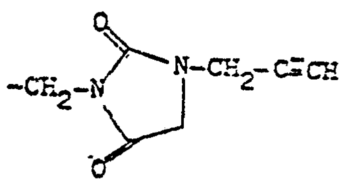

soit un groupement

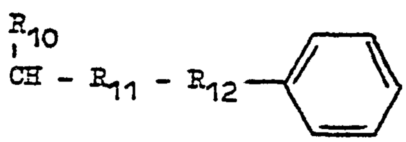

dans lequel $R_{10}$ représente un atome d'hydrogène ou un radical CN, $R_{12}$ représente un radical $—CH_2—$ ou un atome d'oxygène, $R_{11}$ représente un radical thiazolyle ou thiadiazolyle dont la liaison avec

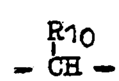

peut se trouver à l'une quelconque des positions disponibles, $R_{12}$ étant lié à $R_{11}$ par l'atome de carbone compris entre l'atome de soufre et un atome d'azote,

soit un groupement

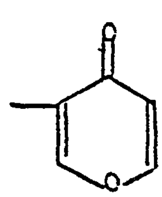

soit un groupement

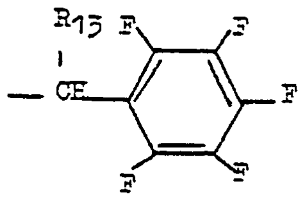

dans lequel $R_{13}$ représente un atome d'hydrogène ou un radical CN,
soit un groupement

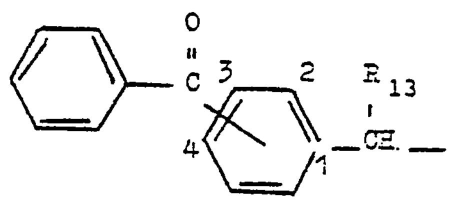

dans lequel $R_{13}$ est défini, comme ci-dessus et le radical benzoyle est en position 3 ou 4,
soit un groupement

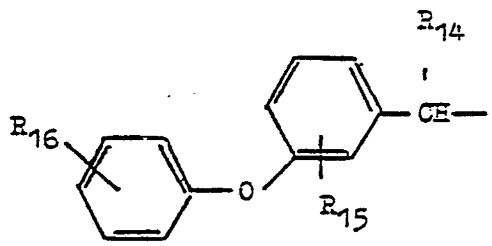

dans lequel $R_{14}$ représente un atome d'hydrogène, un radical méthyle, éthymyle ou cyano et $R_{15}$ et $R_{16}$, différents, représentent un atome d'hydrogène, de fluor ou de brome,
soit un groupement

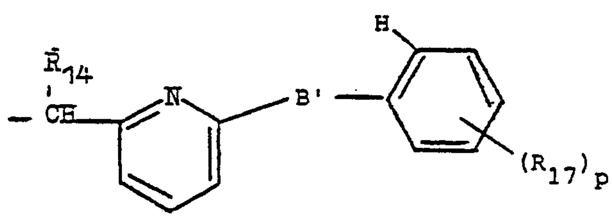

. dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre,

et R représente un radical alcoyle linéaire, ramifié ou cyclique, saturé ou insaturé, renfermant de 1 à 18 atomes de carbone, ainsi que les mélanges de ces isomères, caractérisé en ce que l'on soumet un acide de formule (II)

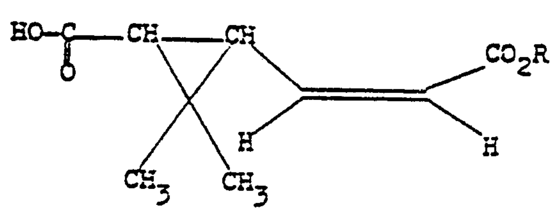

(II)

dans laquelle la double liaison a la géométrie Z, R étant défini comme précédemment ou un dérivé fonctionnel de cet acide, avec un alcool de formule (III)

A'OH (III)

dans laquelle A' conserve la même signification que précédemment et obtient ainsi le composé de formule (I') correspondant.

2. Procédé selon la revendication 1, pour préparer sous toutes les formes isomères possibles, les composés de formule (I') tels que définis à la revendication 1, répondant à la formule (I)

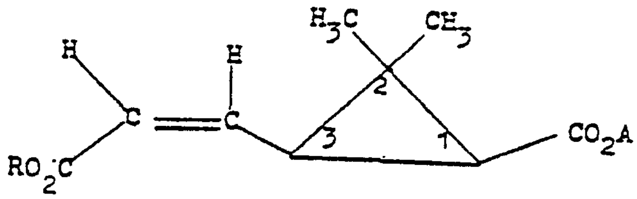

(I)

dans laquelle la double liaison a la géométrie Z, A représente,

soit un radical alcoyle renfermant de 1 à 18 atomes de carbone,

soit un radical benzyle éventuellement substitué tel que défini à la revendication 1,

soit un groupement

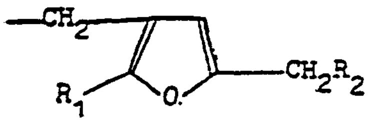

dans lequel les substituants $R_1$ et $R_2$ sont tels que définis à la revendication 1,

soit un groupement

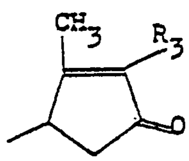

dans lequel $R_3$ est tel que défini à la revendication 1,

soit un groupement

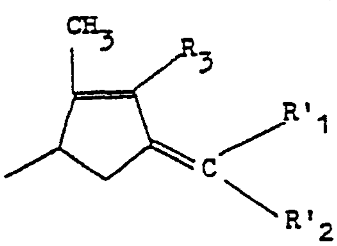

dans lequel $R_3$, $R_1'$ et $R_2'$ sont tels que définis à la revendication 1,

soit un groupement

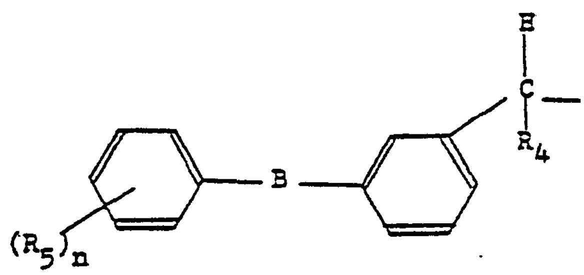

dans lequel B, $R_4$, $R_5$ et n sont tels que définis à la revendication 1,

soit un groupement

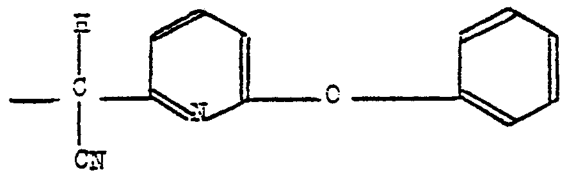

soit un groupement

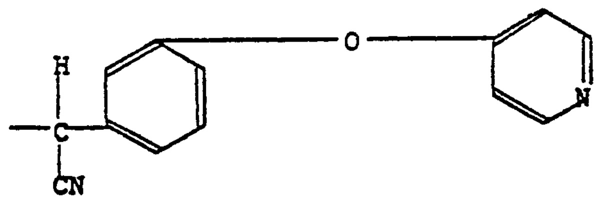

soit un groupement

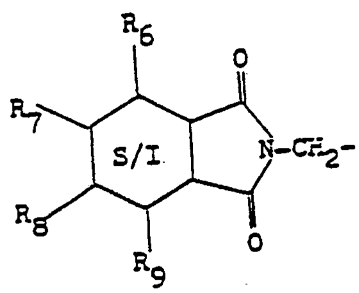

dans lequel les substituants $R_6$, $R_7$, $R_8$, $R_9$ et S/I sont tels que définis à la revendication 1, soit un groupement

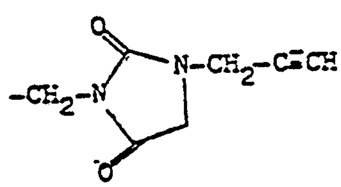

et R est tel que défini à la revendication 1, ainsi que les mélanges de ces isomères, caractérisé en ce que l'on utilise au départ un alcool de formule III dans laquelle A' a les valeurs de A indiquées précédemment.

3. Procédé de préparation des composés de formule (I'), tels que définis à la revendication 1, caractérisé en ce que l'on soumet un composé de formule (VIII)

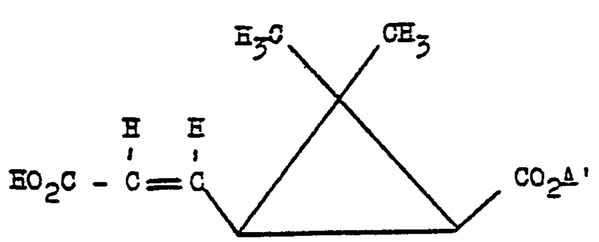

(VIII)

dans lequel A' est défini comme à la revendication 1, à l'action d'un agent d'estérification, pour obtenir le composé de formule (I') correspondant.

4. Procédé selon la revendication 3, caractérisé en ce que le composé de formule (VIII) est préparé en soumettant un composé de formule (IX) :

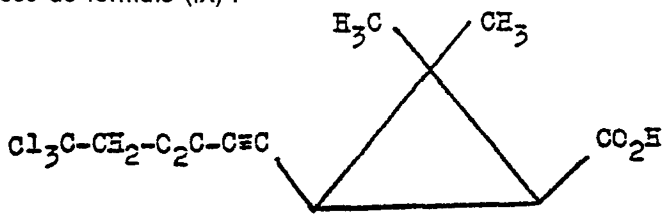

(IX)

à l'action d'un alcool de formule (III)

A'—OH (III)

dans lequel A' est défini comme à la revendication 1, pour obtenir le composé de formule (X)

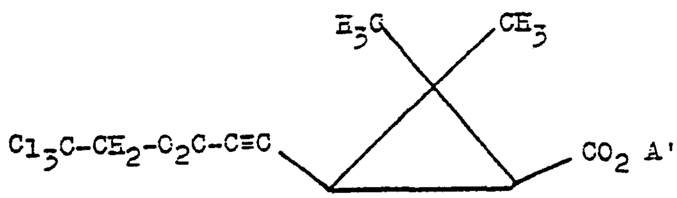

(X)

que l'on soumet à l'action d'un agent de clivage de la fonction ester portée par le carbone acétylinique, pour obtenir le composé de formule (XI)

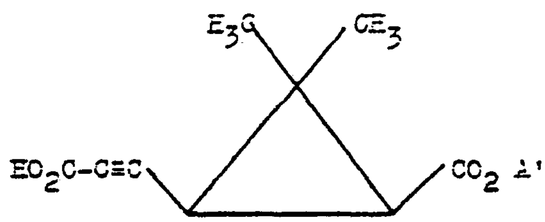

(XI)

que l'on soumet à l'action d'un agent d'hydrogénation ménagée pour obtenir le composé de formule (VIII).

5. Procédé selon la revendication 3 ou 4, caractérisé en ce que l'on soumet un composé de formule (XI)

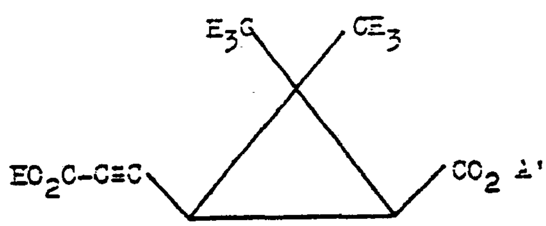

(XI)

dans laquelle A' est défini comme à la revendication 1, à l'action d'un agent d'estérification, pour obtenir le composé de formule (XII)

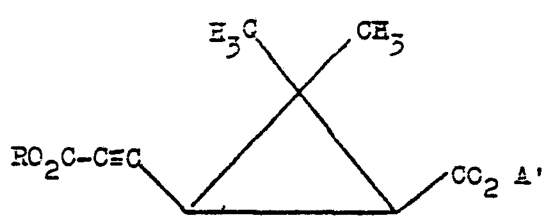

(XII)

dans laquelle R et A' sont définis comme à la revendication 1, que l'on soumet à l'action d'un agent d'hydrogénation ménagée, pour obtenir le composé de formule (I').

6. Procédé selon l'une quelconque des revendications 1, 3, 4 et 5, caractérisé en ce que l'on utilise au départ un composé de formule II, VIII, IX ou XI dont la structure est 1R cis ou 1R trans.

7. Procédé selon l'une quelconque des revendications 1, 3, 4 et 5, caractérisé en ce que l'on utilise au départ un composé de formule II ou un agent d'estérification, dans lequel R représente un radical méthyle, éthyle, n-propyle, isopropyle, terbutyle ou cyclopropyl méthyle.

8. Procédé selon l'une quelconque des revendications 1, 3, 4 et 5, caractérisé en ce que l'on utilise au départ un composé de formule III, VIII ou XI dans lequel A' est choisi dans le groupe constitué par le groupement (4S) 3-méthyl 2-(2-propenyl) 1-oxo-cyclopent-2-en-4-yl, le groupement (1,3,4,5,6,7 hexahydro-1,3-dioxo-2H-isoindol-2-yl) méthyl, le groupement (RS) cyano (6-phénoxy-2-pyridinyl) méthyle, le groupement [5 (phénylméthyl)-3-furanyl]méthyl, le groupement 1-(3-propargyl 2,5-dioxo imidazolidinyl) méthyle et le groupement (R) 3-phénoxyphényl éthyle.

9. Procédé selon la revendication 2, caractérisé en ce que l'on utilise au départ un composé de formule II de structure 1R cis ou 1R trans, dans lequel R représente un radical méthyle, éthyle, n-propyle, isopropyle, terbutyle ou cyclopropyl méthyle et un alcool de formule III dans lequel A' est choisi dans le groupe constitué par le groupement (4S) 3-méthyl 2-(2-propenyl) 1-oxo cyclopent-2-en-4-yl, le groupement (1,3,4,5,6,7 hexahydro-1,3-dioxo-2H-isoindol-2-yl) méthyl, le groupement (RS) cyano (6-phénoxy-2-pyridinyl) méthyle, le groupement [5 (phénylméthyl)-3-furanyl]méthyl, le groupement 1-(3-propargyl 2,5-dioxo imidazolidinyl) méthyle et le groupement (R) 3-phénoxy phényl éthyle.

10. Procédé selon l'une quelconque des revendications 1 à 5, caractérisé en ce que l'on prépare l'un quelconque des composés de formule I' dont les noms suivent :

— le (1R cis) 2,2-diméthyl-3[(Z) 3-méthoxy-3-oxo-1-propenyl]cyclopropane carboxylate de (1S) 2-méthyl-4-oxo 3-(2-propenyl) 2-cyclopenten-1-yl ;

— le (1R cis) 2,2-diméthyl-3[(Z) 3-terbutoxy 3-oxo 1-propenyl]cyclopropane carboxylate de 1-(3-propargyl 2,5-dioxo imidazolidinyl) méthyle ;

— le (1R cis) 2,2-diméthyl-3[(Z) 2-cyclopropyl méthoxy carbonyl éthényl]cyclopropane carboxylate de 1-(3-propargyl 2,5-dioxo imidazolidinyl) méthyle ;

— le (1R cis) 2,2-diméthyl-3[(Z) 2-isopropoxycarbonyl éthényl]cyclopropane carboxylate de 1(R) 3-phénoxyphényl éthyle ;

— le (1R cis) 2,2-diméthyl-3[(Z) 2-isopropoxycarbonyl éthényl]cyclopropane carboxylate de 1-(3-propargyl 2,5-dioxo imidazolidinyl) méthyle ;

— le (1R trans) 2,2-diméthyl-3[(Z) 2-isopropoxycarbonyl éthényl]cyclopropane carboxylate de 1(R) 3-phénoxyphényl éthyle.

11. Application des composés de formule (I') tels que définis à la revendication 1, à la lutte contre les parasites des végétaux, les parasites domestiques et les parasites des animaux à sang chaud.

12. Application des composés de formule (I') tels que définis à la revendication 2, à la lutte contre les parasites des végétaux, les parasites domestiques et les parasites des animaux à sang chaud.

13. Les compositions destinées à la lutte contre les parasites des végétaux, les parasites domestiques et les parasites à sang chaud, caractérisées en ce qu'elles renferment au moins l'un des produits définis à la revendication 1.

14. Les compositions insecticides renfermant comme principe actif au moins l'un des produits définis à la revendication 1.

15. Les compositions insecticides renfermant comme principe actif l'un au moins des produits définis à la revendication 10.

16. Les compositions destinées à l'alimentation animale renfermant comme principe actif au moins l'un des produits définis à la revendication 1.

17. Compositions douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5-6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I' peuvent exister sous toutes leurs formes stéréoisomères possibles de même que les copules acides et alcools des esters pyréthrinoïdes ci-dessus.

18. Compositions selon l'une quelconque des revendications 13 à 17, caractérisées en ce que les composés de formule I' répondent à la formule I, telle que définie à la revendication 2.

**Claims** (for the contracting States : BE, CH, DE, FR, GB, IT, LI, LU, NL, SE)

1. In all the possible isomer forms, the compounds with the formula (I') :

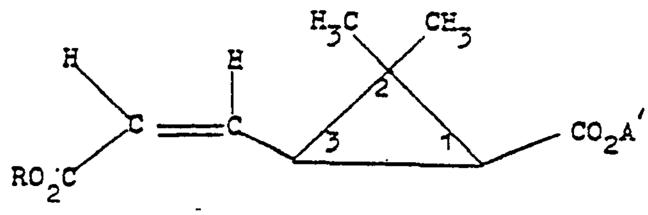

(I')

in which the double bond has Z geometry, the cyclopropane copula is of 1R cis structure and A' represents :

either an alkyl radical containing 1-18 carbon atoms,

or a benzyl radical possibly substituted by one or more radicals chosen from the group constituted by alkyl radicals containing 1-4 carbon atoms, alkenyl radicals containing 2-6 carbon atoms, alkenyloxy radicals containing 2-6 carbon atoms, alkadienyl radicals containing 4-8 carbon atoms, the methylenedioxy residue, and the halogen atoms,

or a

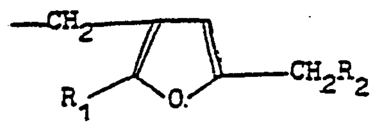

group, in which the substituent $R_1$ represents a hydrogen atom or a methyl radical and the substituent $R_2$ represents a monocyclic aryl or a $—CH_2—C{\equiv}CH$ group and notably a 5-benzyl-3-furylmethyl group,

or a

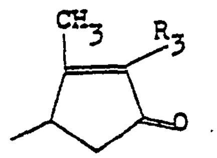

group in which $R_3$ represents an aliphatic organic radical containing 2-6 carbon atoms and one or more carbon-carbon unsaturations and notably the radicals $—CH_2—CH{=}CH_2$, $—CH_2—CH{=}CH—CH_3$, $—CH_2—CH{=}CH—C_2H_5$, $—CH_2—CH{=}CH—CH{=}CH_2$,

or a

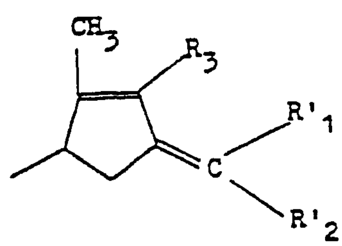

group, in which $R_3$ retains the same significance as previously, each of $R_1'$ and $R_2'$, identical or different, represents a hydrogen atom, a halogen atom, an alkyl radical containing 1-6 carbon atoms, an aryl radical containing 6-10 carbon atoms, an alkyloxycarbonyl group containing 2-5 carbon atoms, or a cyano group,

or a

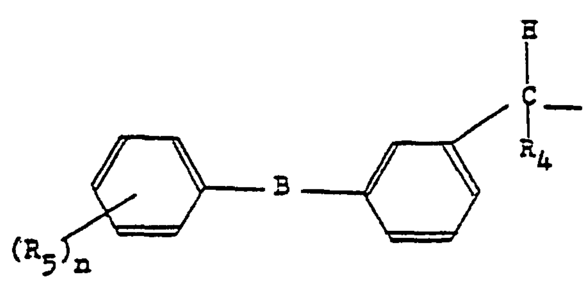

group, in which B represents a $CH_2$ group, or a C=O group, or a hetero element chosen from oxygen and sulphur, $R_4$ represents a hydrogen atom, a methyl radical, a $-CONH_2$ radical, a $-CSNH_2$ radical or a

$-C\equiv CH$ radical

$R_5$ represents a halogen atom or a methyl radical and n represents a numeral 0, 1 or 2, and notably the 3-phenoxybenzyl, α-ethynyl-3-phenoxybenzyl, 3-benzoylbenzyl, 1-(3-phenoxyphenyl) ethyl or α-thioamido-3-phenoxybenzyl groups,

or a

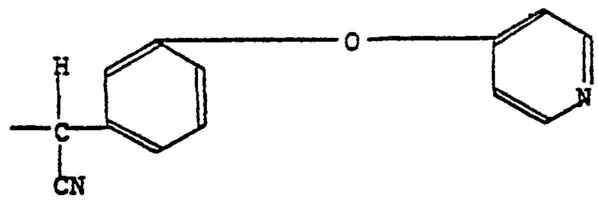

group,

or a

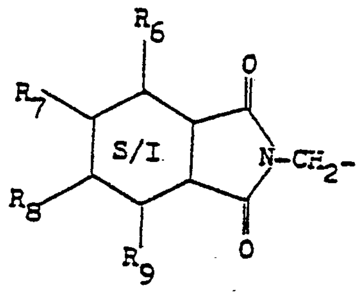

group, in which each of the substituents $R_6$, $R_7$, $R_8$, $R_9$ represents a hydrogen atom, a chlorine atom, or a methyl radical and in which S/I signifies an aromatic ring or a similar dihydro or tetrahydro ring,

or a

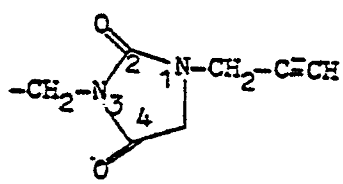

group,

or a

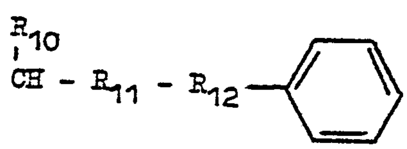

group, in which $R_{10}$ represents a hydrogen atom or a CN radical, $R_{12}$ represents a $-CH_2-$ radical or an oxygen atom, $R_{11}$ represents a thiazolyl or thiadiazolyl radical of which the bond with

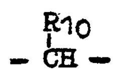

may be found at any one of the available positions, $R_{12}$ being linked to $R_{11}$ by the carbon atom situated between the sulphur atom and a nitrogen atom,

or a

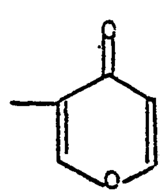

group,

or a

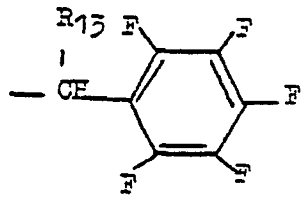

group in which $R_{13}$ represents a hydrogen atom or a CN radical,

or a

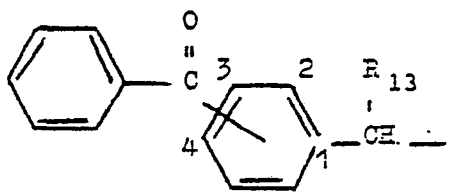

group, in which $R_{13}$ is defined as above and the benzoyl radical is in position 3 or 4,

or a

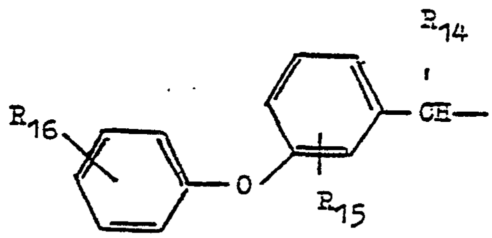

group, in which $R_{14}$ represents a hydrogen atom, a methyl, ethynyl or cyano radical and $R_{15}$ and $R_{16}$, which are different from one another, represents a hydrogen, fluorine or bromine atom,

or a

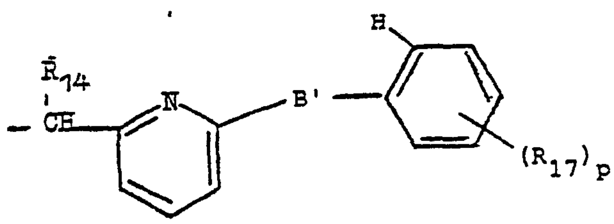

group in which $R_{14}$ is defined as above, each of the $R_{17}$'s represents, independently, an alkyl group containing 1-4 carbon atoms, an alkoxy group containing 1-4 carbon atoms, an alkylthio group containing 1-4 carbon atoms, an alkylsulphonyl group containing 1-4 carbon atoms, or a trifluoromethyl, 3,4-methylenedioxy, chloro, fluoro, or bromo group, p represents a numeral 0, 1 or 2 and B′ represents an oxygen atom or a sulphur atom, and R represents an alkyl radical, linear, branched or cyclic, saturated or unsaturated, containing 1-18 carbon atoms, as well as the mixtures of these isomers.

2. In all the possible isomer forms, the compounds with the formula (I′) as defined in Claim 1, corresponding to formula (I)

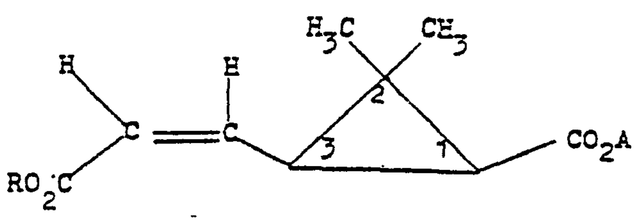

(I)

in which the double bond has Z geometry and the cyclopropane copula is of 1Rcis structure, A represents, either an alkyl radical containing 1-18 carbon atoms,
or a benzyl radical possibly substituted as defined in Claim 1,
or a

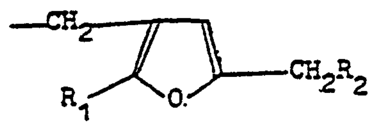

group, in which the substituents $R_1$ and $R_2$ are as defined in Claim 1,
or a

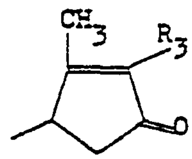

group, in which $R_3$ is as defined in Claim 1,
or a

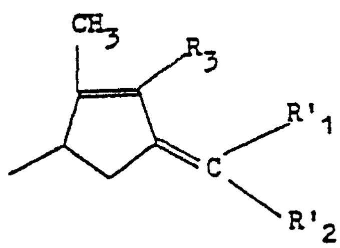

group, in which $R_3$, $R_1'$ and $R_2'$ are as defined in Claim 1,
or a

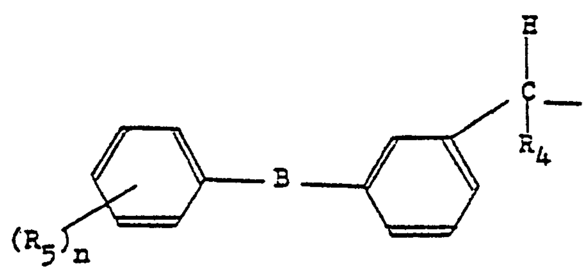

group, in which B, $R_4$, $R_5$ and n are as defined in Claim 1,
or a

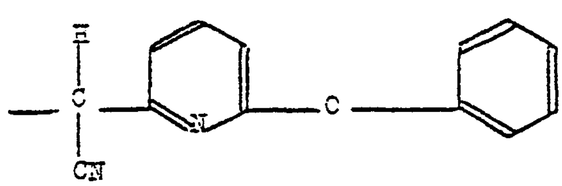

group,
or a

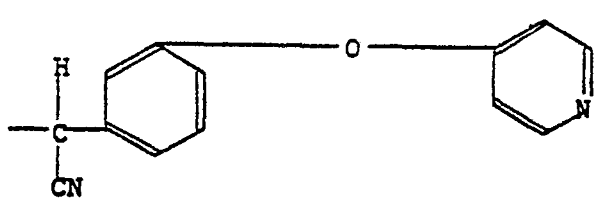

group,
or a

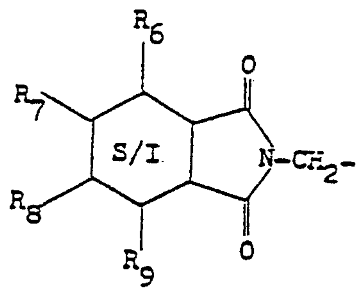

group, in which the substituents $R_6$, $R_7$, $R_8$, $R_9$ and S/I are as defined in Claim 1,
or a

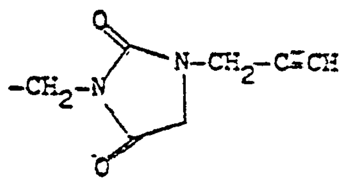

group and R is as defined in Claim 1, as well as the mixtures of these isomers.

3. The compounds with the formula (I') as defined in any of Claims 1 or 2, for which R represents a methyl radical.

4. The compounds with the formula (I') as defined in any of Claims 1 or 2, for which R represents an ethyl, n-propyl, isopropyl, tert-butyl or cyclopropylmethyl radical.

5. The compounds with the formula (I') as defined in any of Claims 1-4, for which A' is chosen from the group constituted by the (4S) 3-methyl-2-(2-propenyl)-1-oxo-cyclopent-2-en 4-yl group, the (1,3,4,5,6,7 hexahydro-1,3-dioxo-2H-isoindol-2-yl)methyl group, the (RS) cyano-(6-phenoxy-2-pyridinyl)methyl group, the [5-(phenylmethyl)-3-furanyl]methyl group, the 1-(3-propargyl-2,5-dioxo imidazolidinyl)methyl group and the (R) 3-phenoxyphenyl ethyl group.

6. Any one of the compounds with the formula (I') the names of which follow :

— (1R,cis) 2,2-dimethyl-3[(Z) 3-methoxy-3-oxo-1-propenyl] cyclopropane carboxylate of (1S) 2-methyl-4-oxo-3-(2-propenyl)-2-cyclopenten-1-yl ;

— (1R,cis) 2,2-dimethyl-3[(Z) 3-tertbutoxy-3-oxo-1-propenyl]cyclopropane carboxylate of 1-(3-propargyl-2.5-dioxo imidazolidinyl)methyl ;

— (1R,cis) 2,2-dimethyl-3[(Z) 2-cyclopropylmethoxy-carbonylethenyl]cyclopropane carboxylate of 1-(3-propargyl-2,5-dioxo imidazolidinyl)methyl ;

— (1R,cis) 2,2-dimethyl-3[(Z) 2-isopropoxycarbonylethenyl] cyclopropane carboxylate of 1(R) 3-phenoxyphenylethyl ;

— (1R,cis) 2,2-dimethyl-3[(Z) 2-isopropoxycarbonylethenyl] cyclopropane carboxylate of 1-(3-propargyl-2,5-dioxo imidazolidinyl)methyl.

7. Preparation process for the compounds with the formula (I') as defined in any one of Claims 1-6, characterized in that an acid with the formula (II) :

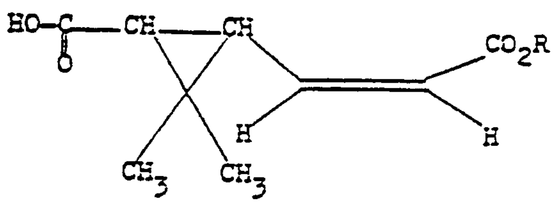

(II)

in which the double bond has Z geometry and the cyclopropane copula is of 1R,cis structure, R being defined as in Claim 1, or a functional derivative of this acid is submitted to the action of an alcohol with the formula (III)

A'OH

in which A' retains the same significance as in Claim 1 and in this way the corresponding compound with the formula (I') is obtained.

8. Preparation process for the compounds with the formula (I'), as defined in any one of Claims 1-6, characterized in that a compound with the formula (VIII) :

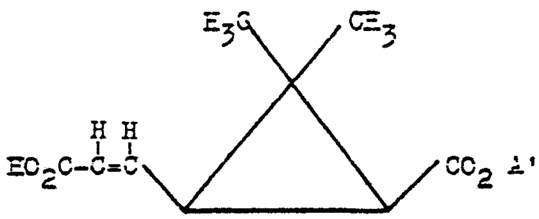

(VIII)

in which the cyclopropane copula is of 1R,cis structure and A' is defined as in Claim 1, is submitted to the action of an esterification agent so as to obtain the corresponding compound with the formula (I').

9. Process according to Claim 8, characterized in that the compound with the formula (VIII) is prepared by submitting a compound with the formula (IX) :

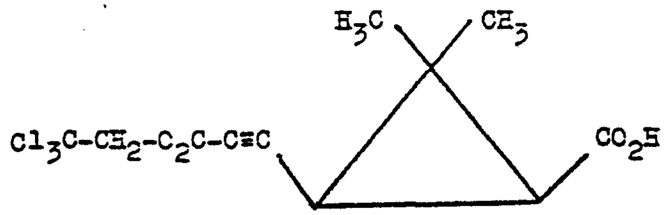

(IX)

to the action of an alcohol with the formula (III) :

A'OH (III)

in which A' is defined as in Claim 1, so as to obtain a compound with the formula (X) :

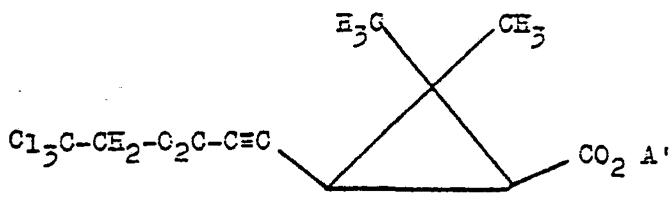

(X)

which is submitted to the action of a cleaving agent of the ester function carried by the acetylene carbon, so as to obtain the compound with the formula (XI) :

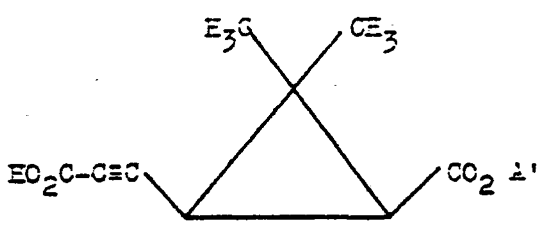

(XI)

which is submitted to the action of a carefully applied hydrogenation agent, so as to obtain the compound with the formula (VIII).

10. Process according to claim 8 or 9, characterized in that a compound with the formula (XI) :

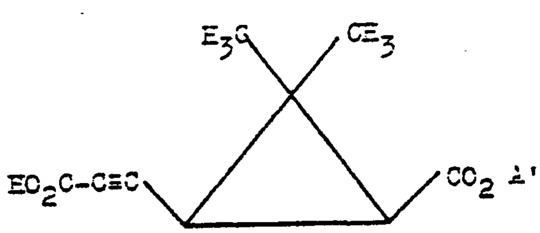

(XI)

in which A' is defined as in Claim 1, is submitted to the action of an esterification agent, so as to obtain the compound with the formula (XII) :

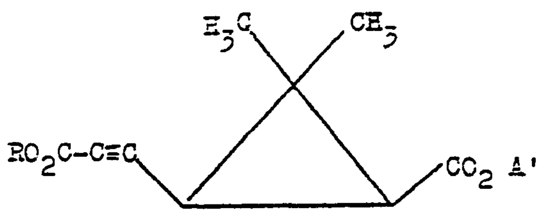

(XII)

in which R and A' are defined as in Claim 1, which is submitted to the action of a carefully applied hydrogenation agent, so as to obtain the compound with the formula (I').

11. As new industrial products and notably as intermediate products, the products with the formulae : (VIII), (X), (XI) and (XII), as defined in Claim 9 or 10.

12. Use of the compounds with the formula (I') as defined in any one of Claims 1-6, in combatting parasites of vegetation, parasites of premises and parasites of warm-blooded animals.

13. The compositions intended for combatting parasites of vegetation, parasites of premises and parasites of warm-blooded animals, characterized in that they contain at least one of the products defined in any one of Claims 1-6.

14. The insecticidal compositions containing as active principle at least one of the products defined in any one of Claims 1-5.

15. The insecticidal compositions containing as active principle at least one of the products defined in Claim 6.

16. The compositions intended for animal food containing as active principle at least one of the products defined in any one of Claims 1-6.

17. Combinations endowed with insecticidal, acaricidal or nematocidal activity, characterized in that they contain as active material, for the one part at least one of the compounds with the general formula (I'), and for the other part, at least one of the pyrethrinoid esters chosen from the group constituted by the esters of allethrolones, of 3,4,5-6-tetrahydrophthalimidomethyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol, with 2,2-dimethyl-3-(2-oxo-3-tetrahydrothiophenylidenemethyl)-cyclopropane-1-carboxylic acids, by the esters of 3-phenoxybenzyl alcohol, and of α-cyano-3-phenoxybenzyl alcohols, with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with 2,2-dimethyl-3(1,2,2,2-tetrahalo)cyclopropane-1-carboxylic acids, in which « halo » represents a fluorine, chlorine or bromine atom, it being understood that the compounds with the formula (I') may exist in all their possible stereoisomeric forms, likewise the acid and alcohol copulae of the above pyrethrinoid esters.

**Claims** (for the contracting State AT)

1. Preparation process for all the possible isomer forms, of compounds with the formula (I') :

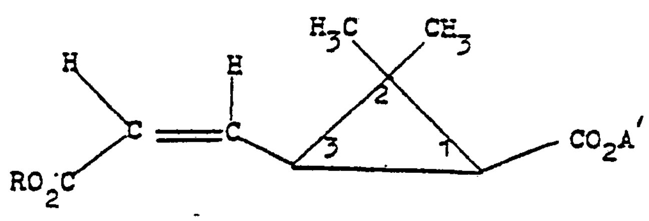

(I')

in which the double bond has Z geometry, A' represents :

either an alkyl radical containing 1-18 carbon atoms, or a benzyl radical possibly substituted by one or more radicals chosen from the group constituted by the alkyl radicals containing 1-4 carbon atoms, the alkenyl radicals containing 2-6 carbon atoms, the alkenyloxy radicals containing 2-6 carbon atoms, the

alkadienyl radicals containing 4-8 carbon atoms, the methylene dioxy residue, and the halogen atoms, or a

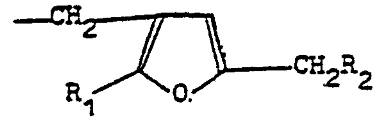

group, in which the substituent $R_1$ represents a hydrogen atom or a methyl radical and the substituent $R_2$ represents a monocyclic aryl or a $—CH_2—C{\equiv}CH$ group and notably a 5-benzyl-3-furylmethyl group, or a

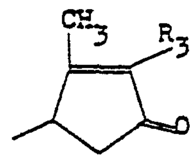

group, in which $R_3$ represents an aliphatic organic radical containing 2-6 carbon atoms and or more carbon-carbon unsaturations and notably the $—CH_2—CH{=}CH_2$, $—CH_2CH{=}CH—CH_3$, $—CH_2CH{=}CH—C_2H_5$, $—CH_2—CH{=}CH—CH{=}CH_2$ radicals, or a

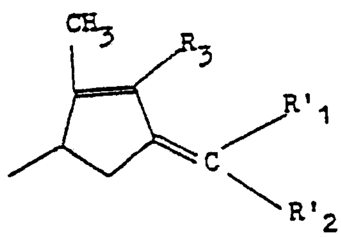

group, in which $R_3$ retains the same significance as previously, $R_1'$ and $R_2'$ identical or different, each represent a hydrogen atom, a halogen atom, an alkyl radical containing 1-6 carbons atoms, an aryl radical containing 6-10 carbon atoms, an alkyloxycarbonyl group containing 2-5 carbon atoms, or a cyano group, or a

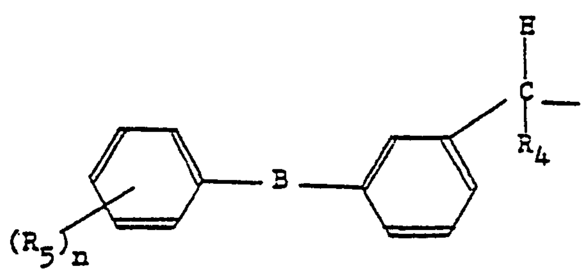

group, in which B represents a $CH_2$ or C=O group, or a hetero element chosen from oxygen and sulphur, $R_4$ represents a hydrogen atom, a methyl radical, a $—CONH_2$ radical, a $—CSNH_2$ radical or a $—C{\equiv}CH$ radical, $R_5$ represents a halogen atom or a methyl radical and n represents a numerical 0, 1 or 2, and notably the 3-phenoxybenzyl, α-ethynyl-3-phenoxybenzyl, 3-benzoylbenzyl, 1-(3-phenoxyphenyl)ethyl or α-thiomado-3-phenoxybenzyl group, or a

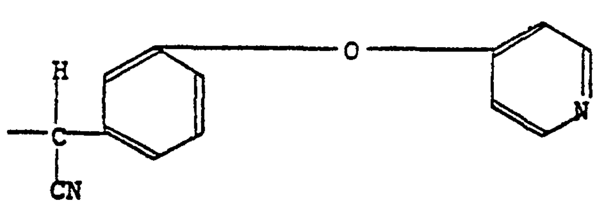

group

or a

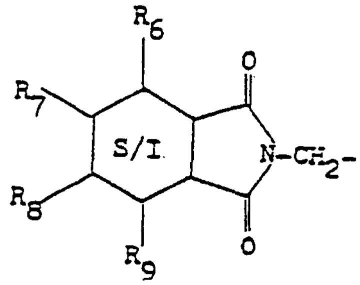

group, in which each of the substituents $R_6$, $R_7$, $R_8$, $R_9$, represents a hydrogen atom, a chlorine atom or a methyl radical and in which S/I signifies an aromatic ring or a similar dihydro or tetrahydro ring,

or a

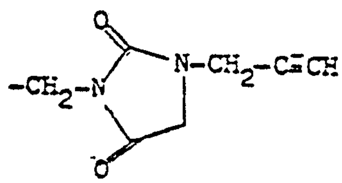

group,

or a

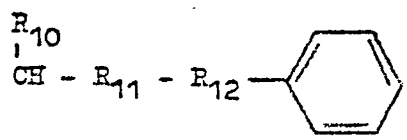

group in which $R_{10}$ represents a hydrogen atom or a CN radical, $R_{12}$ represents a $-CH_2-$ radical or an oxygen atom, $R_{11}$ represents a thiazolyl or thiadiazolyl radical of which the bond with

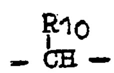

may be found in any of the available positions, $R_{12}$ being linked to $R_{11}$ by the carbon atom situated between the sulphur atom and a nitrogen atom,

or a

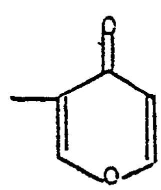

group,

or a

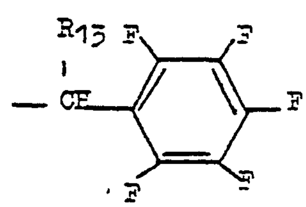

group, in which $R_{13}$ represents a hydrogen atom or a CN radical,
or a

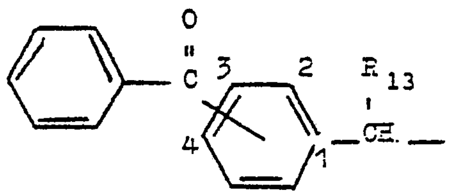

group, in which $R_{13}$ is defined as above and the benzoyl radical is in position 3 or 4,
or a

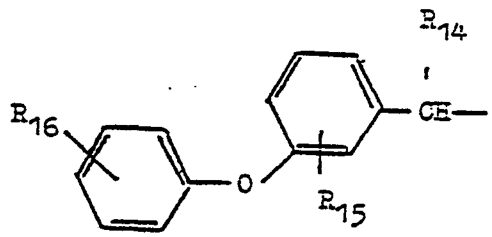

group, in which $R_{14}$ represents a hydrogen atom, a methyl, ethynyl or cyano radical, and each of $R_{15}$ and $R_{16}$, which are different, represents a hydrogen, fluorine or bromine atom,
or a

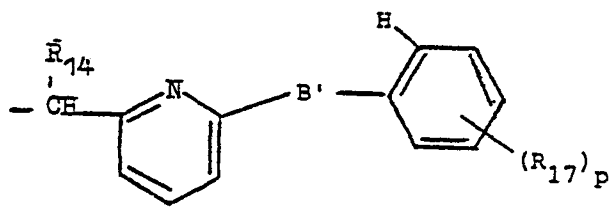

group, in which $R_{14}$ is defined as above, each of the $R_{17}$'s represents, independently, an alkyl group containing 1-4 carbon atoms, an alkoxy group containing 1-4 carbon atoms, an alkylthio group containing 1-4 carbon atoms, an alkylsulphonyl group containing 1-4 carbon atoms, or a trifluoromethyl, 3,4-methylenedioxy, chloro, fluoro or bromo group, p represents a numeral 0, 1 or 2 and B' represents an oxygen atom or a sulphur atom, and R represents an alkyl radical, linear branched or cyclic, saturated or unsaturated, containing 1-18 carbon atoms, as well as the mixtures of these isomers, characterized in that an acid with the formula (II) :

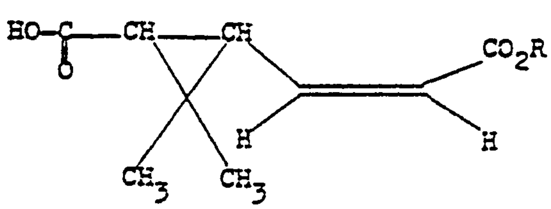

(II)

in which the double bond has Z geometry, R being defined as previously, or a functional derivative of this acid, is submitted to the action of an alcohol with the formula (III) :

A'OH (III)

in which A' retains the same significance as previously, and in this way the corresponding compound with the formula (I') is obtained.

2. Process according to Claim 1, for preparation in all the possible isomeric forms of the compounds

with the formula (I') as defined in Claim 1, corresponding to the formula (I) :

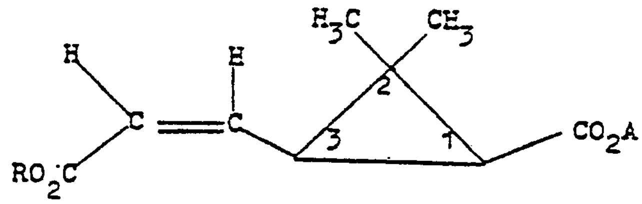

(I)

in which the double bond has Z geometry, A represents :

either an alkyl radical containing 1-18 carbon atoms,

or a benzyl radical possibly substituted as defined in Claim 1,

or a

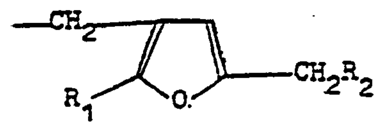

group, in which the substituents $R_1$ and $R_2$ are as defined in Claim 1,

or a

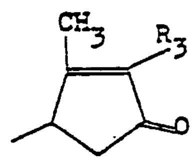

group, in which $R_3$ is defined as in Claim 1,

or a

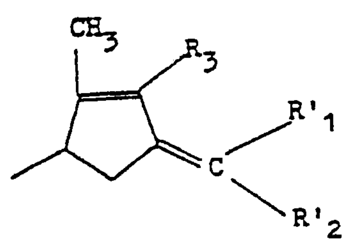

group, in which $R_3$, $R_1'$ and $R_2'$ are as defined in Claim 1,

or a

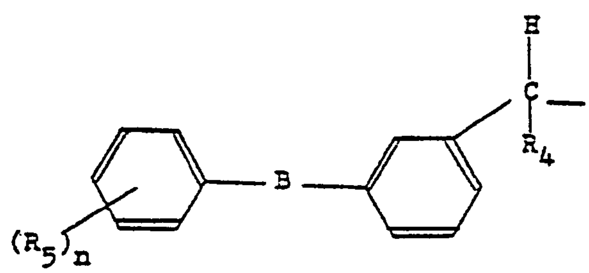

group, in which B, $R_4$, $R_5$ and n are as defined in Claim 1,

or a

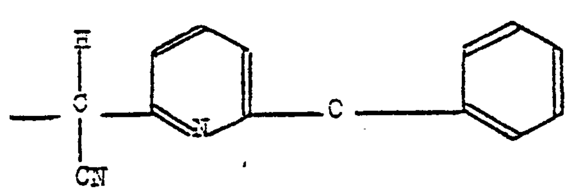

group,
or a

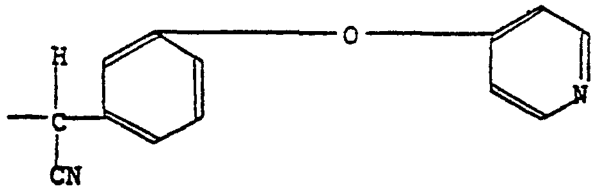

group,
or a

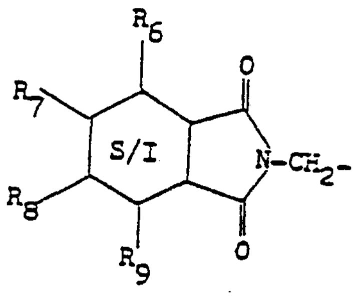

group, in which the substituents $R_6$, $R_7$, $R_8$, $R_9$ and S/I are as defined in Claim 1,
or a

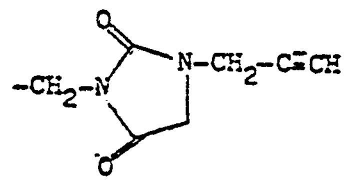

group, and R is defined as in Claim 1, as well as the mixtures of these isomers, characterized in that an alcohol with the formula (III) is used at the start, in which A' has the values of A indicated previously.

3. Preparation process for the compounds with the formula (I'), as defined in Claim 1, characterized in that a compound with the formula (VIII)

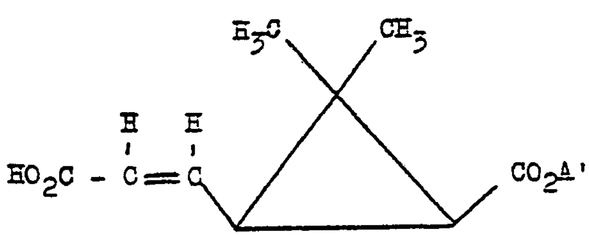

(VIII)

in which A' is defined as in Claim 1, is submitted to the action of an esterification agent, so as to obtain the corresponding compound with the formula (I').

4. Process according to Claim 3, characterized in that the compound with the formula (VIII) is prepared by submitting a compound with the formula (IX) :

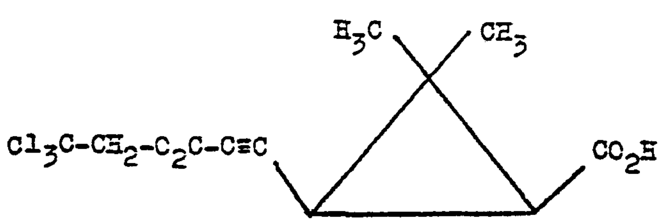

(IX)

to the action of an alcohol with the formula (III) :

$$A'—OH \quad (III)$$

in which A' is defined as in Claim 1, so as to obtain the compound with the formula (X) :

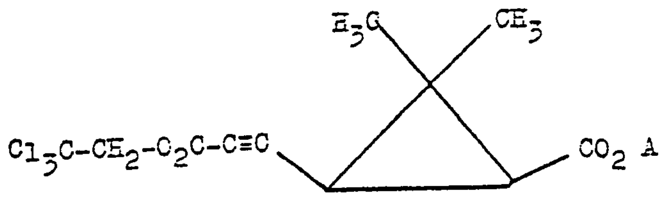

(X)

which is submitted to the action of a cleaving agent of the ester function carried by the acetylene carbon, so as to obtain the compound with the formula (XI) :

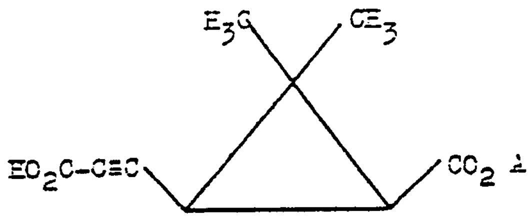

(XI)

which is submitted to the action of a carefully applied hydrogenation agent, so as to obtain the compound with the formula (VIII).

5. Process according to Claim 3 or 4, characterized in that a compound with the formula (XI) :

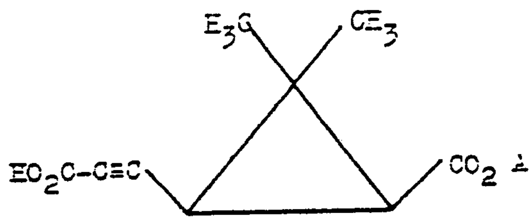

(XI)

in which A' is defined as in Claim 1, is submitted to the action of an esterification agent, so as to obtain the compound with the formula (XII) :

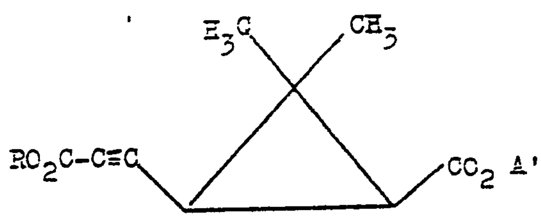

(XII)

in which R and A' are defined as in Claim 1, which is submitted to the action of a carefully applied hydrogenation agent, so as to obtain the compound with the formula (I').

6. Process according to any one of Claims 1, 3, 4 and 5, characterized in that a compound with the formula (II), (VIII), (IX) or (XI) is used at the start, the structure of which is 1R,cis or 1R,trans.

7. Process according to any one of Claims 1, 3, 4 and 5, characterized in that a compound with the formula (II) or an esterification agent is used at the start, in which R represents a methyl, ethyl, n-propyl, isopropyl, tertbutyl or cyclopropylmethyl radical.

8. Process according to any one of Claims 1, 3, 4 and 5, characterized in that a compound with the formula (III), (VIII) or (XI) is used at the start, in which A' is chosen from the group constituted by the (4S) 3-methyl-2-(2-propenyl)-1-oxo-cyclopent-2-en-4-yl, (1,3,4,5,6,7-hexahydro-1,3-dioxo-2H-isoindol-2-yl)methyl, (RS) cyano-(6-phenoxy-2-pyridinyl)methyl, [5-(phenylmethyl)-3-furanyl]methyl, 1-(3-propargyl-2,5-dioxo imidazolidinyl)methyl and (R) 3-phenoxyphenylethyl groups.

9. Process according to Claim 2, characterized in that a compound with the formula (II) of 1R,cis or 1R,trans structure, is used at the start, in which R represents a methyl, ethyl, n-propyl, isopropyl, tertbutyl or cyclopropylmethyl radical and an alcohol with the formula (III) in which A' is chosen from the group constituted by the (4S) 3-methyl-2-(2-propenyl)-1-oxo-cyclopent-2-en-4-yl, (1,3,4,5,6,7-hexahydro-1,3-dioxo-2H-isoindol-2-yl)methyl, (RS) cyano-(6-phenoxy-2-pyridinyl)methyl, [5-(phenylmethyl)-3-furanyl]methyl, 1-(3-propargyl-2,5-dioxo imidazolidinyl)methyl and (R) 3-phenoxyphenylethyl groups.

10. Process according to any one of Claims 1-5, characterized in that any one of the compounds with the formula (I'), the names of which follow, is prepared :

— (1R,cis) 2,2-dimethyl-3-[(Z)3-methoxy-3-oxo-1-propenyl]-cyclopropane carboxylate of (1S) 2-methyl-4-oxo-3-(2-propenyl)-2-cyclopenten-1-yl ;

— (1R,cis 2,2-dimethyl-3-[(Z)3-tertbutoxy-3-oxo-1-propenyl]-cyclopropane carboxylate of 1-(3-propargyl-2,5-dioxo-imidazolidinyl)methyl ;

— (1R,cis 2,2-dimethyl-3-[(Z)2-cyclopropylmethoxycarbonylethenyl]-cyclopropane carboxylate of 1-(3-propargyl-2,5-dioxo-imidazolidinyl)methyl ;

— (1R,cis) 2,2-dimethyl-3-[(Z)2-isopropoxycarbonylethenyl]-cyclopropane carboxylate of 1(R)-3-phenoxyphenylethyl ;

— (1R,cis) 2,2-dimethyl-3-[(Z)2-isopropoxycarbonylethenyl]-cyclopropane carboxylate of 1-(3-propargyl-2,5-dioxoimidazolidinyl)methyl ;

— (1R,trans) 2,2-dimethyl-3-[(Z)2-isopropoxycarbonylethenyl]-cyclopropane carboxylate of 1(R) 3-phenoxyphenylethyl.

11. Use of the compounds with the formula (I') as defined in Claim 1, in combatting parasites of vegetation, parasites of premises and parasites of warm-blooded animals.

12. Use of the compounds with the formula (I') as defined in Claim 2, in combatting parasites of vegetation, parasites of premises and parasites of warm-blooded animals.

13. The compositions intended for combatting parasites of vegetation, parasites of premises and parasites of warm-blooded animals, characterized in that they contain at least one of the products defined in Claim 1.

14. The insecticidal compositions containing as active principle at least one of the products defined in Claim 1.

15. The insecticidal compositions containing as active principle at least one of the products defined in Claim 10.

16. The compositions intended for animal feeding, containing as active principle at least one of the products defined in Claim 1.

17. Compositions endowed with insecticidal, acaricidal or nematocidal activity, characterized in that they contain as active material, for the one part at least one of the compounds with the general formula (I'), and for the other part, at least one of the pyrethrinoid esters chosen from the group constituted by esters of allethrolones, of 3,4,5-6-tetrahydrophthalimidomethyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol, with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with 2,2-dimethyl-3-(1,2,2,2-tetrahalo)cyclopropane-1-carboxylic acids, in which « halo » represents a fluorine, chlorine or bromine atom, it being understood that the compounds with the formula (I') may exist in all their possible stereoisomeric forms, and likewise the acid and alcohol copulae of the above pyrethrinoid esters.

18. Compositions according to any one of Claims 13-17, characterized in that the compounds with the formula (I') correspond to the formula (I), as defined in Claim 2.

**Ansprüche** (für die Vertragsstaaten : BE, CH, DE, FR, GB, IT, LI, LU, NL, SE)

1. In ihren sämtlichen möglichen isomeren Formen die Verbindungen der Formel (I')

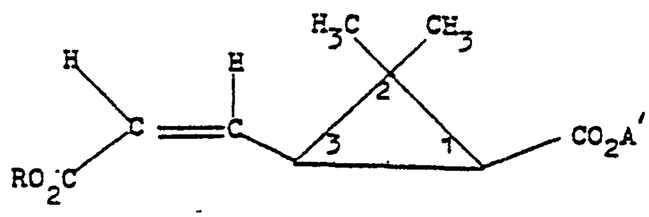

(I')

worin die Doppelbindung die Z-Geometrie aufweist, die Cyclopropan-Verknüpfung 1R-cis-Struktur besitzt und A' bedeutet

entweder einen Alkylrest mit 1 bis 18 Kohlenstoffatomen,

oder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,

oder eine Gruppe

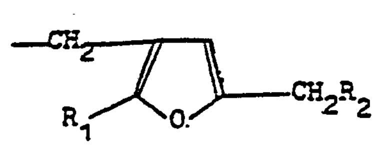

worin der Substituent $R_1$ ein Wasserstoffatom oder einen Methylrest bedeutet und der Substituent $R_2$ einen monocyclischen Arylrest oder eine Gruppe $—CH_2—C{\equiv}CH$ bedeutet, und insbesondere eine 5-Benzyl-3-furylmethylgruppe,

oder eine Gruppe

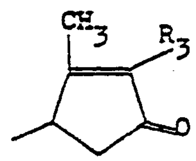

worin $R_3$ einen aliphatischen, organischen Rest mit 2 bis 6 Kohlenstoffatomen und einer oder mehreren Kohlenstoff-Kohlenstoff-Unsättigungen und insbesondere die Reste $—CH_2—CH{=}CH_2$, $—CH_2—CH{=}CH—CH_3$, $—CH_2—CH{=}CH—C_2H_5$, $—CH_2—CH{=}CH—CH{=}CH_2$ bedeutet,

oder eine Gruppe

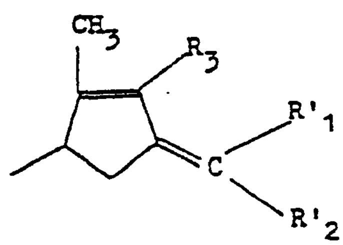

worin $R_3$ die vorstehend angegebene Bedeutung besitzt, $R_1'$ und $R_2'$, die gleich oder verschieden sein können, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

oder eine Gruppe

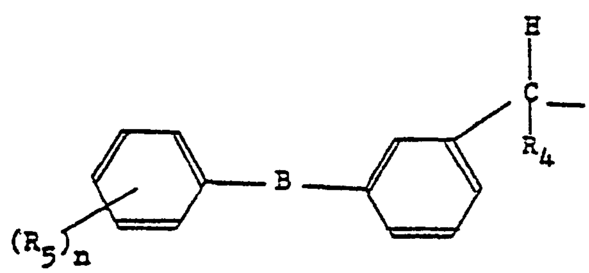

worin B eine Gruppe $CH_2$ oder C=O oder ein Heteroelement, ausgewählt unter Sauerstoff und Schwefel, bedeutet, $R_4$ ein Wasserstoffatom, einen Methylrest, einen $—CONH_2$-Rest, einen $—CSNH_2$-Rest oder einen Rest $—C{\equiv}CH$ bedeutet, $R_5$ ein Halogenatom oder einen Methylrest bedeutet und n eine ganze Zahl entsprechend 0, 1 oder 2 bedeutet, und insbesondere die 3-Phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzyl-Gruppe,

oder eine Gruppe

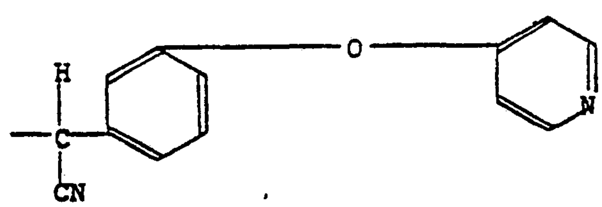

oder eine Gruppe

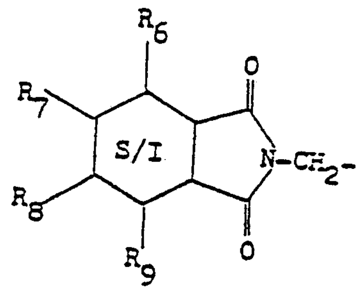

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydroring symbolisiert,

oder eine Gruppe

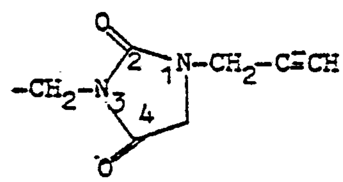

oder eine Gruppe

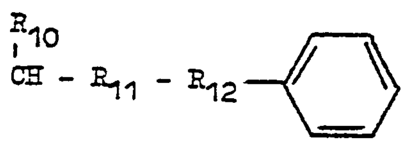

worin $R_{10}$ ein Wasserstoffatom oder einen CN-Rest bedeutet, $R_{12}$ einen Rest $-CH_2-$ oder ein Sauerstoffatom bedeutet, $R_{11}$ einen Thiazolyl- oder Thiadiazolylrest bedeutet, deren Bindung mit

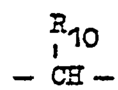

sich in irgendeiner der verfügbaren Positionen befinden kann, wobei $R_{12}$ an $R_{11}$ durch das zwischen dem Schwefel- und dem Stickstoffatom befindliche Kohlenstoffatom gebunden ist,

oder eine Gruppe

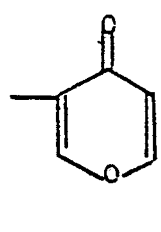

oder eine Gruppe

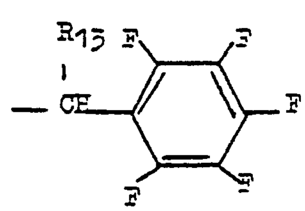

worin $R_{13}$ ein Wasserstoffatom oder einen CN-Rest bedeutet,

oder eine Gruppe

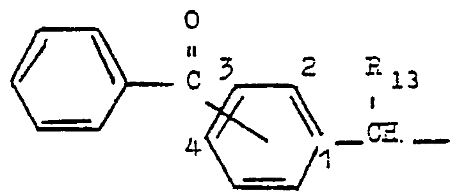

worin $R_{13}$ wie vorstehend definiert ist und der Benzoylrest sich in 3- oder 4-Stellung befindet, oder eine Gruppe

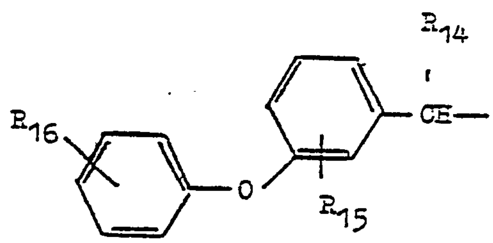

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten, oder eine Gruppe

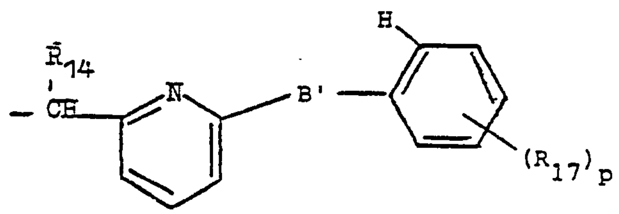

worin $R_{14}$ wie vorstehend definiert ist, ein jeder der Reste $R_{17}$ unabhängig einen Alkylrest mit 1 bis 4 Kohlenstoffatomen, Alkoxyrest mit 1 bis 4 Kohlenstoffatomen, Alkylthiorest mit 1 bis 4 Kohlenstoffatomen, Alkylsulfonylrest mit 1 bis 4 Kohlenstoffatomen, Trifluormethylrest, 3,4-Methylendioxyrest, oder ein Chlor-, Fluor- oder Bromatom bedeutet, p eine Zahl entsprechend 0, 1 oder 2 bedeutet und B' ein Sauerstoff- oder Schwefelatom bedeutet,

und R einen gesättigten oder ungesättigten, linearen, verzweigten oder cyclischen Alkylrest mit 1 bis 18 Kohlenstoffatomen bedeutet,

sowie die Gemische dieser Isomeren.

2. In ihren sämtlichen möglichen isomeren Formen die Verbindungen der Formel (I') gemäß Anspruch 1 der Formel (I)

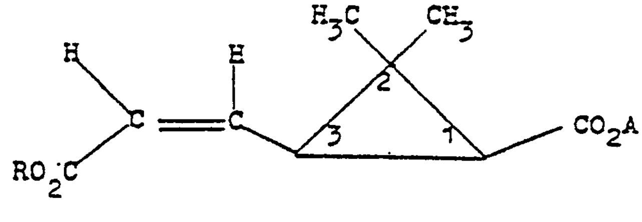

(I)

worin die Doppelbindung die Z-Geometrie aufweist und die Cyclopropan-Verknüpfung 1R-cis-Struktur besitzt, A bedeutet

entweder einen Alkylrest mit 1 bis 18 Kohlenstoffatomen,

oder einen Benzylrest, der gegebenenfalls, wie in Anspruch 1 definiert, substituiert ist,

oder eine Gruppe

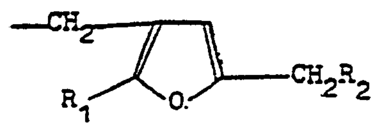

worin die Substituenten $R_1$ und $R_2$ wie in Anspruch 1 definiert sind,

oder eine Gruppe

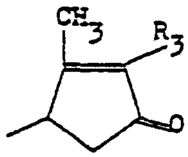

worin $R_3$ wie in Anspruch 1 definiert ist,
oder eine Gruppe

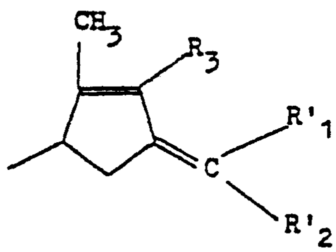

worin $R_3$, $R_1'$ und $R_2'$ wie in Anspruch 1 definiert sind,
oder eine Gruppe

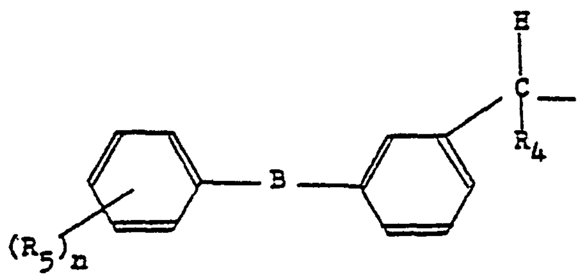

worin B, $R_4$, $R_5$ und n wie in Anspruch 1 definiert sind,
oder eine Gruppe

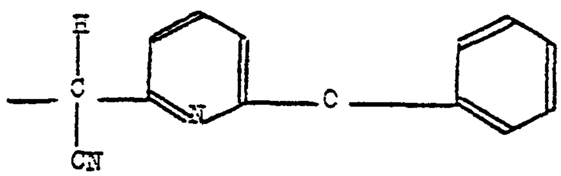

oder eine Gruppe

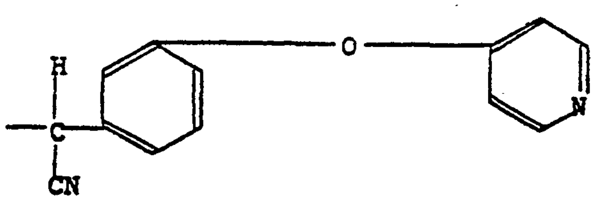

oder eine Gruppe

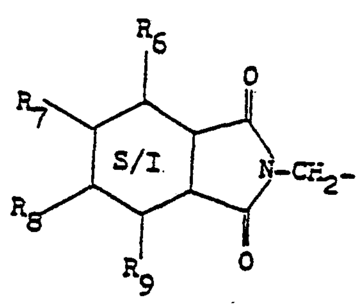

worin die Substituenten $R_6$, $R_7$, $R_8$, $R_9$ und S/I wie in Anspruch 1 definiert sind, oder eine Gruppe

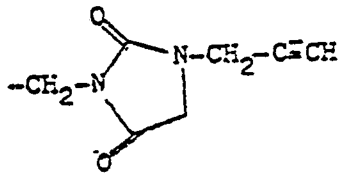

und R wie in Anspruch 1 definiert ist ; sowie die Gemische dieser Isomeren.

3. Die Verbindungen der Formel (I') gemäß einem der Ansprüche 1 oder 2, worin R einen Methylrest bedeutet.

4. Die Verbindungen der Formel (I') gemäß einem der Ansprüche 1 oder 2, worin R einen Ethyl-, n-Propyl-, Isopropyl-, tert.-Butyl- oder Cyclopropylmethylrest bedeutet.

5. Die Verbindungen der Formel (I') gemäß einem der Ansprüche 1 bis 4, worin A' ausgewählt ist unter der (4S)-3-Methyl-2-(2-propenyl)-1-oxo-cyclopent-2-en-4-yl-Gruppe, der (1,3,4,5,6,7-Hexahydro-1,3-dioxo-2H-isoindol-2-yl)-methyl-Gruppe, der (RS)-Cyano-(6-phenoxy-2-pyridinyl)-methyl-Gruppe, der [5-(Phenylmethyl)-3-furanyl]-methyl-Gruppe, der 1-(3-Propargyl-2,5-dioxoimidazolidinyl)-methyl-Gruppe und der (R)-3-Phenoxyphenylethyl-Gruppe.

6. Eine der Verbindungen der Formel (I') mit den folgenden Bezeichnungen :

— (1S)-2-Methyl-4-oxo-3-(2-propenyl)-2-cyclopenten-1-yl-(1R-cis)-2,2-dimethyl-3-[(Z)-3-methoxy-3-oxo-1-propenyl]-cyclopropan-carboxylat ;

1-(3-Propargyl-2,5-dioxo-imidazolidinyl)-methyl-(1R-cis)-2,2-dimethyl-3-[(Z)-3-tert.-butoxy-3-oxo-1-propenyl]-cyclopropan-carboxylat ;

1-(3-Propargyl-2,5-dioxoimidazolidinyl)-methyl-(1R-cis)-2,2-dimethyl-3-[(Z)-2-cyclopropylmethoxy-carbonylethenyl]-cyclopropan-carboxylat ;

1(R)-3-Phenoxyphenylethyl-(1R-cis)-2,2-dimethyl-3-[(Z)-2-isopropoxycarbonylethenyl]-cyclopropan-carboxylat ;

1-(3-Propargyl-2,5-dioxo-imidazolidinyl)-methyl-(1R-cis)-2,2-dimethyl-3-[(Z)-2-isopropoxycarbonyl-ethenyl]-cyclopropan-carboxylat.

7. Verfahren zur Herstellung der Verbindungen der Formel (I') gemäß einem der Ansprüche 1 bis 6, dadurch gekennzeichnet, daß man eine Säure der Formel (II)

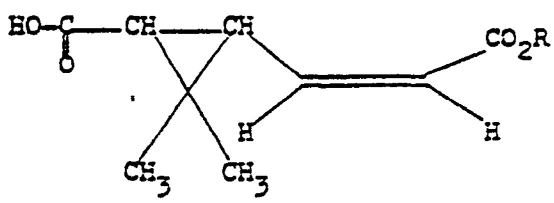

(II)

worin die Doppelbindung die Z-Geometrie besitzt und die Cyclopropanverknüpfung 1R-cis-Struktur aufweist, wobei R wie in Anspruch 1 definiert ist, oder ein funktionelles Derivat dieser Säure mit einem Alkohol der Formel (III)

A'OH (III)

worin A' die in Anspruch 1 angegebene Bedeutung besitzt, umsetzt und so die entsprechende Verbindung der Formel (I') erhält.

8. Verfahren zur Herstellung der Verbindungen der Formel (I') gemäß einem der Ansprüche 1 bis 6, dadurch gekennzeichnet, daß man eine Verbindung der Formel (VIII)

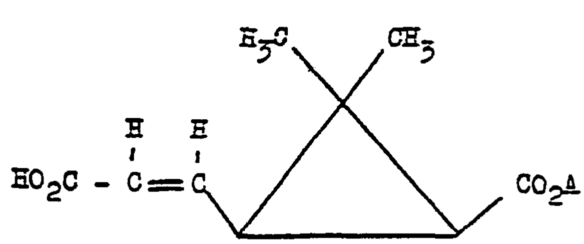

(VIII)

worin die Cyclopropanverknüpfung 1R-cis-Struktur besitzt und A' wie in Anspruch 1 definiert ist, der Einwirkung eines Veresterungsmittels unterzieht, um die entsprechende Verbindung der Formel (I') zu erhalten.

9. Verfahren gemäß Anspruch 8, dadurch gekennzeichnet, daß die Verbindung der Formel (VIII) hergestellt wird, indem man eine Verbindung der Formel (IX)

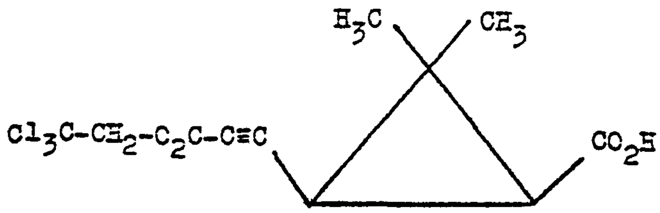

(IX)

der Einwirkung eines Alkohols der Formel (III)

A'—OH (III)

worin A' wie in Anspruch 1 definiert ist, unterzieht, um die Verbindung der Formel (X)

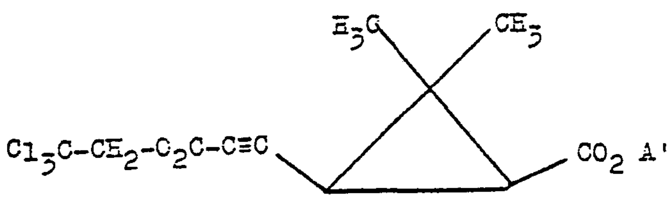

(X)

zu erhalten, die man der Einwirkung eines Mittels zur Spaltung der von dem acetylenischen Kohlenstoffatom getragenen Esterfunktion unterzieht, um die Verbindung der Formel (XI)

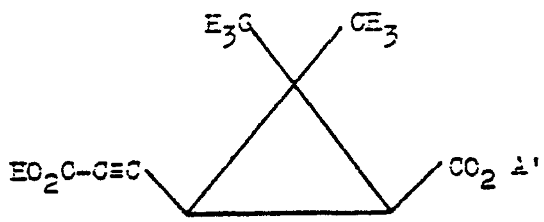

(XI)

zu erhalten, die man der Einwirkung eines milden Hydrierungsmittels unterzieht, um die Verbindung der Formel (VIII) zu erhalten.

10. Verfahren gemäß Anspruch 8 oder 9, dadurch gekennzeichnet, daß man eine Verbindung der Formel (XI)

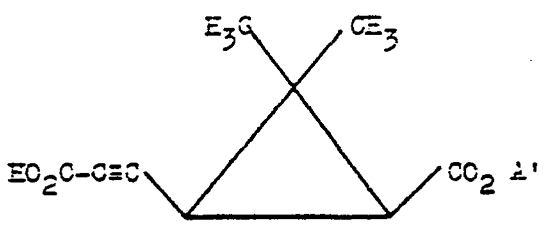

(XI)

worin A' wie in Anspruch 1 definiert ist, der Einwirkung eines Veresterungsmittels unterzieht, um die Verbindung der Formel (XII)

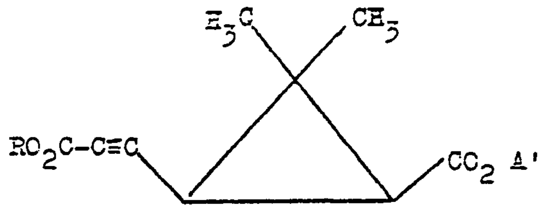

(XII)

zu erhalten, worin R und A' wie in Anspruch 1 definiert sind, die man der Einwirkung eines milden Hydrierungsmittels unterzieht, um die Verbindung der Formel (I') zu erhalten.

11. Als neue, industrielle Produkte und insbesondere als Zwischenprodukte die Produkte der Formeln (VIII), (X), (XI) und (XII) gemäß Anspruch 9 oder 10.

12. Verwendung der Verbindungen der Formel (I') gemäß einem der Ansprüche 1 bis 6 zur Bekämpfung von Parasiten der Pflanzen, Parasiten im häuslichen Bereich und Parasiten warmblütiger Tiere.

13. Zusammensetzungen für die Bekämpfung von Parasiten der Pflanzen, Parasiten im häuslichen Bereich und Parasiten warmblütiger Tiere, dadurch gekennzeichnet, daß sie zumindest eines der Produkte gemäß einem der Ansprüche 1 bis 6 enthalten.

14. Insektizide Zusammensetzungen, enthaltend als Wirkstoff zumindest eines der in einem der Ansprüche 1 bis 5 definierten Produkte.

15. Insektizide Zusammensetzungen, enthaltend als Wirkstoff zumindest eines der in Anspruch 6 definierten Produkte.

16. Zusammensetzungen für die tierische Ernährung, enthaltend als Wirkstoff zumindest eines der Produkte gemäß einem der Ansprüche 1 bis 6.

17. Assoziationen mit insektizider, akarizider oder nematizider Aktivität, dadurch gekennzeichnet, daß sie als Wirkstoff einesteils zumindest eine der Verbindungen der allgemeinen Formel (I') und anderenteils zumindest einen Pyrethrinoidester enthalten, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahalo)-cyclopropan-1-carbonsäuren, worin « Halo » ein Fluor, Chlor- oder Bromatom bedeutet, wobei sich versteht, daß die Verbindungen (I') in sämtlichen ihrer möglichen stereoisomeren Formen ebenso wie die Säure- und Alkohol-Verknüpfungen der vorstehenden Pyrethrinoidester vorliegen können.

**Ansprüche** (für den Vertragsstaat AT)

1. Verfahren zur Herstellung der Verbindungen der Formel (I') in sämtlichen ihrer möglichen isomeren Formen

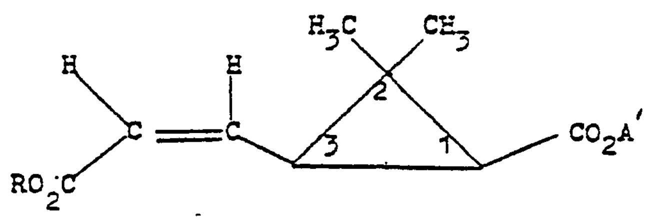

(I')

worin die Doppelbindung die Z-Geometrie besitzt, A' bedeutet :

entweder einen Alkylrest mit 1 bis 18 Kohlenstoffatomen,

oder einen Benzylrest, gegebenenfalls substituiert durch einen oder mehrere Reste, ausgewählt unter den Alkylresten mit 1 bis 4 Kohlenstoffatomen, den Alkinylresten mit 2 bis 6 Kohlenstoffatomen, den

Alkenyloxyresten mit 2 bis 6 Kohlenstoffatomen, den Alkadienylresten mit 4 bis 8 Kohlenstoffatomen, dem Methylendioxyrest und den Halogenatomen,

oder eine Gruppe

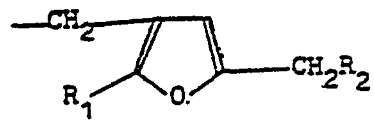

worin der Substituent $R_1$ ein Wasserstoffatom oder einen Methylrest bedeutet und der Substituent $R_2$ einen monocyclischen Arylrest oder eine Gruppe $-CH_2-C\equiv CH$ bedeutet und insbesondere eine 5-Benzyl-3-furylmethyl-Gruppe,

oder eine Gruppe

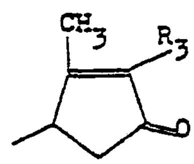

worin $R_3$ einen aliphatischen, organischen Rest mit 2 bis 6 Kohlenstoffatomen und einer oder mehreren Kohlenstoff-Kohlenstoff-Unsättigungen und insbesondere die Reste $-CH_2-CH=CH_2$, $-CH_2-CH=CH-CH_3$, $-CH_2-CH=CH-C_2H_5$, $-CH_2-CH=CH-CH=CH_2$ bedeutet,

oder eine Gruppe

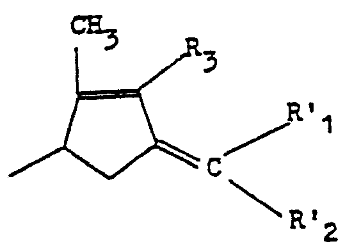

worin $R_3$ die vorstehend angegebene Bedeutung besitzt, $R'_1$ und $R'_2$ gleich oder verschieden sind und ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

oder eine Gruppe

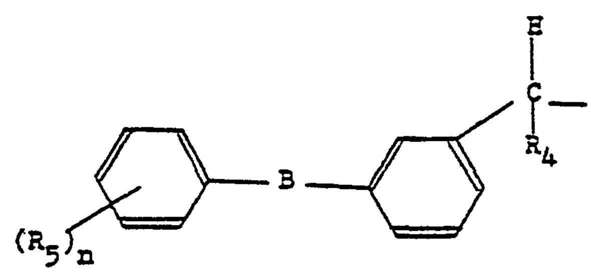

worin B eine $CH_2$- oder C=O-Gruppe oder ein Heteroelement, ausgewählt unter Sauerstoff und Schwefel, bedeutet, $R_4$ ein Wasserstoffatom, einen Methylrest, einen Rest $-CONH_2$, einen Rest $-CSNH_2$ oder einen Rest $-C\equiv CH$ bedeutet, $R_5$ ein Halogenatom oder einen Methylrest bedeutet und n eine Zahl gleich 0, 1 oder 2 bedeutet, und insbesondere die 3-Phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzyl-Gruppe,

oder eine Gruppe

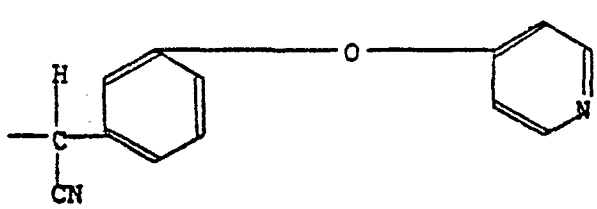

oder eine Gruppe

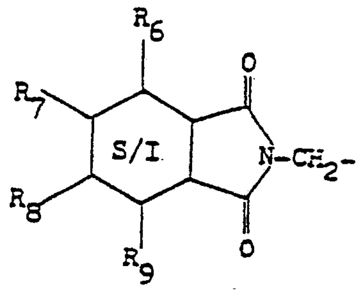

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydroring symbolisiert,

oder eine Gruppe

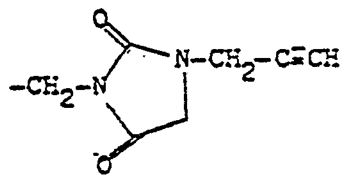

oder eine Gruppe

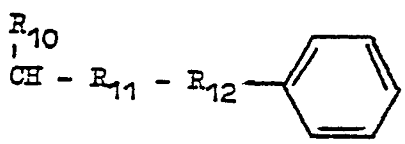

worin $R_{10}$ ein Wasserstoffatom oder einen CN-Rest bedeutet, $R_{12}$ einen Rest $—CH_2—$ oder ein Sauerstoffatom bedeutet, $R_{11}$ einen Thiazolyl- oder Thiadiazolylrest bedeutet, dessen Bindung mit

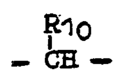

sich in irgendeiner der verfügbaren Positionen befinden kann, wobei $R_{12}$ an $R_{11}$ durch ein sich zwischen dem Schwefel- und einem Stickstoffatom befindlichen Kohlenstoffatom gebunden ist,

oder eine Gruppe

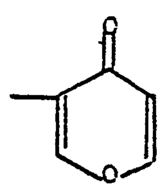

oder eine Gruppe

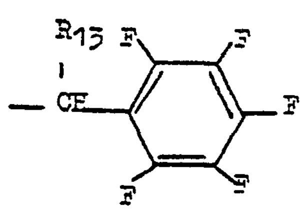

worin $R_{13}$ ein Wasserstoffatom oder einen CN-Rest bedeutet,

oder eine Gruppe

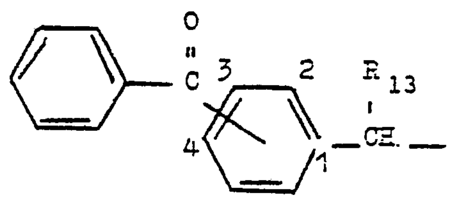

worin $R_{13}$ wie vorstehend definiert ist und der Benzoylrest sich in 3- oder 4-Stellung befindet, oder eine Gruppe

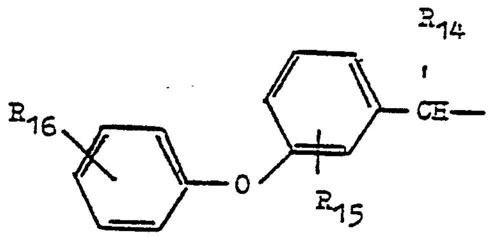

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten, oder eine Gruppe

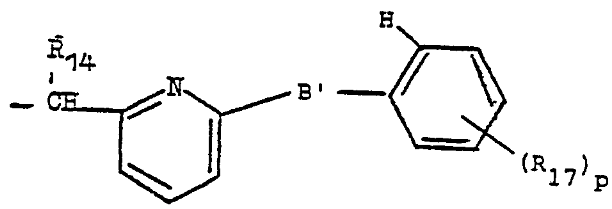

worin $R_{14}$ wie vorstehend definiert ist, ein jeder der Reste $R_{17}$ unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, eine Trifluormethylgruppe, eine 3,4-Methylendioxygruppe oder ein Chlor-, Fluor- oder Bromatom bedeutet, p eine Zahl gleich 0, 1 oder 2 ist und B' ein Sauerstoff- oder ein Schwefelatom darstellt,
und R einen gesättigten oder ungesättigten, linearen, verzweigten oder cyclischen Alkylrest mit 1 bis 18 Kohlenstoffatomen bedeutet, sowie von den Gemischen dieser Isomeren,
dadurch gekennzeichnet, daß man eine Säure der Formel (II)

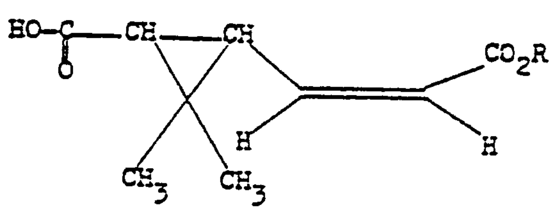

(II)

worin die Doppelbindung die Z-Geometrie aufweist und R wie vorstehend definiert ist, oder ein funktionelles Derivat dieser Säure mit einem Alkohol der Formel (III)

A'OH (III)

worin A' die vorstehend angegebene Bedeutung besitzt, umsetzt und so die entsprechende Verbindung der Formel (I') erhält.

2. Verfahren gemäß Anspruch 1 zur Herstellung der Verbindungen der Formel (I'), wie in Anspruch 1 definiert, in sämtlichen ihrer möglichen isomeren Formen, entsprechend der Formel (I)

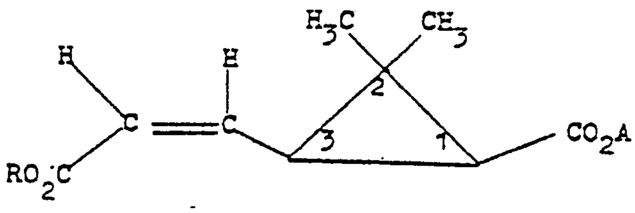

(I)

worin die Doppelbindung die Z-Geometrie besitzt, A bedeutet :
entweder einen Alkylrest mit 1 bis 18 Kohlenstoffatomen,
oder einen Benzylrest, der gegebenenfalls, wie in Anspruch 1 definiert, substituiert ist,
oder eine Gruppe

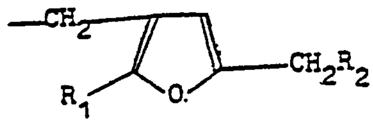

worin die Substituenten $R_1$ und $R_2$ wie in Anspruch 1 definiert sind,
oder eine Gruppe

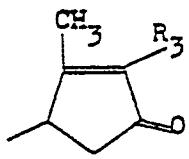

worin $R_3$ wie in Anspruch 1 definiert ist,
oder eine Gruppe

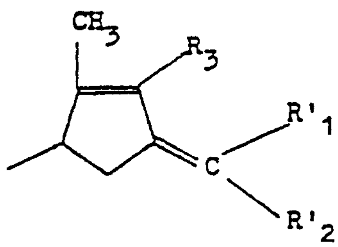

worin $R_3$, $R_1'$ und $R_2'$ wie in Anspruch 1 definiert sind,
oder eine Gruppe

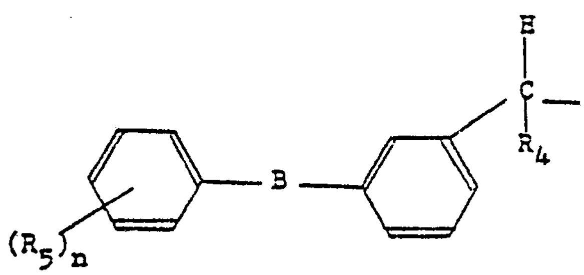

worin B, $R_4$, $R_5$ und n wie in Anspruch 1 definiert sind,
oder eine Gruppe

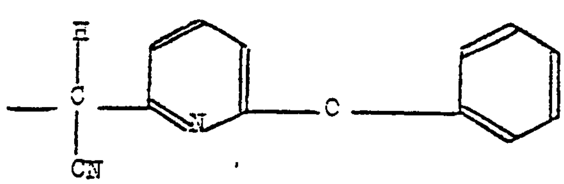

oder eine Gruppe

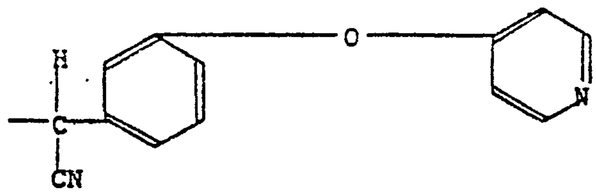

oder eine Gruppe

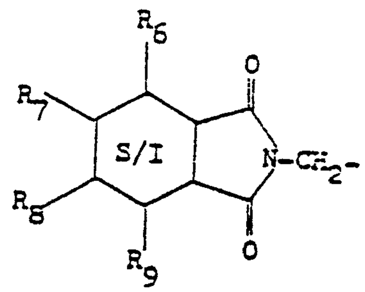

worin die Substituenten $R_6$, $R_7$, $R_8$, $R_9$ und S/I wie in Anspruch 1 definiert sind,
oder eine Gruppe

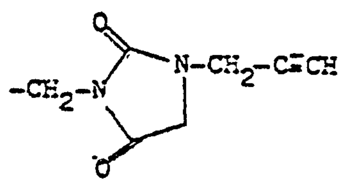

und R wie in Anspruch 1 definiert ist, sowie der Gemische dieser Isomeren, dadurch gekennzeichnet, daß man von einem Alkohol der Formel (III) ausgeht, worin A' die vorstehend angegebenen Bedeutungen von A besitzt.

3. Verfahren zur Herstellung der Verbindungen der Formel (I') gemäß Anspruch 1, dadurch gekennzeichnet, daß man eine Verbindung der Formel (VIII)

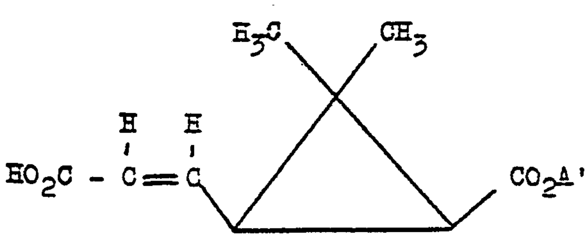

(VIII)

worin A' wie in Anspruch 1 definiert ist, der Einwirkung eines Veresterungsmittels unterzieht, um die entsprechende Verbindung der Formel (I') zu erhalten.

4. Verfahren gemäß Anspruch 3, dadurch gekennzeichnet, daß die Verbindung der Formel (VIII) hergestellt wird, indem man eine Verbindung der Formel (IX)

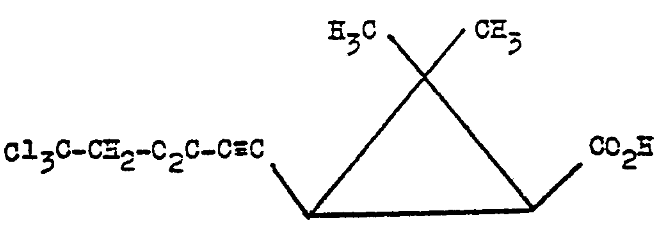

(IX)

der Einwirkung eines Alkohols der Formel (III)

A'—OH (III)

unterzieht, worin A' wie in Anspruch 1 definiert ist, um die Verbindung der Formel (X)

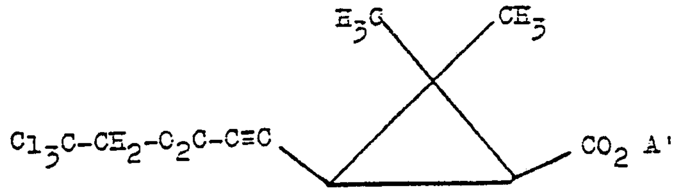

(X)

zu erhalten, die man der Einwirkung eines Mittels zur Spaltung der von acetylenischen Kohlenstoffatom getragenen Esterfunktion unterzieht, um die Verbindung der Formel (XI)

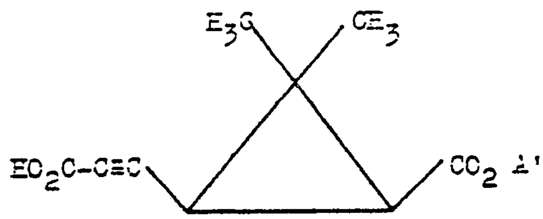

(XI)

zu erhalten, die man der Einwirkung eines milden Hydrierungsmittels unterzieht, um die Verbindung der Formel (VIII) zu erhalten.

5. Verfahren gemäß Anspruch 3 oder 4, dadurch gekennzeichnet, daß man eine Verbindung der Formel (XI)

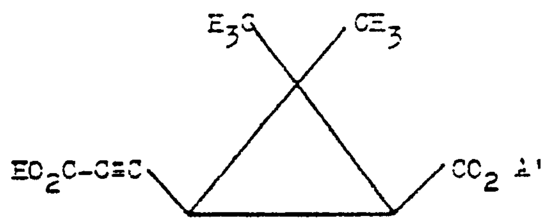

(XI)

worin A' wie in Anspruch 1 definiert ist, der Einwirkung eines Veresterungsmittels unterzieht, um die Verbindung der Formel (XII)

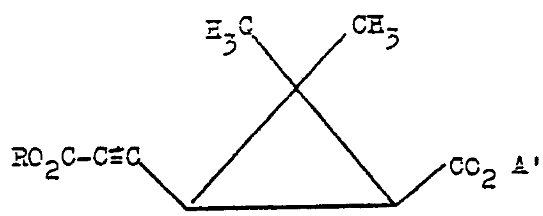

(XII)

zu erhalten, worin R und A' wie in Anspruch 1 definiert sind, die man der Einwirkung eines milden Hydrierungsmittels unterzieht, um die Verbindung der Formel (I') zu erhalten.

6. Verfahren gemäß einem der Ansprüche 1, 3, 4 und 5, dadurch gekennzeichnet, daß man von einer Verbindung der Formel (II), (VIII), IX) oder (XI) ausgeht, deren Struktur 1R-cis- oder 1R-trans ist.

7. Verfahren gemäß einem der Ansprüche 1, 3, 4 und 5, dadurch gekennzeichnet, daß man von einer Verbindung der Formel (II) oder einem Veresterungsmittel ausgeht, worin R einen Methyl-, Ethyl-, n-Propyl-, Isopropyl-, tert.-Butyl- oder Cyclopropylmethylrest bedeutet.

8. Verfahren gemäß einem der Ansprüche 1, 3, 4 und 5, dadurch gekennzeichnet, daß man von einer Verbindung der Formel (III), (VIII) oder (XI) ausgeht, worin A' ausgewählt ist unter der (4S)-3-Methyl-2-(2-propenyl)-1-oxo-cyclopent-2-en-4-yl-Gruppe, der (1,3,4,5,6,7-Hexahydro-1,3-dioxo-2H-isoindol-2-yl)-methylgruppe, der (RS)-Cyano-(6-phenoxy-2-pyridinyl)-methylgruppe, der [5-Phenylmethyl)-3-furanyl]-methylgruppe, der 1-(3-Propargyl-2,5-dioxoimidazolidinyl)-methylgruppe und der (R)-3-Phenoxyphenyl-ethylgruppe.

9. Verfahren gemäß Anspruch 2, dadurch gekennzeichnet, daß man von einer Verbindung der Formel (II) mit 1R-cis- oder 1R-trans-Struktur ausgeht, worin R einen Methyl-, Ethyl-, n-Propyl-, Isopropyl-, tert.-Butyl- oder Cyclopropylmethylrest bedeutet, und einem Alkohol der Formel (III), worin A' ausgewählt ist unter der (4S)-3-Methyl-2-(2-propenyl)-1-oxo-cyclopent-2-en-4-yl-Gruppe, der (1,3,4,5,6,7-Hexahydro-1,3-dioxo-2H-isoindol-2-yl)-methylgruppe, der (RS)-Cyano-(6-phenoxy-2-pyridinyl)-methylgruppe, der [5-Phenylmethyl)-3-furanyl]-methylgruppe, der 1-(3-Propargyl-2,5-dioxoimidazolidinyl)-methylgruppe und der (R)-3-Phenoxyphenylethylgruppe.

10. Verfahren gemäß einem der Ansprüche 1 bis 5, dadurch gekennzeichnet, daß man eine der Verbindungen der Formel (I') mit den folgenden Bezeichnungen herstellt :

— (1S)-2-Methyl-4-oxo-3-(2-propenyl)-2-cyclopenten-1-yl-(1R-cis)-2,2-dimethyl-3-[(Z)-3-methoxy-3-oxo-1-propenyl]-cyclopropan-carboxylat ;

— 1-(3-Propargyl-2,5-dioxoimidazolidinyl)-methyl-(1R-cis-2,2-dimethyl-3-[(Z)-3-tert.-butoxy-3-oxo-1-propenyl]-cyclopropan-carboxylat ;

— 1-(3-Propargyl-2,5-dioxo-imidazolidinyl)-methyl-(1R-cis)-2,2-dimethyl-3-[(Z)-2-cyclopropylmethoxycarbonylethenyl]-cyclopropan-carboxylat ;

— 1(R)-3-Phenoxyphenylethyl-(1R-cis)-2,2-dimethyl-3-[(Z)-2-isopropoxycarbonylethenyl]-cyclopropan-carboxylat ;

— 1-(3-Propargyl-2,5-dioxo-imidazolidinyl)-methyl-(1R-cis)-2,2-dimethyl-3-[(Z)-2-isopropoxycarbonylethenyl]-cyclopropan-carboxylat ;

— 1(R)-3-Phenoxyphenylethyl-(1R-trans)-2,2-dimethyl-3-[(Z)-2-isopropoxycarbonylethenyl]-cyclopropancarboxylat.

11. Verwendung der Verbindungen der Formel (I') gemäß Anspruch 1 zur Bekämpfung von Parasiten der Pflanzen, Parasiten im häuslichen Bereich und Parasiten warmblütiger Tiere.

12. Verwendung der Verbindungen der Formel (I') gemäß Anspruch 2 zur Bekämpfung von Parasiten der Pflanzen, Parasiten im häuslichen Bereich und Parasiten warmblütiger Tiere.

13. Zusammensetzungen für die Bekämpfung von Parasiten der Pflanzen, Parasiten im häuslichen Bereich und Parasiten warmblütiger Tieere, dadurch gekennzeichnet, daß sie zumindest eines der Produkte gemäß Anspruch 1 enthalten.

14. Insektizide Zusammensetzungen, enthaltend als Wirkstoff zumindest eines der Produkte gemäß Anspruch 1.

15. Insektizide Zusammensetzungen, enthaltend als Wirkstoff zumindest eines der Produkte gemäß Anspruch 10.

16. Zusammensetzungen für die tierische Ernährung, enthaltend als Wirkstoff zumindest eines der Produkte gemäß Anspruch 1.

17. Mit insektizider, akarizider oder nematizider Aktivität ausgestattete Zusammensetzungen, dadurch gekennzeichnet, daß sie als Wirkstoff einesteils zumindest eine der Verbindungen der allgemeinen Formel (I') und anderenteils zumindest einen der Pyrethrinoidester ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxy-benzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahalo)-cyclopropan-1-carbonsäuren, enthalten, worin « Halo » ein Fluor-, Chlor- oder Bromatom bedeutet, wobei die Verbindungen der Formel (I') in sämtlichen ihrer möglichen stereoisomeren Formen ebenso wie die Säure- und Alkohol-Verknüpfungen der vorstehenden Pyrethrinoidester vorliegen können.

18. Zusammensetzungen gemäß einem der Ansprüche 13 bis 17, dadurch gekennzeichnet, daß die Verbindungen der Formel (I') der Formel (I), wie in Anspruch 2 definiert, entsprechen.